(19)

Europäisches
Patentamt
European
Patent Office
Office européen
des brevets

(11) **EP 4 098 267 A1**

(12) **EUROPEAN PATENT APPLICATION**
published in accordance with Art. 153(4) EPC

(43) Date of publication:
**07.12.2022 Bulletin 2022/49**

(21) Application number: **21748145.6**

(22) Date of filing: **29.01.2021**

(51) International Patent Classification (IPC):
*A61K 35/28* (2015.01)        *A61K 9/10* (2006.01)
*A61K 47/02* (2006.01)        *A61K 47/12* (2006.01)
*A61K 47/20* (2006.01)        *A61K 47/22* (2006.01)
*A61K 47/42* (2017.01)        *A61P 1/18* (2006.01)
*A61P 7/04* (2006.01)        *A61P 9/04* (2006.01)
*A61P 9/10* (2006.01)        *A61P 9/14* (2006.01)
*A61P 11/00* (2006.01)        *A61P 11/06* (2006.01)
*A61P 17/00* (2006.01)        *A61P 27/02* (2006.01)
*A61P 29/00* (2006.01)        *A61P 31/04* (2006.01)
*A61P 31/12* (2006.01)        *A61P 31/14* (2006.01)
*A61P 31/16* (2006.01)

(52) Cooperative Patent Classification (CPC):
**A61K 9/10; A61K 35/28; A61K 47/02; A61K 47/12;
A61K 47/20; A61K 47/22; A61K 47/42; A61P 1/18;
A61P 7/04; A61P 9/04; A61P 9/10; A61P 9/14;
A61P 11/00; A61P 11/06; A61P 17/00;** (Cont.)

(86) International application number:
**PCT/JP2021/003201**

(87) International publication number:
**WO 2021/153719 (05.08.2021 Gazette 2021/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.01.2020 JP 2020014235
14.10.2020 JP 2020173105**

(71) Applicant: **JCR Pharmaceuticals Co., Ltd.
Ashiya-shi, Hyogo 659-0021 (JP)**

(72) Inventors:
• **IMAGAWA, Kiwamu
Kobe-shi, Hyogo 651-2241 (JP)**
• **MINAMI, Kohtaro
Kobe-shi, Hyogo 651-2241 (JP)**
• **MAEDA, Kenichi
Kobe-shi, Hyogo 651-2241 (JP)**

• **HOSODA, Yuki
Kobe-shi, Hyogo 651-2241 (JP)**
• **WATANABE, Shunsuke
Kobe-shi, Hyogo 651-2241 (JP)**
• **SATO, Kazutoshi
Kobe-shi, Hyogo 651-2241 (JP)**
• **HIGASHIGUCHI, Yasuna
Kobe-shi, Hyogo 651-2241 (JP)**
• **KUSHIDA, Takashi
Osaka-shi, Osaka 530-0005 (JP)**
• **ISHIWARI, Ayumi
Osaka-shi, Osaka 530-0005 (JP)**
• **TSUSHIMA, Yu
Tokyo 100-0013 (JP)**
• **MIYAUCHI, Yoshiteru
Osaka-shi, Osaka 530-0005 (JP)**

(74) Representative: **Isarpatent
Patent- und Rechtsanwälte Barth
Charles Hassa Peckmann & Partner mbB
Friedrichstrasse 31
80801 München (DE)**

(54) **MEDICINAL COMPOSITION COMPRISING DENTAL PULP-DERIVED CELLS**

(57)    [Problem] To provide a pharmaceutical composition having a novel use comprising a dental pulp-derived multipotent stem cell preparation that can be administered to humans.
   [Solution] A pharmaceutical composition comprising dental pulp-derived stem cells as an active ingredient for

suppressing infiltration into a tissue of at lease neutrophils, monocytes, or lymphocytes.

Fig. 26

(52) Cooperative Patent Classification (CPC): (Cont.)
   **A61P 27/02; A61P 29/00; A61P 31/04;**
   **A61P 31/12; A61P 31/14; A61P 31/16;**
   **A61P 43/00**

**Description**

TECHNICAL FIELD

The present invention relates to multipotent stem cells obtained from dental pulp having a capacity to differentiate into osteocytes and chondrocytes, and to a method for production of such multipotent stem cells, for example.

BACKGROUND ART

Stem cells having the ability to differentiate into cells of multiple lineages (multipotent stem cells) are known to be obtained from various tissues. Mesenchymal stem cells isolated from bone marrow are a such groups of cells and have the ability to differentiate into various cells such as osteocytes, cardiomyocytes, chondrocytes, and adipocytes (Patent Documents 1 to 4). Using this ability to differentiate, attempts have been made to apply mesenchymal stem cells in regenerative treatment of various tissues. For example, it has been reported that administration of mesenchymal stem cells to patients with myocardial infarction was tested aiming to regenerate the cardiac muscle cells which had undergone necrosis in myocardial infarction, resulting in an improvement of the patients' cardiac functions (Non-patent Document 1).
Mesenchymal stem cells are known to be obtainable not only from bone marrow mentioned above but also from various tissues such as adipose tissue (Patent Document 5), placental, umbilical cord tissues (Patent Document 6), and the like.
Multipotent stem cells can be obtained from dental tissues, too, and it has been reported that multipotent stem cells are obtainable from dental pulp (Patent Documents 7-11), dental follicle (Patent Document 12), dental sac (Patent Documents 11, 13), dental papilla (Patent Document 14), periodontal ligament (Patent Document 15), for example. Multipotent stem cells derived from dental tissues are generally considered to have an ability to differentiate into adipocytes (Non-patent Documents 2-4).
Multipotent stem cells from dental pulp are obtained along the following procedure (Patent Document 16) in general. Namely, a tissue obtained by fracturing an extracted tooth is treated with type I collagenase and Dispase (registered trademark) and passed through a filter to remove cell aggregates and thereby prepare a cell suspension. The cells then are allowed to grow on a cell culture plate using a DMEM medium containing 20% FBS. The cells that have grown and adhered to the inner surfaces of the cell culture plate are detached by trypsin treatment and recovered. The cells thus recovered are considered to be multipotent stem cells derived from dental pulp. Besides, Dispase is a neutral protease originating from *Bacillus polymyxa.*
Dental pulp is a loose fibrous connective tissue that occupies the dental pulp cavity of a tooth, and is divided into coronal pulp and radicular pulp depending on their location. While multipotent stem cells derived from tooth tissues are generally considered to have an ability to differentiate into adipocytes (Non-patent Documents 2-4), there also is a report that stem cells derived from dental pulp do not differentiated into adipocytes (Patent Document 8). Thus, stem cells obtained from dental pulp may include at least two types which are distinguished from each other based on their ability, or inability, to differentiate into adipocytes. It has also been reported that stem cells obtained from the dental pulp of a deciduous tooth have different properties from those occurring in the dental pulp of a permanent tooth in that, for example, the former have a higher proliferative ability and exhibit higher expression levels of FGF2, TGF-β, collagen I and collagen III compared with those occurring in dental pulp of permanent teeth (Patent Document 16). Further, there also is a report showing that multipotent stem cells derived from dental pulp differ from bone marrow-derived mesenchymal stem cells in their property relating to their induction to differentiate into osteoblasts (Patent Document 11).
On the other hand, it is considered that mesenchymal stem cells produce a variety of secretory factors, which play a role in exhibiting therapeutic effects on various diseases. Indeed, there have been reported that mesenchymal stem cells that have been potentiated in their expression levels of factors such as angiopoietin-1 (Ang-1), and HGF by means of gene modification technique showed improved therapeutic effects on animal models of disorders such as cerebral infarction, myocardial infarction, acute lung injury. However, since gene-modified mesenchymal stem cells may involve risks like tumor growth because of vectors or introduced genes used to perform gene modification, their clinical safety is unestablished (Non-patent Documents 5-19).
It is known that in patients of cerebral infarction, their blood concentration of angiopoietin-1 (Ang-1) is lower with statistical significance at the onset of cerebral infarction than in normal humans, and that patients with the lower concentration of it are in the severer conditions at 3 months after the onset of cerebral infarction (Non-patent Document 20). Further, it is also known that blood concentration of angiopoietin-1 (Agn-1) is low in patients of sepsis or acute infection accompanied by acute lung injury, with its blood concentration being especially low in severe patients (Non-patent Documents 21, 22).
Cerebral infarction is largely grouped into cardiogenic infarction, thrombotic infarction, and lacunar infarction. Among these, it is reported that angiopoietin-1 may play a role in prophylaxis of lacunar infarction. Also reported is that angiopoietin-1 may have prophylactic effect on cardiogenetic infarction and thrombotic infarction (Non-patent Document 23).

Multipotent stem cells derived from dental pulp is expected for their clinical use such as therapeutic agents of neurological diseases (Patent Document 17).

PRIOR ART DOCUMENTS

PATENT DOCUMENTS

[Patent Document 1] US Patent No. 5486359
[Patent Document 2] US Patent No. 5827740
[Patent Document 3] US Patent No. 6835377
[Patent Document 4] US Patent No. 6387369
[Patent Document 5] JP Patent Application Publication No. 2004-129549
[Patent Document 6] JP Patent Application Publication No. 2004-210713
[Patent Document 7] JP Patent Application Publication No. 2010-252778
[Patent Document 8] WO 2002/007679
[Patent Document 9] JP Patent Application Publication No. 2008-507962
[Patent Document 10] WO 2009/072527
[Patent Document 11] JP Patent Application Publication No. 2004-201612
[Patent Document 12] JP Patent Application Publication No. 2009-527223
[Patent Document 13] JP Patent Application Publication No. 2005-516616
[Patent Document 14] JP Patent Application Publication No. 2006-238875
[Patent Document 15] JP Patent Application Publication No. 2008-295420
[Patent Document 16] JP Patent Application Publication No. 2010-268715
[Patent Document 17] JP Patent Application Publication No. 2011-219432

NON-PATENT DOCUMENTS

[Non-patent Document 1] Katritsis DG. et. al., Catheter Cardiovasc Interv. 65(3): 321-9 (2005)
[Non-patent Document 2] Gronthos S. et. al., J Dent Res. 81(8): 531-5 (2002)
[Non-patent Document 3] Tirino V. et. al., Stem Cell Rev. 7(3): 608-15 (2011)
[Non-patent Document 4] Vishwanath VR et. al., J Conserv Dent. 16(5): 423-8 (2013)
[Non-patent Document 5] Huang ZW et. al., Yonsei Med J. 2017 Jan;58(1):206-216. doi: 10.3349/ymj.2017.58.1.206.
[Non-patent Document 6] Halim NSS et. al., Stem Cell Rev Rep. 2019 Feb;15(1):112-125. doi: 10.1007/s12015-018-9844-7.
[Non-patent Document 7] Xu J et. al., J Pathol. 2008 Mar;214(4):472-81. doi: 10.1002/path.2302.
[Non-patent Document 8] Mei SH et.al., Version 2. PLoS Med. 2007 Sep;4(9):e269. doi: 10.1371/journal.pmed.0040269.
[Non-patent Document 9] Shao Y et. al., Inhal Toxicol. 2018 Jun-Jul;30(7-8):313-320. doi: 10.1080/08958378.2018.1521483. Epub 2018 Nov 5.
[Non-patent Document 10] Sun L et. al., Biochem Biophys Res Commun. 2007 Jun 8;357(3):779-84. doi: 10.1016/j.bbrc.2007.04.010. Epub 2007 Apr 11.
[Non-patent Document 11] Chen SL et. al., J Int Med Res. 2009 Jan-Feb;37(1):68-78. doi: 10.1177/147323000903700108.
[Non-patent Document 12] Toyama K et. al., Exp Neurol. 2009 Mar;216(1):47-55. doi: 10.1016/j.expneurol.2008.11.010. Epub 2008 Nov 27.
[Non-patent Document 13] Onda T et. al., J Cereb Blood Flow Metab. 2008 Feb;28(2):329-40. doi: 10.1038/sj.jcbfm.9600527. Epub 2007 Jul 18.
[Non-patent Document 14] Liu FY et. al., J Cell Physiol. 2018 Nov;233(11):8567-8577. doi: 10.1002/jcp.26501. Epub 2018 May 15.
[Non-patent Document 15] Hua J et. al., Int J Clin Exp Pathol. 2014 Jun 15;7(7):3580-95. eCollection 2014.
[Non-patent Document 16] Piao W et. al., Angiogenesis. 2010 Sep;13(3):203-10. doi: 10.1007/s10456-010-9169-x. Epub 2010 May 11.
[Non-patent Document 17] Li Y et. al., Stem Cell Res Ther. 2013 Sep 16;4(5):113. doi: 10.1186/scrt324.

[Non-patent Document 18] Cao L et.al., J Biomed Mater Res B Appl Biomater. 2012 Jul;100(5):1229-36. doi: 10.1002/jbm.b.32687. Epub 2012 May 11.

[Non-patent Document 19] Kota S et. al. Molecular Therapy:Method & Clinical Development Vol 10 September 2018 281-290.

[Non-patent Document 20] J. Golledge et. al., J the American Heart Association Stroke, 2014 ;45:1064-1068 doi:10.1161/STROKEAHA.113.004339 Epub 2014 Feb 25.

[Non-patent Document 21] Limangel A.M. et. al., Critical Care 2010,14:R91.

[Non-patent Document 22] Sascha D. et. al., Critical Care 2010,14:180.

[Non-patent Document 23] Chen J. et. al., Hum Mol Genet. 2010 Jun 15;19(12):2524-33. doi:10.1093/hmg/ddq131. Epub 2010 Apr8.

## SUMMARY OF INVENTION

### TECHNICAL PROBLEM

It is an objective of the present invention to provide a pharmaceutical composition comprising a dental pulp-derived multipotent stem cell preparation that can be administered to a human for novel uses.

### SOLUTION TO PROBLEM

As a result of intensive studies directed to the above objective, the present inventors found that dental pulp-derived cells which have been enriched in multipotent cells starting from dental pulp obtained from extracted human tooth (hereinafter referred to also "dental pulp-derived stem cells") have an ability to secrete angiopoietin-1 (Ang-1), one of factors for vascular regeneration, in a very large amount as compared with bone marrow-derived multipotent stem cells, and the dental pulp-derived stem cells lower the permeability of vascular endothelium, suppress infiltration of cells such as neutrophils into tissues, and suppress expression of adhesion factors, thereby having completed the invention on the basis of these findings. Thus, the present invention includes what follows:

1. A pharmaceutical composition comprising dental pulp-derived stem cells for suppressing infiltration of at least one of neutrophils, monocytes, or lymphocytes into tissues.
2. The pharmaceutical composition according to 1 above that suppresses expression of an adhesion factor.
3. The pharmaceutical composition according to 2 above, wherein the adhesion factor is expressed on the surfaces of vascular endothelial cells of the tissues.
4. The pharmaceutical composition according to 2 or 3 above, wherein the adhesion factor is VCAM-1.
5. The pharmaceutical composition according to one of 2 to 4 above, wherein the dental pulp-derived stem cells increase the tissue concentration of a factor that suppresses expression of the adhesion factors.
6. The pharmaceutical composition according to 5 above, wherein the factor includes angiopoietin-1.
7. The pharmaceutical composition according to one of 1 to 6 above, wherein the composition suppresses excessive increase of vascular permeability in the tissues.
8. The pharmaceutical composition according to one of 1 to 7 above for treatment and/or prophylaxis of a disorder accompanied by inflammation.
9. The pharmaceutical composition according to 8 above, wherein the disorder accompanied by inflammation is selected from the group consisting of cerebral infarction, cerebral bleeding, retinopathy accompanied by macular degeneration, acute lung injury, chronic inflammatory pulmonary disease, asthma, acute pancreatitis, myocardial infarction, heart failure, dermatitis, and scepsis.
10. The pharmaceutical composition according to 9 above, wherein the disorder accompanied by inflammation is acute lung injury, wherein the acute lung injury is selected from the group consisting of aspiration pneumonitis, bacterial pneumonitis, viral pneumonitis, acute respiratory distress syndrome (also written as ARDS), and interstitial pneumonia.
11. The pharmaceutical composition according to 10 above, wherein the acute lung injury is viral pneumonitis, wherein the viral pneumonitis is caused by infection with corona virus, influenza virus, or RS virus.
12. The pharmaceutical composition according to 11 above, wherein the viral pneumonitis is caused by the infection with corona virus, wherein the corona virus is SARS-CoV, MARS-CoV, or SARS-CoV-2.
13. The pharmaceutical composition according to 9 above, wherein the disorder accompanied by inflammation is chronic inflammatory pulmonary disease, wherein the chronic inflammatory pulmonary disease is selected from the group consisting of cystic fibrosis (also written as CF), chronic obstructive pulmonary disease (also written as COPD), lung dysplasia, chronic pulmonary disease, and idiopathic pulmonary fibrosis (also written as IPF).
14. The pharmaceutical composition according to one of 1 to 7 above comprising dental pulp-derived stem cells as an active ingredient for treatment of infection with SARS-CoV, MARS-CoV, or SARS-CoV-2.

15. The pharmaceutical composition according to one of 1 to 14 above for administration to a human.

16. The pharmaceutical composition according to one of 1 to 15 above, wherein the dental pulp-derived stem cells are of human origin

17. The pharmaceutical composition according to one of 1 to 16 above, wherein the pharmaceutical composition is for administration via a route of administration selected from the group consisting of intravenous administration, intra-arterial administration, intraportal administration, intracutaneous administration, subcutaneous administration, intramuscular administration, intracerebroventricular administration, intra-endocardium administration, intra-respiratory tract administration, intraocular administration, intra-aural administration, intranasal application, and intra-articular administration.

18. The pharmaceutical composition according to one of 1 to 17 above, wherein the pharmaceutical composition is administered either only once or repeatedly.

19. The pharmaceutical composition according to one of 1 to 18 above, wherein the dental pulp-derived stem cells have at least one of the characteristics defined in (1) to (4) below:

(1) positive for CD73, CD90, CD105, and CD166; negative for CD34, CD40, CD45, CD80, CD86, and MHC-class II antigen; turning positive for MHC-class II antigen when stimulated with interferon $\gamma$; expressing a prostaglandin $E_2$ and/or vascular endothelial growth factor, wherein the levels of prostaglandin $E_2$ expression increasing when stimulated with TNF-$\alpha$.

(2) positive for at least one of CD73, CD90, D105, and D166; and negative for CD34 and CD45,

(3) positive for at least one of CD47, CD81, and CD147; and negative for at least one of CD19, CD34, and CD206,

(4) positive for at least one of CD47, CD81, and CD147; and negative for at least one of CD19, CD31, CD33, CD34, CD38, CD45, CD206, CD235a, and SSEA-1.

20. The pharmaceutical composition according to one of 1 to 19 above, wherein the dental pulp-derived stem cells have an ability to differentiate into osteocytes and chondrocytes.

21. The pharmaceutical composition according to one of 1 to 20 above, wherein the mean diameter of the dental pulp-derived stem cells is 16-20 $\mu$m when suspended in a culture medium.

22. The pharmaceutical composition according to one of 1 to 21 above, wherein the dental pulp-derived stem cells have an ability to undergo cell divisions not less than three times in an in vitro environment, and their mean doubling time is not more than 96 hours.

23. The pharmaceutical composition according to one of 1 to 21 above, wherein the dental pulp-derived stem cells are characterized as follows:

having undergone cell divisions in an in vitro environment not less than 10 times, not less than 15 times, not less than 16 times or not less than 17 times, with their mean time required per cell division being not more than 48 hours; and

having an ability to undergo cell divisions in an in vitro environment not less than 10 times, not less than 14 times of not less than 15 times, with their mean time required per cell division being not more than 96 hours, not more than 84 hours, not more than 72 hours, not more than 48 hours, or not more than 36 hours.

24. The pharmaceutical composition according to one of 1 to 21 above, wherein the dental pulp-derived stem cells are characterized as follows:

having undergone cell divisions under an in vitro condition not less than 16 times, not less than 21 times, not less than 22 times, or not less than 23 times; and

having an ability to undergo cell divisions in an in vitro environment not less than 3 times, not less than 4 times, or not less than 5 times, and their mean time per cell division being not more than 96 hours, not more than 84 hours, not more than 72 hours, not more than 48 hours, or not more than 36 hours.

25. The pharmaceutical composition according to one of 1 to 24 above, wherein the dental pulp-derived stem cells are negative for at least one of CD19, CD26, CD106, CD117, and CD271.

26. The pharmaceutical composition according to one of 1 to 25 above, wherein the dental pulp-derived stem cells are positive for at least one of CD140b and HLA-A, B, C.

27. The pharmaceutical composition according to one of 1 to 26 above, wherein the dental pulp-derived stem cells are negative for at least one of CD56 and CD146.

28. The pharmaceutical composition according to one of 1 to 27 above, wherein the dental pulp-derived stem cells are positive for at least one of CD49e and CD95.

29. The pharmaceutical composition according to one of 1 to 28 above, wherein the dental pulp-derived stem cells are positive for at least one of CD10, CD46, CD47, CD55, CD58, and CD59.

30. The pharmaceutical composition according to one of 1 to 29 above, wherein the dental pulp-derived stem cells express at least one of MMP-2, IGFBP-4, cystatin C, IL-6, IL-11, MCP-1, IL-8, HGF, VEGF, TIMP-1, TIMP-2, and TIMP-3.

31. The pharmaceutical composition according to one of 1 to 30 above, wherein the dental pulp-derived stem cells express at least one of GRO$\alpha$, VCAM-I, and IP-10.

32. The pharmaceutical composition according to one of 1 to 31 above, wherein the dental pulp-derived stem cells express IL-6, wherein the levels of expression thereof increase when stimulated with TNF-$\alpha$, and when stimulated with interferon $\gamma$.

33. The pharmaceutical composition according to one of 1 to 32 above, wherein the dental pulp-derived stem cells express IL-11, wherein the levels of expression thereof increase when stimulated with TNF-$\alpha$, and when stimulated with interferon $\gamma$.

34. The pharmaceutical composition according to one of 1 to 33 above, wherein the dental pulp-derived stem cells express IP-10 (CXCL 10), wherein the levels of expression thereof increase when stimulated TNF-$\alpha$, and when stimulated with interferon $\gamma$.

35. The pharmaceutical composition according to one of 1 to 34 above, wherein the dental pulp-derived stem cells express MCP-1, wherein the levels of expression thereof increase when stimulated with TNF-$\alpha$, and when stimulated with interferon $\gamma$.

36. The pharmaceutical composition according to one of 1 to 35 above, wherein the dental pulp-derived stem cells are induced to express GM-CSF when stimulated with TNF-$\alpha$.

37. The pharmaceutical composition according to one of 1 to 36 above, wherein the dental pulp-derived stem cells express HGF, wherein the levels of expression thereof decrease when stimulated with TNF-$\alpha$, and increase when stimulated with interferon $\gamma$.

38. The pharmaceutical composition according to one of 1 to 37 above, wherein the dental pulp-derived stem cells express IL-8, wherein the level of expression thereof increases when stimulated with TNF-$\alpha$.

39. The pharmaceutical composition according to one of 1 to 38 above, wherein the dental pulp-derived stem cells express G-CSF, wherein the level of expression thereof increases when stimulated with TNF-$\alpha$.

40. The pharmaceutical composition according to one of 1 to 39 above, wherein the amount of angiopoietin-1 produced by the dental pulp-derived stem cells is not less than three times the amount thereof produced by human bone marrow-derived stem cells, when the dental pulp-derived stem cells and the human bone marrow-derived stem cells are respectively cultured under the same condition.

41. The pharmaceutical composition according to one of 1 to 40 above, wherein the dental pulp-derived stem cells are induced to express G-CSF when stimulated with TNF-$\alpha$.

42. The pharmaceutical composition according to one of 1 to 41 above, wherein the dental pulp-derived stem cells are derived from dental pulp obtained from a human permanent tooth or deciduous tooth.

43. The pharmaceutical composition according to one of 1 to 42 above, wherein the dental pulp-derived stem cells are contained in a suspended form in a solution that comprises sodium ion, potassium ion, calcium ion, magnesium ion, bicarbonate ion, citrate ion, human serum albumin, and dimethyl sulfoxide.

44. The pharmaceutical composition according to one of 1 to 42 above, wherein the dental pulp-derived stem cells are contained in a suspended form in bicarbonate Ringer's solution that contains human serum albumin and dimethyl sulfoxide.

45. The pharmaceutical composition according to 43 above containing 91-113 mM sodium ion, 2.52-3.08 mM potassium ion, 0.95-1.16 mM calcium ion, 0.315-0.385 mM magnesium ion, 15.6-19.2 mM bicarbonate ion, 1.04-1.28 mM citrate ion, 46-56 g/L human serum albumin, and 9-11 % (v/v) dimethyl sulfoxide.

46. The pharmaceutical composition according to one of 43 to 45 above, further comprising acetyltryptophan or a salt thereof and caprylic acid or a salt thereof.

47. The pharmaceutical composition according to one of 43 to 46 above, wherein the dental pulp-derived stem cells are contained at a density of $5 \times 10^6$ to $8 \times 10^7$ cells/mL.

48. The pharmaceutical composition according to one of 43 to 47 above, sealed in a container in an amount of 1-20 mL.

49. The pharmaceutical composition according to 48 above, wherein the container is made of a glass or plastic.

50. The pharmaceutical composition according to one of 43 to 49 above in a frozen state.

51. A pharmaceutical composition for elevating angiopoietin-1 concentration in a tissue, wherein the composition comprises dental pulp-derived stem cells as an active ingredient.

52. A pharmaceutical composition suppressing infiltration of at least neutrophils, monocytes, or lymphocytes into tissues, wherein the composition comprises dental pulp-derived stem cells as an active ingredient.

53. A pharmaceutical composition for suppressing expression of an adhesion factor, wherein the composition comprises dental pulp-derived stem cells as an active ingredient.

54. A pharmaceutical composition for treatment or prophylaxis of a disorder accompanied by inflammation, wherein the composition comprises dental pulp-derived stem cells as an active ingredient.

EFFECTS OF INVENTION

The present invention provides a pharmaceutical composition comprising dental pulp-derived stem cells as an active ingredient and having a therapeutic effect to disorders accompanied by infiltration of neutrophils to tissues, for example.

BRIEF DESCRIPTION OF DRAWINGS

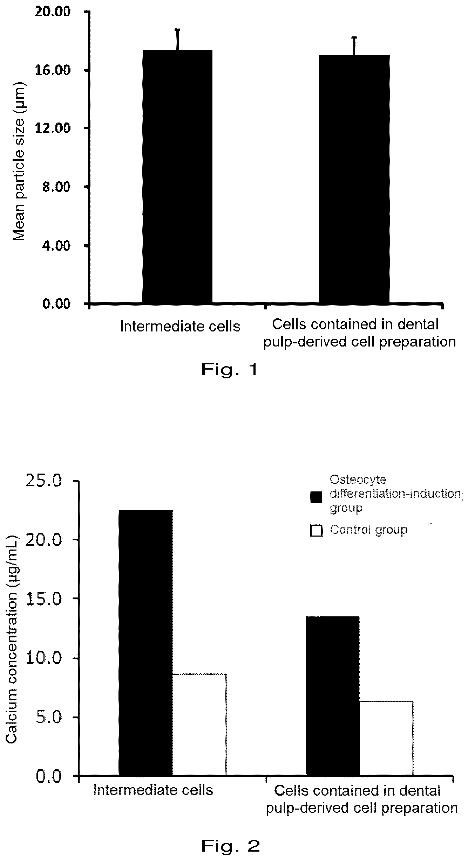

[Fig.1] Fig. 1 shows the mean of measurements of particle size of the dental pulp-derived cells contained in the intermediate and the dental pulp-derived cell preparation obtained in Example 7 and Example 16, respectively (the intermediate cells, and the cells contained in the dental pulp-derived cell preparation). The vertical axis represents mean particle size ($\mu$m). From the left, mean particle sizes of intermediate cells and the cells contained in the dental pulp-derived cell preparation are shown. The error bars indicate standard deviation (n=3).

[Fig. 2] Fig.2 is a graph showing the results of the test of the dental pulp-derived cells obtained in Example 7 and Example 16 for their ability to differentiate into osteocytes. The vertical axis represents calcium concentration ($\mu$g/mL). Black bars indicate the concentration of calcium released from the osteocyte differentiation-induction groups, and white bars indicate that from the control groups, either in the intermediate cells (Example 7) or the cells contained in the dental pulp-derived cell preparation (Example 16), respectively.

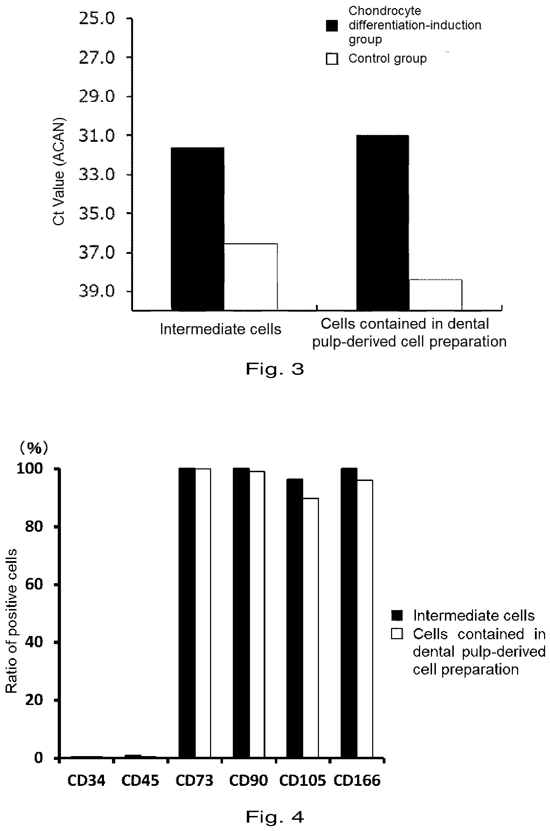

[Fig. 3] Fig. 3 is a graph showing the results of the test of dental pulp-derived cells obtained in Example 7 and Example 16 for their ability to differentiate into chondrocytes. The vertical axis represents Ct value for aggrecan (ACAN). Black bars indicate the Ct values for aggrecan (ACAN) in the chondrocyte differentiation-induction groups, and white bars indicate that in the control groups, respectively, either in the intermediate cells or the cells contained in the dental pulp-derived cell preparation, respectively.

[Fig. 4] Fig. 4 is a graph showing the results of measurement of dental pulp-derived cells obtained in Example 7 and Example 16 for their surface antigens. The vertical axis represents ratio (%) of positive cells. The ratio of cells positive for CD34, CD45, CD73, CD90, CD105, and CD166, from the left, respectively, are shown, in the intermediate cells (black bars) and the cells contained in the dental pulp-derived cell preparation (white bars).

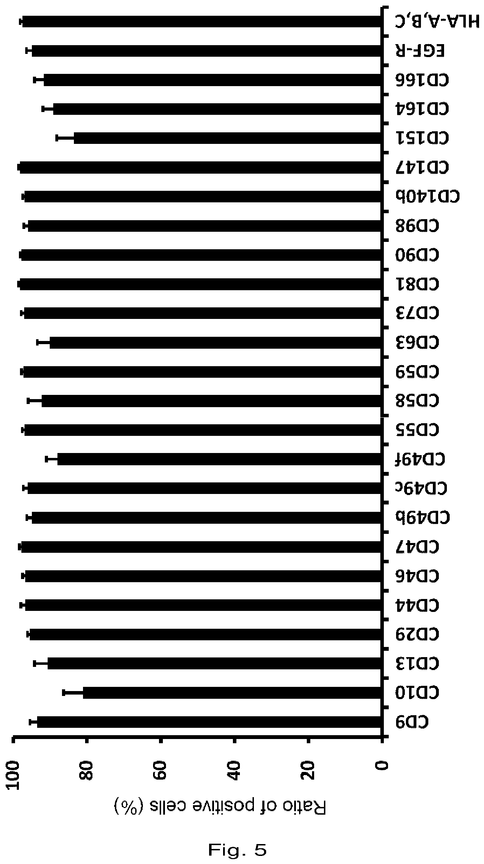

[Fig. 5] Fig. 5 is a graph showing the results of measurement of dental pulp-derived cells contained in the intermediate obtained in Example 7 (the intermediate cells) for their surface antigens. The vertical axis represents ratios (%) of positive cells. From the left, the ratio of cells positive for CD9, CD10, CD13, CD29, CD44, CD46, CD47, CD49b, CD49c, CD49f, CD55, CD58, CD59, CD63, CD73, CD81, CD90, CD98, CD140b, CD147, CD151, CD164, CD166, EGF-R (epidermal growth factor receptor), and HLA-A, B, C (human leukocytic antigen -A, B, C), respectively, are shown. Values are means, and error bars indicate standard errors (n=9).

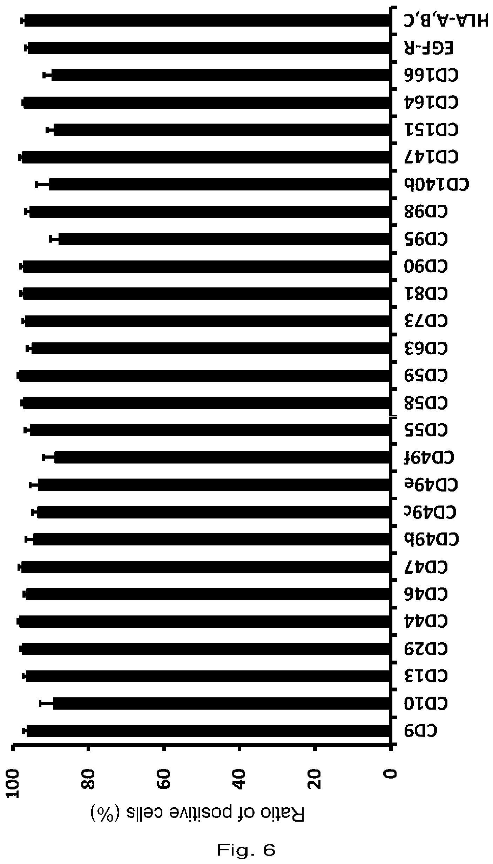

[Fig. 6] Fig. 6 is a graph showing the results of measurement of the cells contained in the dental pulp-derived cell preparation prepared in Example 16 for their surface antigens. The vertical axis represents ratio (%) of positive cells. From the left, the cells positive for CD9, CD10, CD13, CD29, CD44, CD46, CD47, CD49b, CD49c, CD49e, CD49f, CD55, CD58, CD59, CD63, CD73, CD81, CD90, CD95, CD98, CD140b, CD147, CD151, CD164, CD166, EGF-R, and HLA-A, B, C, respectively, are shown. Values are means, and error bars indicate standard errors (n=7).

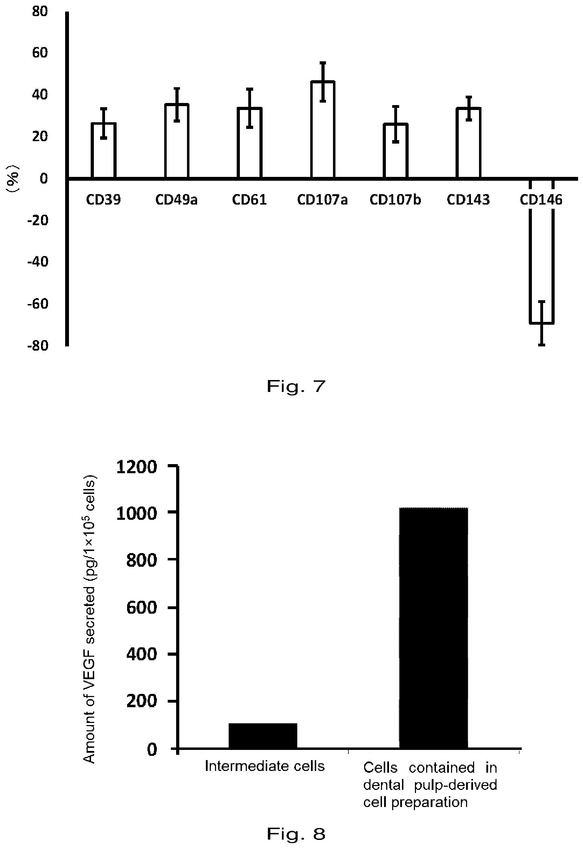

[Fig. 7] Fig. 7 is a graph showing the difference between surface antigens for which not less than 10% difference in the positive ratio was found between the dental pulp-derived cells contained in the intermediate and the preparation obtained in Example 7 and Example 16 (positive ratio (%) in the preparation - positive ratio (%) in the intermediate). The results are shown for CD39, CD49a, CD61, CD107a, CD107b, CD143, and CD146, from the left, respectively. Values are means, and error bars indicate standard errors (n=7).

[Fig. 8] Fig. 8 is a graph showing the test results of the ability to secrete VEGF of the dental pulp-derived cells contained in the intermediate and the preparation obtained in Example 7 and Example 16. The vertical axis represents the amount (pg/1$\times$10$^5$ cells) of vascular endothelial growth factor (VEGF) secreted.

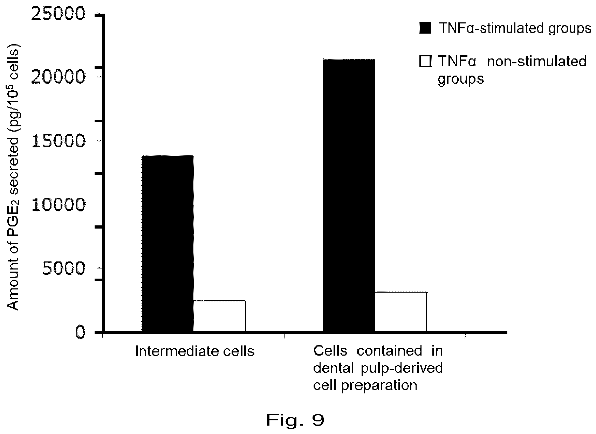

[Fig. 9] Fig. 9 is a graph showing the test results of the ability to secrete prostaglandin E$_2$ (PGE$_2$) of the dental pulp-derived cells contained in the intermediate and the preparation obtained in Example 7 and Example 16. The vertical axis represents the amount (pg/1$\times$10$^5$ cells) of PGE$_2$ secreted. Black bars indicate the results in the TNF$\alpha$ (tumor necrosis factor a)-stimulated groups and white bars indicate the TNF$\alpha$ non-stimulated groups, respectively.

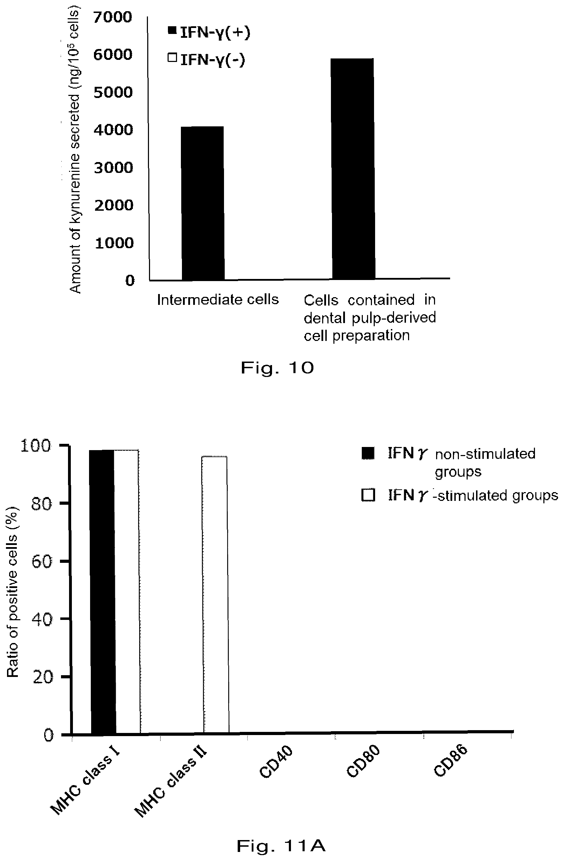

[Fig. 10] Fig. 10 is a graph showing the test result of the ability to secrete kynurenine of the dental pulp-derived cells contained in the intermediate and the preparation obtained in Example 7 and Example 16. The vertical axis represents the amount (ng/1$\times$10$^5$ cells) of kynurenine secreted by the intermediate cells and the cells contained in the dental pulp-derived cell preparation, respectively.

[Fig. 11A] Fig. 11A is a graph showing the results of low-antigenicity test of the intermediate dental pulp-derived cells obtained in Example 7. The vertical axis represents the ratio (%) of positive cells. Black bars indicate the results in the IFNy (interferon γ)-non-stimulated group, and white bars indicate the results in the IFNy-stimulated group. The positive ratios of the cells are shown for, from the left, MHC (major histocompatibility complex)-class I antigen, MHC (major histocompatibility complex)-class II antigen, CD40, CD80, and CD86.

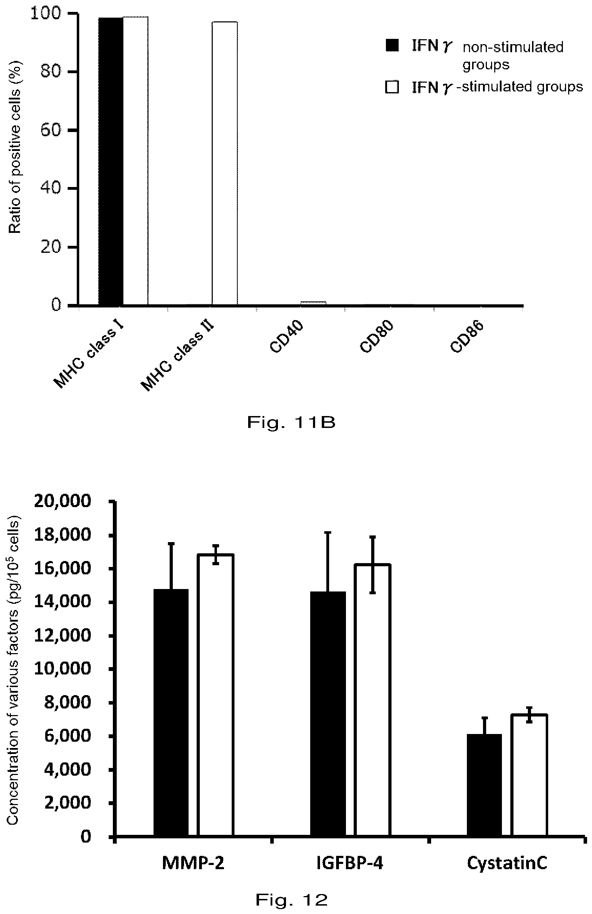

[Fig. 11B] Fig. 11B is a graph showing the results of low-antigenicity test of the dental pulp-derived cells in the preparation obtained in Example 16. The vertical axis represents the ratio (%) of positive cells. Black bars indicate the results in the IFNy-non-stimulated group, and the white bars indicate the results in the IFNy-stimulated group, respectively. The positive ratios of the cells are shown for, from the left, MHC-class I antigen, MHC-class II antigen, CD40, CD80, and CD86.

[Fig. 12] Fig. 12 is a graph showing the results of measurement of the concentration of various factors such as cytokines in the culture supernatant of the dental pulp-derived cells contained in the intermediate and the preparation obtained in Example 7 and Example 16, respectively, under no stimulation. The vertical axis represents the concentration (pg/$1\times10^5$ cells) of those various factors. The concentration of MMP-2 (matrix metalloproteinase-2), IGFBP-4 (insulin-like growth factor binding protein-4), and cystatin C, from the left, with the intermediate cells (black bar) and the cells contained in the dental pulp-derived preparation (white bars). The values are means and the error bars indicate standard errors (n=3).

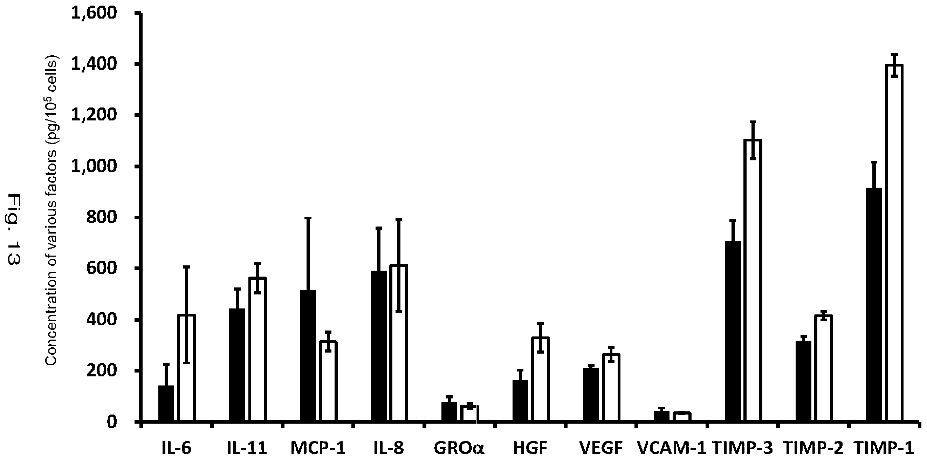

[Fig. 13] Fig. 13 is a graph showing the results of measurement of the concentration of various factors such as cytokines in the culture supernatant of the dental pulp-derived cells contained in the intermediate and the preparation obtained in Example 7 and Example 16, respectively, under no stimulation. The vertical axis represents the concentration (pg/$1\times10^5$ cells) of those various factors. , From the left, the concentration of IL-6 (interleukin-6), IL-11 (interleukin-11), MCP-1 (monocyte chemotactic protein-1), IL-8 (interleukin-8), GROα (human growth regulated protein alpha), HGF (hepatocyte growth factor), VEGF (vascular endothelial growth factor), VCAM-1 (vascular cell adhesion molecule-1), TIMP-3 (tissue inhibitor of metalloproteinase-3), TIMP-2 (tissue inhibitor of metalloproteinase-2), and TIMP-1 (tissue inhibitor of metalloproteinase-1) are shown with the intermediate cells (black bar) and the cells contained in the dental pulp-derived preparation (white bars). The values are means and the error bars indicate standard errors (n=3).

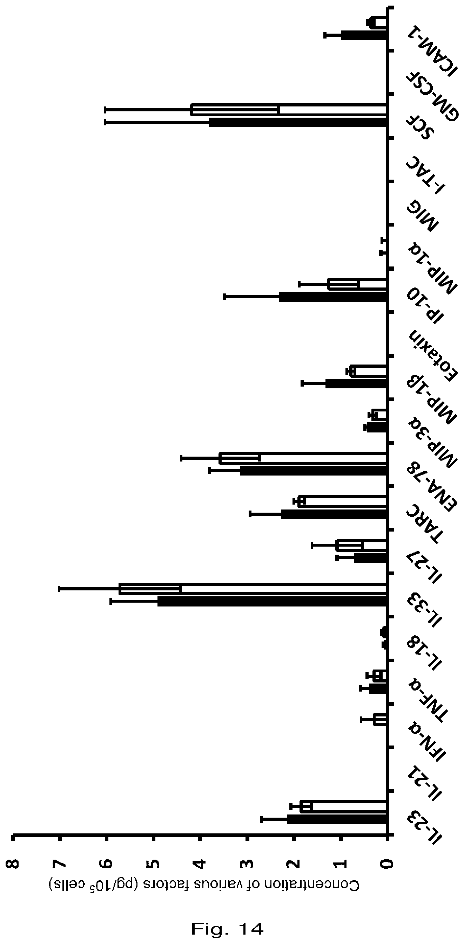

[Fig. 14] Fig. 14 is a graph showing the results of measurement of the concentration of various factors in the culture supernatant of the dental pulp-derived cells contained in the intermediate and the preparation obtained in Example 7 and Example 16, respectively, under no stimulation. The vertical axis represents the concentration (pg/$1\times10^5$ cells) of those various factors. From the left, the concentration of IL-23 (interleukin-23), IL-21(interleukin-21), IFN-α(interferon-α), TNF-α, IL-18 (interleukin-18), IL-33(interleukin-33), IL-27(interleukin-27), TARC (thymus and activation-regulated chemokine), ENA-78 (epithelial neutrophil-activating protein-78), MIP-3α (macrophage inflammatory protein-3α), MIP-1β(macrophage inflammatory protein-1β), Eotaxin, IP-10 (interferon-γ-inducible protein-10), MIP-1α (macrophage inflammatory protein-1α), MIG (monokine induced by gamma interferon), I-TAC (interferon-inducible t-cell alpha chemoattractant), SCF (stem cell factor), GM-CSF (granulocyte-macrophage colony-stimulating factor), and ICAM-1 (intercellular adhesion moleculue-1) are shown with the intermediate (black bar) and the preparation (white bars), respectively. The values are means and the error bars indicate standard errors (n=3).

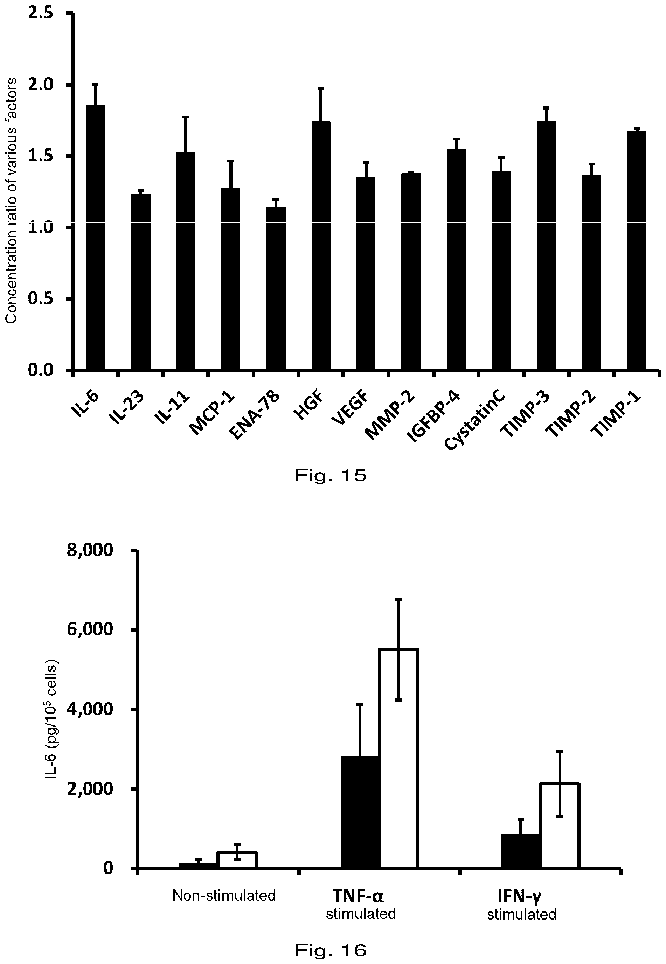

[Fig. 15] Fig. 15 is a graph showing concentration ratio of those factors such as cytokines (concentration in the preparation/concentration in the intermediate), between the intermediate and the preparation prepared in Example 7 and Example 16, in the supernatant of the culture of the cells contained in them, under no stimulation, and the expression amount of which were greater in the preparation than the intermediate. From the left, concentration ratios are shown of IL-6, IL-23, IL-11, MCP-1, ENA-78, HGF, VEGF, MMP-2, IGFBP-4, cystatin C, TIMP-3, TIMP-2, and TIMP-1. Values are means and error bars indicate standard errors (n=3).

[Fig. 16] Fig. 16 is a graph showing the concentration of IL-6 in the culture supernatant in the non-stimulated, the TNF-α-stimulated, and the IFN-γ-stimulated groups of the dental pulp-derived cells contained in the intermediate and the preparation obtained in Example 7 and example 16, respectively. The vertical axis represents concentration (pg/$1\times10^5$ cells). The concentration in the non-stimulated group, the TNF-α-stimulated group, and the IFN-γ-stimulated group are shown, from the left, with respect to the intermediate (black bars) and the preparation (white bars). Values are means and error bars indicate standard errors (n=3).

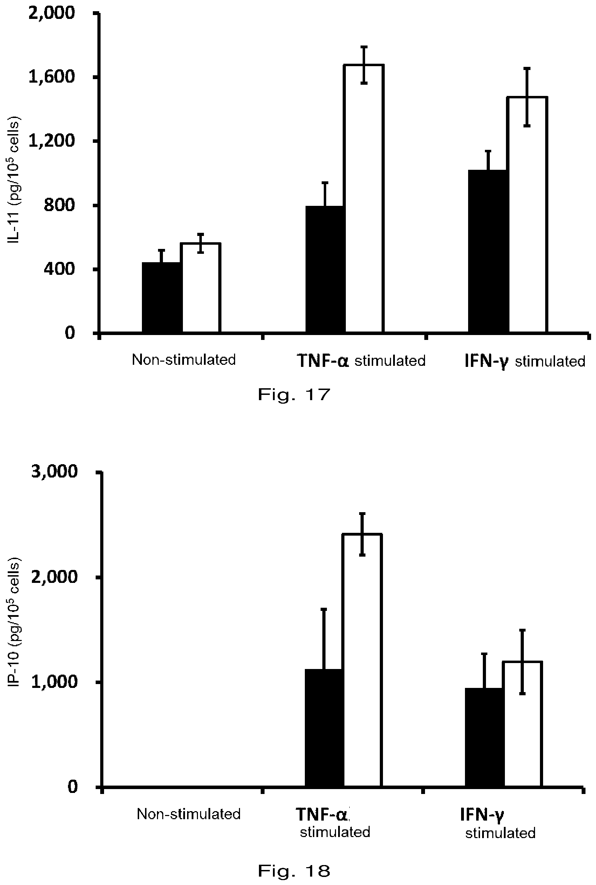

[Fig. 17] Fig. 17 is a graph showing the concentration of IL-11 in the culture supernatant in the non-stimulated, TNF-α-stimulated, and the IFN-γ-stimulated groups, of the dental pulp-derived cells contained in the intermediate and the preparation obtained in Example 7 and example 16, respectively. The vertical axis represents concentration (pg/$1\times10^5$ cells). From the left, the concentration in the non-stimulated group, the TNF-α-stimulated group, and the IFN-γ-stimulated group are shown, with respect to the intermediate (black bars) and the preparation (white bars). Values are means and error bars indicate standard errors (n=3).

[Fig. 18] Fig. 18 is a graph showing the concentration of IP-10 (CXCL 10) in the culture supernatant in the non-

stimulated, TNF-$\alpha$-stimulated, and the IFN-$\gamma$-stimulated groups, of the dental pulp-derived cells contained in the intermediate and the preparation obtained in Example 7 and example 16, respectively. The vertical axis represents concentration (pg/1×10$^5$ cells). From the left, the concentration in the non-stimulated group, the TNF-$\alpha$-stimulated group, and the IFN-$\gamma$-stimulated group are shown, with respect to the intermediate (black bars) and the preparation (white bars). Values are means and error bars indicate standard errors (n=3).

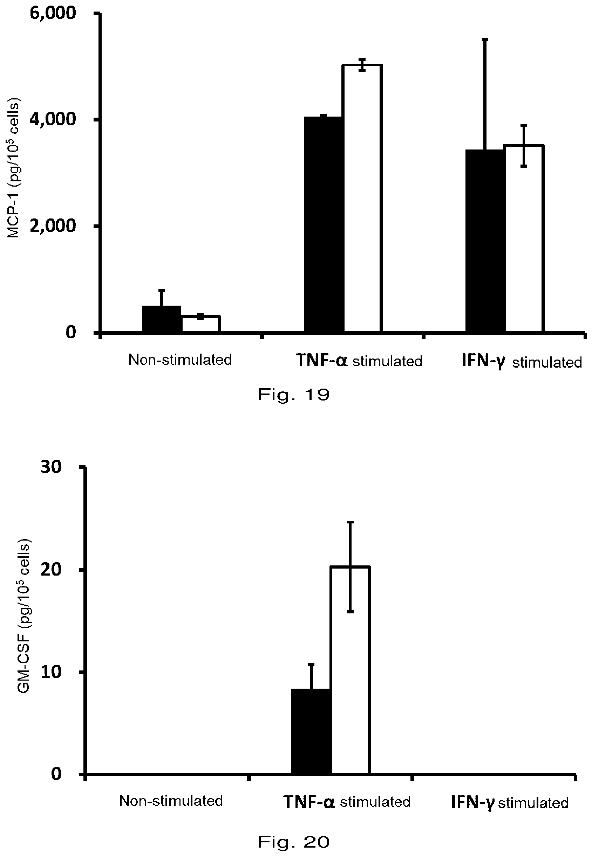

[Fig. 19] Fig. 19 is a graph showing the concentration of MCP-1 in the culture supernatant in the non-stimulated, TNF-$\alpha$-stimulated, and the IFN-$\gamma$-stimulated groups, of the dental pulp-derived cells contained in the intermediate and the preparation obtained in Example 7 and example 16, respectively. The vertical axis represents concentration (pg/1×10$^5$ cells). From the left the concentration in the non-stimulated group, the TNF-$\alpha$-stimulated group, and the IFN-$\gamma$-stimulated group are shown, with respect to the intermediate (black bars) and the preparation (white bars). Values are means and error bars indicate standard errors (n=3).

[Fig. 20] Fig. 20 is a graph showing the concentration of GM-CSF in the culture supernatant in the non-stimulated, TNF-$\alpha$-stimulated, and the IFN-$\gamma$-stimulated groups, of the dental pulp-derived cells contained in the intermediate and the preparation obtained in Example 7 and example 16, respectively. The vertical axis represents concentration (pg/1×10$^5$ cells). From the left, the concentration in the non-stimulated group, the TNF-$\alpha$-stimulated group, and the IFN-$\gamma$-stimulated group are shown, with respect to the intermediate (black bars) and the preparation (white bars). Values are means and error bars indicate standard errors (n=3).

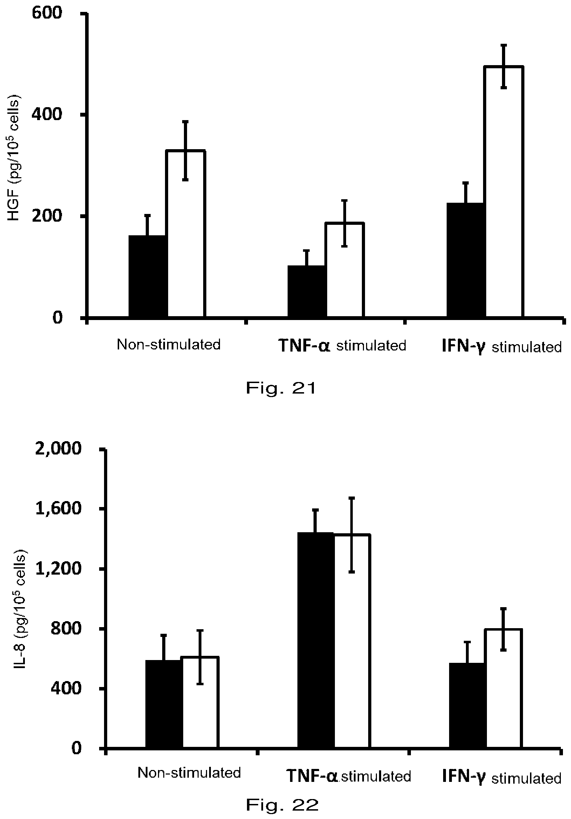

[Fig. 21] Fig. 21 is a graph showing the concentration of HGF in the culture supernatant in the non-stimulated, TNF-$\alpha$-stimulated, and the IFN-$\gamma$-stimulated groups, of the dental pulp-derived cells contained in the intermediate and the preparation obtained in Example 7 and example 16, respectively. The vertical axis represents concentration (pg/1×10$^5$ cells). From the left, the concentration in the non-stimulated group, the TNF-$\alpha$-stimulated group, and the IFN-$\gamma$-stimulated group are shown, with respect to the intermediate (black bars) and the preparation (white bars). Values are means and error bars indicate standard errors (n=3).

[Fig. 22] Fig. 22 is a graph showing the concentration of IL-8 in the culture supernatant in the non-stimulated, TNF-$\alpha$-stimulated, and the IFN-$\gamma$-stimulated groups, of the dental pulp-derived cells contained in the intermediate and the preparation obtained in Example 7 and example 16, respectively. The vertical axis represents concentration (pg/1×10$^5$ cells). From the left, the concentration in the non-stimulated group, the TNF-$\alpha$-stimulated group, and the IFN-$\gamma$-stimulated group are shown, with respect to the intermediate (black bars) and the preparation (white bars). Values are means and error bars indicate standard errors (n=3).

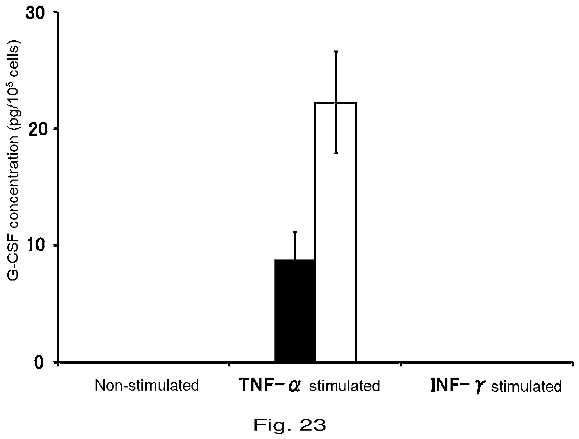

[Fig. 23] Fig. 23 is a graph showing the concentration of G-CSF in the culture supernatant in the non-stimulated, TNF-$\alpha$-stimulated, and the IFN-$\gamma$-stimulated groups, of the dental pulp-derived cells contained in the intermediate and the preparation obtained in Example 7 and example 16, respectively. The vertical axis represents concentration (pg/1×10$^5$ cells). From the left, the concentration in the non-stimulated group, the TNF-$\alpha$-stimulated group, and the IFN-$\gamma$-stimulated group are shown, with respect to the intermediate (black bars) and the preparation (white bars). Values are means and error bars indicate standard errors (n=3).

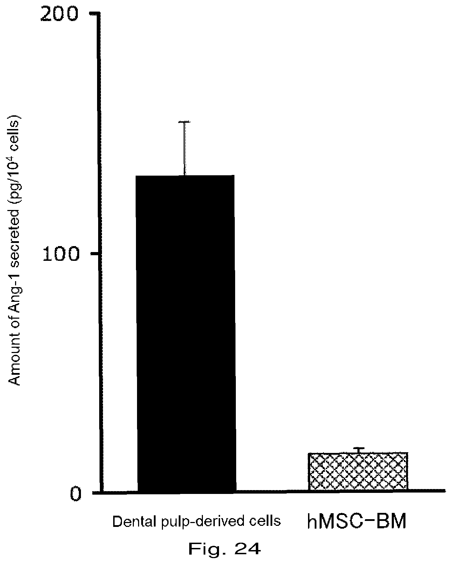

[Fig. 24] Fig. 24 is a graph showing the test results of the ability to secrete angiopoietin-1 (Ang-1) of the dental pulp-derived cells obtained in Example 16 and the human bone marrow-derived mesenchymal stem cells obtained in Comparative Example 1. The vertical axis represents the amount of angiopoioetin-1 (Ang-1) secreted. Black bars indicate the results in the dental pulp-derived cells, and shaded bars indicate the results in the human bone marrow-derived mesenchymal stem cells (hMSC-BM), respectively. Values are means and error bars indicate standard errors (n=3).

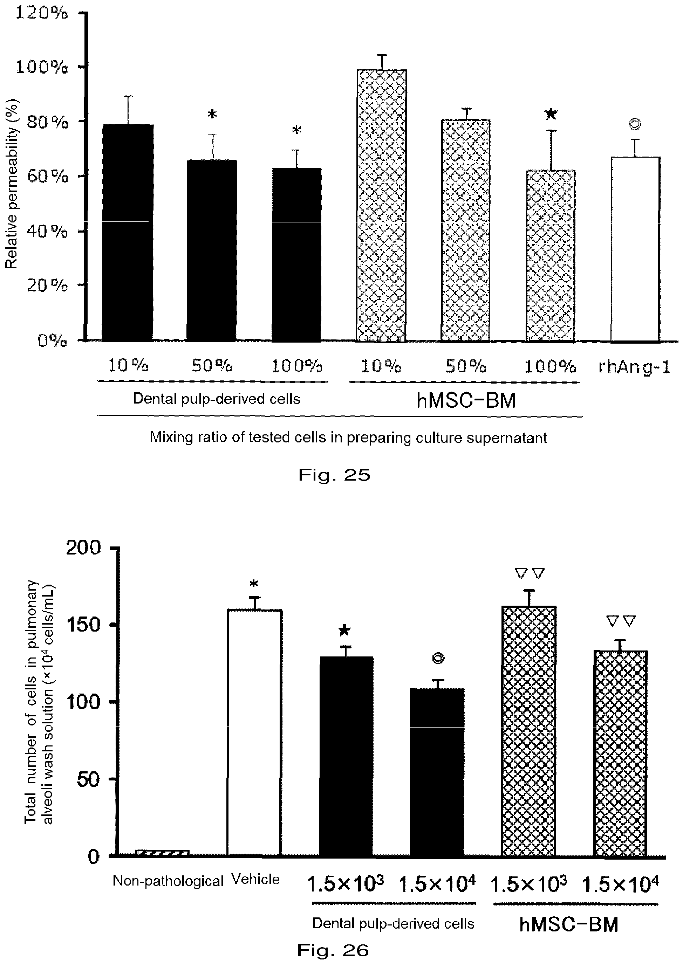

[Fig. 25] Fig. 25 is a graph showing the results of comparison of the effect on vascular permeability, measured utilizing human umbilical vein endothelial cells (HUVEC), between the dental pulp-derived cells obtained in Example 16 and the human bone marrow-derived mesenchymal stem cells (hMSC-BM) obtained in Comparative Example 1. The vertical axis represents relative permeability (%). As to the dental pulp-derived cells (black bars) and hMSC-BM (shaded bars), respectively, the result of addition of the supernatant of culture of tested cells (dental pulp-derived cells or hMSC-BM) and HUVEC, at the mixing ratio of 10%, 50%, and 100%, from the left, are shown, where white bars indicate the results with human recombinant angiopoietin-1 (rhAng-1). Values are means and error bars indicate standard errors (n=3-4). The result at the mixing ratio of 0% was used as the control, and the asterisk (*) denotes $p < 0.05$ vs. control in Dunnett's test, "*" denotes $p < 0.05$ vs. control in Dunnett's test, "◎" denotes $p < 0.01$ vs. control in Student's t test, respectively.

[Fig. 26] Fig. 26 is a graph showing the comparison of the effect on neutrophil infiltration into the lung of LPS-induced acute lung injury model mice between the dental pulp-derived cells obtained in Example 16 and that of the human bone marrow-derived mesenchymal stem cells (hMSC-BM) obtained in Comparative Example 1. The vertical axis represents the total number of cells (×10$^4$ cells/mL) in pulmonary alveoli wash solution. As to the dental pulp-derived cells (black bars) and hMSC-BM (shaded bars), respectively, the results of administration of those cells at doses of 1.5×10$^3$ cells and 1.5×10$^4$ cells, from the left respectively, are shown, where the hatched bar (left end) indicates the result in the non-pathological mice, the white bar indicates that in the LPS-induced acute lung injury model mice administered with the vehicle, respectively. Values are means and error bars indicate standard errors (n=5~6). The asterisk (*) denotes p<0.001 vs. non-pathological mice in Dunnett's test, "*" denotes p<0.05 vs. the LPS-induced acute lung injury model mice administered with the vehicle in Dunnett's test, "◎" denotes p<0.0001 vs. the LPS-induced acute lung injury model mice administered with the vehicle in Dunnett's test, "∇∇" denotes no significance vs. the LPS-induced acute lung injury model mice administered with the vehicle in Dunnett's test.

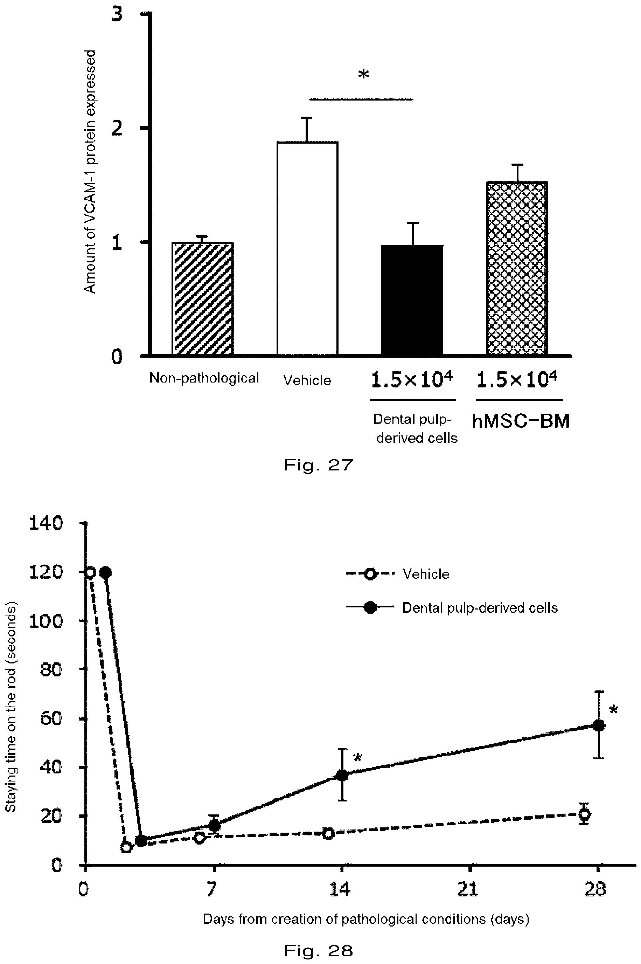

[Fig. 27] Fig. 27 is a graph showing the comparison of the effect on VCAM (Vascular cell adhesion molecule)-1 protein expression in the lung of LPS-induced acute lung injury model mice, between the dental pulp-derived cells obtained in Example 16 and that of the human bone marrow-derived mesenchymal stem cells (hMSC-BM) obtained in Comparative Example 1. The vertical axis represents the amount of VCAM-1 protein expressed. The black bar indicates the result in the LPS-induced acute lung injury model mice administered with the dental pulp-derived cells, the shaded bar indicates the result in the LPS-induced acute lung injury model mice administered with hMSC-BM, the hatched bar indicates the result in the non-pathological mice, and the white bar indicates results in the LPS-induced acute lung injury model mice administered with the vehicle, respectively. Values are means and error bars indicate standard errors (=5~6). The asterisk (*) denotes p<0.05 for the results in the LPS-induced acute lung injury model mice administered with the dental pulp-derived cells vs. the LPS-induced acute lung injury model mice administered with the vehicle, in Student's t-test.

[Fig. 28] Fig. 28 is a graph showing the assessment of restoration of neurological functions (Rota-rod test) with the dental pulp-derived cells obtained in Example 16, using rat cerebral infarction model. The vertical axis represents the staying time (seconds) on the rod, and the horizontal axis represents the number of days (days) from creation of pathological conditions. Black dots indicate the results in the infarction model rats administered with dental pulp-derived cells, and white dots in the infarction model rats administered with the vehicle, respectively. Values are means and error bars indicate standard errors (n=10). The asterisk (*) denotes p<0.05 vs. the results in the cerebral infarction model rats administered with the vehicle, in Student's t test.

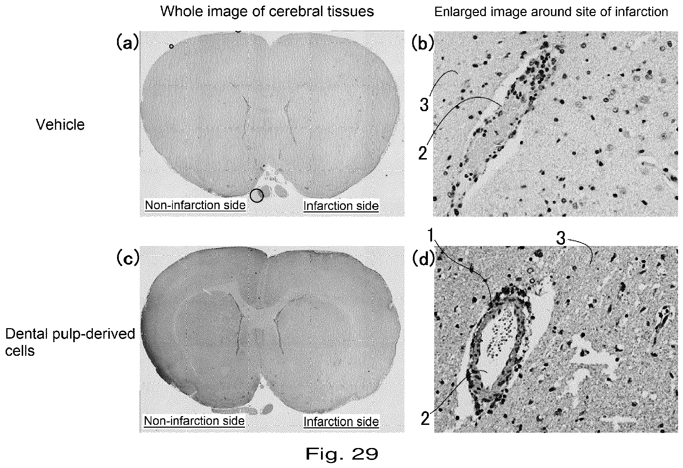

[Fig. 29] Fig. 29 is a figure showing, by comparison, the result with the dental pulp-derived cells obtained in Example 16, in the amount of angiopoietin-1 present in the cerebral tissues of cerebral infarction model rats, using immunostaining with anti-angiopoietin-1 antibody. A stained image of the whole cerebral tissue section is shown, as well as an enlarged stained image around a site of infarction, of a cerebral infarction model rat administered with the vehicle and a cerebral infarction model rat administered with the dental pulp-derived cells, respectively. It is indicated that stained cells are those positive for angiopoietin-1 (Ang-1).

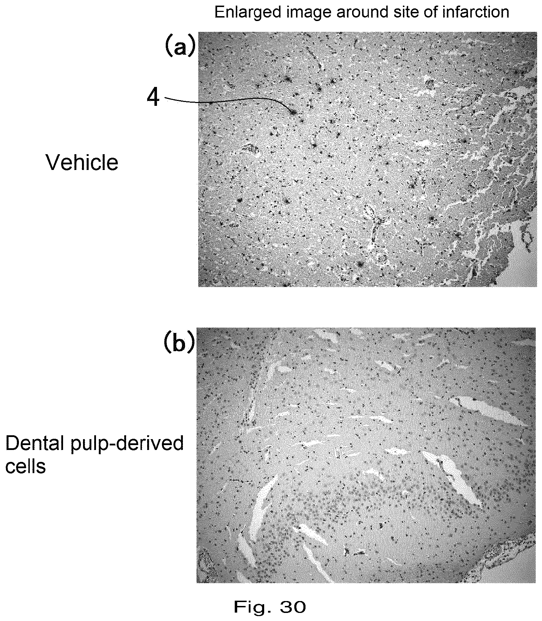

[Fig. 30] Fig. 30 shows the results of examination of the effect of the dental pulp-derived cells obtained in Example 16 on neutrocyte infiltration around a site of cerebral infarction in a cerebral infarction model rat using immunostaining with anti-myeloperoxidase (MPO) antibody. Typical enlarged stained images around a site of infarction are shown of a cerebral infarction model rats administered with the vehicle and a cerebral infarction model rats administered with the dental pulp-derived cells, respectively. It is indicated that stained cells are those positive for MPO.

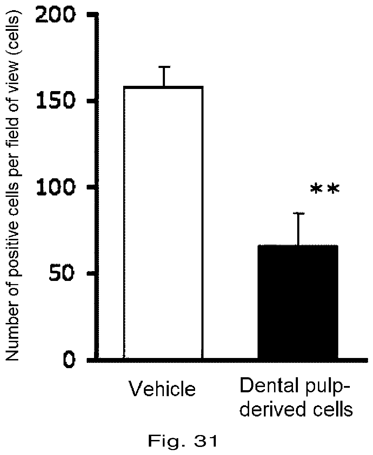

[Fig. 31] Fig. 31 is a graph showing the effect of the dental pulp-derived cells obtained in Example 16 on neutrophil infiltration into the area surrounding a site of cerebral infarction in a cerebral infarction model rat, by quantifying and comparing the number of cells found positive using immunostaining with anti-myeloperoxidase (MPO) antibody. The vertical axis represents the number of positive cells (cells) per field of view. Values are means and error bars indicate standard errors (n=6-7). The black bar indicates the result in the cerebral infarction model rats administered with the dental pulp-derived cells, and the white bar the cerebral infarction model rats administered with the vehicle. Values are means and error bars indicate standard errors (n=6-7). Double-asterisks (**) denotes p<0.01 vs. the cerebral infarction model rats administered with the vehicle in Student's t test.

DESCRIPTION OF EMBODIMENTS

Multipotent stem cells are those cells which have capacities not only to self-replicate but also differentiate into two or more kinds of cells. The human dental pulp-derived multipotent stem cells obtained by the present invention preferably, but without limitation, have abilities to differentiate into chondrocytes and osteocytes.

That the multipotent stem cells obtained by the present invention have an ability to differentiate into chondrocytes can be confirmed by, e.g., measuring the expression levels of aggrecan genes after the cells are cultured in a medium containing a compound known to induce differentiation of cells into chondrocytes. Aggrecans are primary molecules building the extracellular matrix of cartilage, and the levels of expression of aggrecans increase when cells are induced to differentiate into chondrocytes. In the method of the above measurement, an example of compounds which can be used to induce cells to differentiate into chondrocytes (chondrocyte differentiation-inducer compounds) is TGF-b3. The method for measurement described in Example 20 is a preferable example for measuring an ability of cells to differentiate into chondrocytes.

That the multipotent stem cells obtained by the present invention have an ability to differentiate into osteocytes can be confirmed by, e.g., measuring the amount of calcium accumulated in the cells after their culture in a medium containing a compound known to induce differentiation of cells into osteocytes. When induced to differentiate into osteocytes, calcium accumulates in the cells. In the method for above measurement, examples of the compounds which can be used to induce cells to differentiate into osteocytes (osteocytes differentiation-inducer compound) includes dexamethasone, β-glycerophosphate, glycosaminoglycans, ascorbic acid, bone morphogenetic protein 2 (BMP-2), and their salts, any of which can be used alone or in combination. For example, a combination of dexamethasone, ascorbic acid, and β-glycerophosphate can be preferably used as an osteocyte differentiation-inducer. The method for measurement described in Example 19 is a preferable example for measuring an ability of cells to differentiate into osteocytes.

In the present invention, the phrase, "dental pulp-derived cells which have been enriched in multipotent stem cells" or "multipotent stem cell-enriched dental pulp-derived cells" means a population of cells that have been derived from dental pulp and contain multipotent stem cells at a proportion (proportion in number) higher than cells directly collected from dental pulp, and include multipotent stem cells finally isolated through selective culture or the like starting from dental pulp. Cells directly collected from dental pulp consist of a mixture of multipotent stem cells and other cells. As multipotent stem cells have a higher ability to proliferate than other cells, performing culture of those directly collected cells will give at the end culture, an increased proportion of multipotent stem cells as compared with the cells at the start of culture. Because repeating culture cycles will further increase the proportion of multipotent stem cells, it is also possible to prepare cells consisting almost entirely of multipotent stem cells. Thus, "multipotent stem cell-enriched dental pulp-derived cells" includes a population of cells containing increased proportion of multipotent stem cells compared with those cells directly collected from dental pulp, as a result of proliferation of multipotent stem cells in the process of culture and the like. Where human dental pulp is employed, the resulting cells are called "multipotent stem cell-enriched human dental pulp-derived cells".

In the present invention, both multipotent stem cells directly isolated from dental pulp and multipotent stem cells that are contained in multipotent stem cell-enriched dental pulp-derived cells that are obtained from dental pulp through culture are collectively called "dental pulp-derived multipotent stem cells", and also simply called "dental pulp-derived stem cells" or "dental pulp stem cells", when context allows. Where human dental pulp is employed, the resulting cells are specifically called "human dental pulp-derived multipotent stem cells" and also simply called "human dental pulp derived stem cells" or "human dental pulp stem cells", too. "Cells contained in an intermediate (intermediate cells)" and "cells contained in a dental pulp-derived cell preparation", which are embodiments described in the present specification, substantially consist only of multipotent stem cells, and both are "dental pulp-derived multipotent stem cells.

In the present invention, when a cell population tested for expression pattern of surface antigen markers is called positive for a certain antigen, it is meant that in an observation of the cell population as a whole, the cells positive for the said antigen represent preferably not less than 30%, more preferably not less than 40%, further preferably not less than 50%, still more preferably not less than 60%, still more preferably not less than 70%, still more preferably not less than 80%, still more preferably not less than 90%, and particularly preferably not less than 95% of the cells observed. The expression pattern of surface antigens can be determined by the method described in Example 21 or 22, for example but without limitation.

In the present invention, when a cell population tested for expression pattern of surface antigen markers is called negative for a certain antigen, it is meant that in an observation of the cell population as a whole, the cells positive for the said antigen represent preferably less than 20%, more preferably less than 10%, still more preferably less than 5%, still more preferably less than 2%, and particularly less than 1% of the cells observed. The expression pattern of surface antigens can be determined by the method described in Example 21 or 22, for example but without limitation.

When cultured in a medium containing a compound known to induce differentiation into chondrocytes, the multipotent stem cell-enriched dental pulp-derived cells of the present invention are observed as a whole to exhibit increased levels of aggrecan expression. Further, when cultured in a medium containing a compound known to induce differentiation into osteocytes, the multipotent stem cell-enriched dental pulp-derived cells of the present invention are observed as a whole to exhibit an increase in the amount of accumulated calcium in the cells. Thus, the multipotent stem cell-enriched dental pulp-derived cells of the present invention have abilities to differentiate into chondrocytes and osteocytes, when observed as a whole.

Neutrophils, monocytes, and lymphocytes gather at a site of inflammation due to chemotactic factors such as cytokines expressed in the site. These cells reach the site of inflammation by infiltering into the tissues from blood vessels in or near the site of inflammation. The pharmaceutical composition of the present invention is aimed to suppress infiltration of monocytes and lymphocytes into tissues, and specifically to suppress tissue infiltration of those cells which can bind to adhesion factors occurring on the vesicular endothelial cells. A neutrophil is an example of such a cell as can bind to adhesion factors occurring on the surface of vascular endothelial cells.

No particular limitation is put on adhesion factors in the present invention, and for example they are those which at least any of neutrophils, monocytes, and lymphocytes can bind to, including VCAM-1 as one of them. Neutrophils are a type of cells which can bind to VCAM-1.

In the present invention, while "tissue" particularly indicates a tissue having inflammation (site of inflammation) or a site of inflammation and surrounding tissues, no limitation is set as far as the pharmaceutical composition is to exert its effect there. The pharmaceutical composition in the present invention administered before the onset of inflammation, for example, can prevent the onset, or lessen the symptoms, of inflammation. Thus, the term "tissues" include those tissues before the onset of inflammation. Further, tissues may be any of endodermal, mesodermal, or ectodermal tissues, and, in particular, brain tissues, dermal tissues, digestive organs, circulatory organs, and respiratory apparatus.

In an embodiment of the present invention, a pharmaceutical composition is aimed to suppress expression of an adhesion factor in a tissue. For example, the pharmaceutical composition increases the tissue concentration of a factor that suppresses expression of the adhesion factor. An example of such a factor is angiopoietin-1.

The dental pulp-derived stem cells used in the present invention show higher expression levels of angiopoietin-1 (Ang-1) than human bone marrow-derived mesenchymal stem cells, which also are multipotent stem cells, and therefore their administration can lead to increased Ang-1 levels in the body, thereby exhibiting higher therapeutic effect.

As the composition of the present invention, when administered to a human, induces Ang-1 secretion from its pharmaceutically active principle, i.e., the dental pulp-derived stem cells, the composition is used for the treatment of those disorders which progress by excessively increased permeability of vascular endothelium, infiltration of cells, such as neutrophils and the like, into tissues, and expression of adhesion factors.

Specific examples of disorders to which the invention is applied include, without limitation, cerebral infarction, cerebral bleeding, retinopathy accompanied by macular degeneration, acute lung injury, asthma, acute pancreatitis, myocardial infarction, heart failure, dermatitis, and scepsis.

In cerebral infarction, following its onset, decrease in expression levels of angiopoietin-1 (Ang-1) and excessive increase of permeability of endothelial cells trigger neutrophil infiltration into tissues, which bring about disruption of the blood-brain barrier (BBB) and accelerate progression of cerebral microcirculation disturbance, and through induction of expression of adhesion factors, chemotaxis of monocytes, leucocytes, and platelets into tissues are accelerated, and accumulation of those inflammatory cells leads to development of the nest of infarction.

In cerebral bleeding, vascular injury, and angiitis, leucocytes adhere to vascular walls due to excessive expression of adhesion factors, pass across it and infiltrate surrounding tissues, further aggravating inflammation. In retinopathy accompanied by macular degeneration, where a relation between its onset and adhesion factors is suggested, recruitment of inflammatory cells aggravates its symptoms. In acute lung injury and asthma, excessively increased permeability of vascular endothelium causes edema of respiratory tract mucosa, and brings about excessive expression of adhesion factors on bronchial walls, which then causes neutrophils to migrate to lung tissues and lung alveoli system and adhere to vascular endothelial cells, releasing active oxygen to bring about lung injury. In acute pancreatitis, activated pancreatic enzymes activate neutrophil esterase, which causes remote organ injuries, and due to excessive expression of adhesion factors, neutrophils come to adhere to vascular endothelial cells and release peroxides there, thus injuring vascular endothelial cells. In myocardial infarction and heart failure, neutrophils accumulate at the site of infarction at the earliest stage of AMI (Acute myocardial infarction) and boost inflammation, and excessive expression of adhesion factors worsens arterial sclerosis there. In sepsis, it is known that edema and dehydration of organs occur due to excessive increase of permeability of vascular endothelium, and infiltration of neutrophils leads to formation of microabscesses, and tissue factors and adhesion factors expressed on vascular endothelium form the pathological state. Further, in dermatitis and the like, neutrophil infiltration accelerate inflammation, where symptoms are aggravated through accumulation of inflammatory cells such as neutrophils caused by excessive expression of adhesion factors.

The pharmaceutical composition of the present invention is applied to the treatment of various disorders which are advanced by such excessive permeability of vascular endothelium, infiltration of cells such as neutrophils, and excessive expression of adhesion factors.

In an embodiment, the pharmaceutical composition of the present invention is effective to suppress the expression levels of VCAM-1. VCAM-1 is an adhesion factor expressed on the surfaces of vascular endothelial cells. Cells like neutrophils, lymphocytes, and monocytes can adhere to vascular endothelial cells via VCAM-1. Therefore, some of those cells, such as neutrocytes for example, accumulate and infiltrate in a site where expression levels of VCAM-1 is increased.

As specifically described in Example 33, expression levels of VCAM-1 are increased in the injured site of the lung of acute lung injury mouse models. And the pharmaceutical composition of the present invention is shown to be effective to suppress the expression levels of VCAM-1. By suppressing expression levels of VCAM-1, adhesion of cells such as neutrophils, lymphocytes, and monocytes to vascular endothelial cells is also suppressed.

Therefore, the pharmaceutical composition of the present invention can be generally used as a therapeutic agent (including prophylactic agent) for disorders accompanied by acute lung injury. Examples of such disorders include aspiration pneumonitis, bacterial pneumonitis, viral pneumonitis, acute respiratory distress syndrome (also written as ARDS), and interstitial pneumonia. Among these, it is known that neutrophils infiltrate into the site of injury in bacterial pneumonitis, and that lymphocytes infiltrate into the site of injury in viral pneumonitis.

While there is no limitation as to bacterial pneumonitis to be treated with the pharmaceutical composition of the present invention, its examples include bacterial pneumonitis caused by *Streptococcus pneumoniae,* or *Staphylococcus aureus.*

While there is no limitation as to viral pneumonitis to be treated with the pharmaceutical composition of the present invention, its examples include viral pneumonitis caused by corona virus, influenza virus, and RS virus. Corona virus here includes SARS-CoV, MARS-CoV, and SARS-CoV-2. The novel coronavirus infection (COVID-19), which is caused by infection with SARS-CoV-2, is a disorder to be treated with the pharmaceutical composition of the present invention.

In an embodiment of the present invention, the multipotent stem cell-enriched dental pulp-derived cells express angiopoietin-1 (Ang-1) in an amount of 86-161 pg/$10^4$ cells, which amount of expression is preferably not less than 3 times, more preferably not less than 5 times, and still more preferably not less than 8 times greater than human bone marrow-derived mesenchymal stem cells, which also are multipotent stem cells. The amount of angiopoietin-1 expressed is measured by, e.g., the method described in Example 30.

In an embodiment of the present invention, the amount of angiopoietin-1 (Ang-1) produced by the multipotent stem cell-enriched dental pulp-derived cells is preferably not less than 3 times, more preferably not less than 5 times, still more preferably not less than 6 times, and still more preferably not less than 8 times greater than the amount of angiopoietin-1 (Ang-1) produced by human bone marrow-derived mesenchymal stem cells, when the dental pulp-derived stem cells and the human bone marrow-derived mesenchymal stem cells are respectively cultured. The method for culturing those cells employed here is the one described in, e.g., Example 30.

In an embodiment, when observed as a whole (population) with respect to their expression pattern of surface antigen markers, the multipotent stem cell-enriched dental pulp-derived cells of the present invention are positive for at least one of CD73, CD90, CD105, and CD166. Likewise, the multipotent stem cell-enriched dental pulp-derived cells of the present invention are negative for at least one of CD34 and CD45. For example, when observed as a whole with respect to their expression pattern of surface antigen markers, the multipotent stem cell-enriched dental pulp-derived cells of the present invention are positive for CD73 and CD90, while negative for CD34. Further, when observed as a whole with respect to their expression pattern of surface antigen markers, the multipotent stem cell-enriched dental pulp-derived cells of the present invention are positive for CD73, CD90, CD105, and CD166, while negative for CD34 and CD45. This expression patten is in common with mesenchymal stem cells.

Furter, in an embodiment, when observed as a whole with respect to their expression pattern of surface antigen markers, the multipotent stem cell-enriched dental pulp-derived cells of the present invention are negative for at least one of CD40, CD80, CD86, and MHC-class II antigen. For example, when observed as a whole with respect to their expression pattern of surface antigen markers, the multipotent stem cell-enriched dental pulp-derived cells of the present invention are negative for CD40, CD80, CD86, MHC-class II antigen. It is known that those cells which are positive for these surface antigen markers, when implanted in an allogeneic individual, tend to be excluded from the living body by being recognized as an antigen. Being negative for at least one of these surface antigen markers indicates that the multipotent stem cell-enriched dental pulp-derived cells of the present invention have correspondingly low antigenicity and therefore less tend to be excluded from the living body when implanted in an allogenic individual. And with respect to their expression pattern of these surface antigen markers, CD40, CD80, and CD86 remain negative when stimulated with IFN-γ, while MHC-class II antigen may turn positive. For example, when stimulated with IFN-γ, CD40, CD80, and CD86 remain negative, while MHC-class II antigen turns positive.

In an embodiment, when observed as a whole with respect to their expression pattern of surface antigen markers, the multipotent stem cell-enriched dental pulp-derived cells of the present invention are positive for, e.g., CD73, CD90, CD105, and CD166, while negative for CD34, CD40, CD45, CD80, CD86, MHC-class II antigen. Wherein when stimulated with IFN-γ, CD40,CD80, and CD86 remain negative, while MHC-class II antigen may turn positive.

In an embodiment, the multipotent stem cell-enriched dental pulp-derived cells (dental pulp-derived multipotent stem cells) of the present invention are
(a-1) when observed as a whole with respect to their expression pattern of surface antigen markers, positive for at least one of, and preferably for all of CD29, CD46, CD47, CD59, CD73, CD81, CD90, CD147, and HLA-A, B, C. Wherein, when observation is made cell-by-cell in the whole cell population, preferably not less than 70%, more preferably not less than 80%, still more preferably not less than 90% of observed cells are positive for these antigens.

Further, in an embodiment, the multipotent stem cell-enriched dental pulp-derived cells of the present invention are
(a-2) when observed as a whole with respect to their expression pattern of surface antigen markers, positive for at least one of, and preferably for all of CD9, CD44, CD49b, CD49c, CD55, CD98, EGF-R. Wherein, when observation is made cell-by-cell in the whole cell population, preferably not less than 65%, more preferably not less than 75%, and still more preferably not less than 80% of observed cell are positive for these antigens.

Further, in an embodiment, the multipotent stem cell-enriched dental pulp-derived cells of the present invention are
(a-3) when observed as a whole with respect to their expression pattern of surface antigen markers, positive for at least one of, and preferably for all of CD49f, CD140b, and CD166. Wherein when observation is made cell-by-cell in the whole cell population, preferably not less than 60%, more preferably not less than 70%, still more preferably not less than 75% of observed cells are positive for these antigens.

Further, in an embodiment, the multipotent stem cell-enriched dental pulp-derived cells of the present invention are

(a-4) when observed as a whole with respect to their expression pattern of surface antigen markers, positive for at least one of, and preferably for all of CD10, CD13, CD58, CD63, CD105, CD151, and CD164. Wherein, when observation is made cell-by-cell in the whole cell population, preferably not less than 60%, more preferably not less than 65%, and still more preferably not less than 70% of observed cells are positive for these antigens.

In an embodiment, the multipotent stem cell-enriched dental pulp-derived cells of the present invention have preferably not less than 2, more preferably not less than 3, and still more preferably all of the characteristics (a-1), (a-2), (a-3), and (a-4) defined above. For example, cells having the characteristics (a-1) and (a-2) defined above are one of the preferable embodiments of the present invention.

In an embodiment, the multipotent stem cell-enriched dental pulp-derived cells of the present invention are (a-5) when observed as a whole with respect to their expression pattern of surface antigen markers, negative for at least one of, and preferably for all of CD120b, CD132, CD158a, CD161,CD184, CD195, CD206, CD210, CD212, CD226, CD244, CD267, CD278, CD279, CD282, CD294, SSEA-1 (stage-specific embryonic antigen-1), TRA-1-60, SSEA-3(stage-specific embryonic antigen-3), CLA (cutaneous lymphocyte-associated antigen), and integrin $\beta$7. Wherein, when observation is made cell-by-cell in the whole cell population, preferably only not more than 10%, more preferably not more than 5%, still more preferably not more than 2%, and still more preferably not more than 1% of observed cells are positive for these antigens.

In an embodiment, the multipotent stem cell-enriched dental pulp-derived cells of the present invention are (a-6) when observed as a whole with respect to their expression pattern of surface antigen markers, negative for at least one of, and preferably for all of CD8b, CD11b, CD15s, CD16, CD19, CD24, CD31, CD32, CD62E, CD62P, CD66f, CD86, CD88, CD94, CD100, CD103, CD114, CD117, CD118, CD121b, CD122, CD123, CD124, CD126, CD127, CD128b, CD135, CD137, CD137 ligand, CD150, CD163, CD172b, CD177, CD178, CD180, CD197, CD220, CD229, CD231, CD255, CD268, CD305, CD314, CD321, CDw327, CDw328, CD329, CD335, CD336, BLTR-1 (leukotriene B4 receptor), CLIP,CMRF-44,CMRF-56,fMLP-R (N-formylmethionyl-leucyl-phenylalanine receptor), Invariant NKT, and $\gamma\delta$ TCR ($\gamma$ $\delta$T cell receptor). Wherein, when observation is made cell-by-cell in the whole cell population, preferably only not more than 10%, more preferably not more than 5%, and still more preferably not more than 2% of observed cells are positive for these antigens.

In an embodiment, the multipotent stem cell-enriched dental pulp-derived cells of the present invention are (a-7) when observed as a whole with respect to their expression pattern of surface antigen markers, negative for at least one of, and preferably for all of CD1a, CD1b, CD1d, CD2, CD3, CD5, CD6, CD7, CD8a, CD11c, CD15, CD18, CD21, CD22, CD23, CD25, CD26, CD27, CD28, CD33, CD34, CD35, CD37, CD38, CD40, CD41a, CD41b, CD42b, CD45, CD45RB, CD45RO, CD48, CD50, CD53, CD62L, CD64, CD66(a, c, d, e), CD69, CD70, CD72, CD80, CD84, CD85, CD87, CD89, CDw93, CD97, CD106, CD134, CD138, CD144, CD154, CD158b, CD162, CD183, CD205, CD235a, CD271, CD309, CD326, CD337, $\alpha\beta$ TCR ($\alpha\beta$T cell receptor), and MHC-class II antigen. Wherein, when observation is made cell-by-cell in the whole cell population, preferably only not more than 10%, and more preferably not more than 5% of observed cells are positive for these antigens.

In an embodiment, the multipotent stem cell-enriched dental pulp-derived cells of the present invention have preferably not less than 2, more preferably not less than 3, and still more preferably all of the characteristics (a-1), (a-2), (a-3), (a-4), (a-5), (a-6) and (a-7) described above. For example, cells having the characteristics (a-1) and (a-5) described above are one of the preferable embodiments of the present invention.

In an embodiment, the multipotent stem cell-enriched dental pulp-derived cells of the present invention, when observed as a whole, express prostaglandin $E_2$ (PGE$_2$) and/or vascular endothelial growth factor (VEGF), wherein the expression levels of prostaglandin $E_2$ (PGE$_2$) increases when the cells are stimulated with TNF$\alpha$. As VEGF is a compound having an angiogenetic action, the multipotent stem cell-enriched dental pulp-derived cells, when administered to a human, can spur vascularization in the body via VEGF, and are therefore useful as a therapeutic agent for disorders where an angiogenetic action should take place. Further, as PGE$_2$ has a potent anti-inflammatory effect, the multipotent stem cell-enriched dental pulp-derived cells, when administered to a human, act on the site of inflammation in the body via PGE$_2$ and suppress tissue destruction which accompanies inflammation, and are therefore useful as a suppressor agent of tissue destruction accompanying inflammation. However, the effects of the multipotent stem cell-enriched dental pulp-derived cells are not limited to those taking place via VEGF and PGE$_2$.

In an embodiment of the present invention, the multipotent stem cell-enriched dental pulp-derived cells, when cultured in the presence of IFN-$\gamma$, exhibit an increase in the amount of kynurenine secreted. Kynurenine can suppress proliferation of T cells and induce differentiation of monocytes into macrophages. Therefore, the multipotent stem cell-enriched dental pulp-derived cells, when administered to a human, can exhibit anti-inflammatory effects via kynurenine.

In an embodiment of the present invention, when observed as a whole with respect to their expression pattern of surface antigen markers, the multipotent stem cell-enriched dental pulp-derived cells are positive for at least one of, or for all of CD47, CD81, and CD147, while negative for at least one of, for all of CD19, CD34, and CD206. Further, in an embodiment of the present invention, the multipotent stem cell-enriched dental pulp-derived cells, when observed as a whole with respect to their expression pattern of surface antigen markers, are positive for at least one of, or for all of CD47, CD81, and CD147, while negative for at least one of, or for all of CD19, CD31, CD33, CD34, CD38, CD45, CD206, CD235a, and SSEA-1.

In an embodiment of the present invention, the multipotent stem cell-enriched dental pulp-derived cells, when observed as a whole with respect to their expression pattern of surface antigen markers, are negative for at least one of, or, e.g., for all of CD19, CD26, CD106, CD117, and CD271.

In an embodiment of the present invention, the multipotent stem cell-enriched dental pulp-derived cells, when observed as a whole with respect to their expression pattern of surface antigen markers, are positive for at least one of, or, e.g., for all of CD140b, and HLA-A, B, C.

In an embodiment of the present invention, the multipotent stem cell-enriched dental pulp-derived cells, when observed as a whole with respect to their expression pattern of surface antigen markers, are positive for at least one of, or, e.g., for all of CD10, CD46, CD47, CD55, CD58, and CD59.

In an embodiment of the present invention, the multipotent stem cell-enriched dental pulp-derived cells, when observed as a whole with respect to their expression pattern of surface antigen markers, are positive for, e.g., at least one of CD73, CD90, CD105, and CD166, while negative for at least one of CD34 and CD45.

In an embodiment of the present invention, the multipotent stem cell-enriched dental pulp-derived cells, when observed as a whole with respect to their expression pattern of surface antigen markers, are positive for CD73, CD90, CD105, and CD166, while negative for CD34, CD40, CD45, CD80, CD86, and MHC-class II antigen. Wherein, when stimulated with IFN-$\gamma$, CD40, CD80, and CD86 remain negative, for example, while MHC-class II antigen turns positive. And the cells express prostaglandin $E_2$ and/or vascular endothelial growth factor (VEGF), wherein the amount of secreted prostaglandin $E_2$ increases when the cells are stimulated with TNF$\alpha$. Further, stimulation with IFN-$\gamma$ increase the amount of secreted kynurenine.

When cultured, the multipotent stem cell-enriched dental pulp-derived cells of the present invention secrete various humoral factors.

(a-8) In an embodiment, when cultured, the multipotent stem cell-enriched dental pulp-derived cells express at least one of, and preferably all of MMP-2, IGFBP-4, and cystatin C.

(a-9) In an embodiment, when cultured, the multipotent stem cell-enriched dental pulp-derived cells express at least one of, and preferably all of IL-6, IL-11, MCP-1, IL-8, GRO$\alpha$, HGF, VEGF, VCAM-I, TIMP-3, TIMP-2, and TIMP-1.

(a-10) Further, when cultured, the multipotent stem cell-enriched dental pulp-derived cells express at least one of, and preferably all of IL-23, TNF-$\alpha$, IL-18, IL-33, IL-27, TARC, ENA-78, MIP-3$\alpha$, MIP-1$\beta$, IP-10(CXCL10), SCF, and ICAM-1.

(a-11) Further, in an embodiment, when cultured, the multipotent stem cell-enriched dental pulp-derived cells express no, or almost no IL-21, eotaxin, MIP-1$\alpha$, MIG, I-TAC, or GM-CSF.

(a-12) Further, in one embodiment, when cultured in the presence of TNF-$\alpha$ or IFN-$\gamma$, the multipotent stem cell-enriched dental pulp-derived cells express increased levels of IL-6 as compared with their culture in the absence of these.

(a-13) Further, in an embodiment, the multipotent stem cell-enriched dental pulp-derived cells express increased levels of IL-11 when cultured in the presence of TNF-$\alpha$ or IFN-$\gamma$ as compared with their culture in the absence of these.

(a-14) Further, in an embodiment, the multipotent stem cell-enriched dental pulp-derived cells express increased levels of IP-10 (CXCL 10) when cultured in the presence of TNF-$\alpha$ or IFN-$\gamma$ as compared with their culture in the absence of these.

(a-15) Further, in an embodiment, the multipotent stem cell-enriched dental pulp-derived cells express increased levels of MCP-1 when cultured in the presence of TNF-$\alpha$ or IFN-$\gamma$ as compared with their culture in the absence of these.

(a-16) Further, in an embodiment, the multipotent stem cell-enriched dental pulp-derived cells are induced to express GM-CSF when cultured in the presence of TNF-$\alpha$, whereas not induced to express GM-CSF when cultured in the presence of IFN-$\gamma$.

(a-17) Further, in an embodiment, the multipotent stem cell-enriched dental pulp-derived cells show decreased expression levels of HGF when cultured in the presence of TNF-$\alpha$ as compared with their culture in its absence, whereas the cells show increased expression levels of HGF when cultured in the presence of IFN-$\gamma$ as compared with when cultures in its absence.

(a-18) Further, in an embodiment, the multipotent stem cell-enriched dental pulp-derived cells, when cultured in the presence of TNF-$\alpha$, shows increased expression levels of IL-8, as compared with their culture in its absence.

(a-19) Further, in an embodiment, the multipotent stem cell-enriched dental pulp-derived cells are induced to express G-CSF when cultured in the presence of TNF-$\alpha$, whereas not induced to express G-CSF when cultured in the presence of IFN-$\gamma$.

In an embodiment, the multipotent stem cell-enriched dental pulp-derived cells of the present invention have preferably not less than 2, more preferably not less than 3, still more preferably not less than 4, and still more preferably

EP 4 098 267 A1

all of the characteristics (a-8) to (a-19) defied above. For example, cells having the characteristics (a-8), (a-14), and (a-19) defined above and cells having the characteristics (a-8), (a-13), (a-14), and (a-19) defined above are a preferred embodiment of the present invention.

The multipotent stem cell-enriched dental pulp-derived cells of the present invention may consist almost entirely of dental pulp-derived multipotent stem cells. In many cases, dental pulp-derived multipotent stem cells during plate culture are observed as generally spindle-shaped cells adhering to a cell culture plate under an optical microscope. Depending on culture condition, however, they are not necessarily observed as spindle-shaped adhering cells.

In the present invention, the term "dental pulp" means loose fibrous connective tissues consisting of connective tissue containing blood vessels, nerves, and lymph vessels, and layers of odontoblasts in the peripheral area, which have the ability to cause dentin deposition from inside and to repair dentin. Further, while dental pulp can be divided into coronal pulp and radicular pulp depending on their location, the term dental pulp in the present invention encompasses at least one of them. Furthermore, though there is no particular limitation as to the animal species from which the dental pulp employed in the present invention is derived, such animal is preferably a mammal, and more preferably a primate, and still more preferably a human.

A tooth from which dental palp is to be obtained may be ether a permanent tooth or a deciduous tooth, and may be any of a cutting tooth, canine tooth, premolar tooth, or a molar tooth. Such a tooth is preferably a healthy one without caries. For example, a surgically extracted healthy tooth can be preferably employed in the present invention. A third molar tooth, in particular, can be employed most preferably, for the amount/tooth of dental pulp obtained from it is larger, and further because it is easily obtained in a healthy condition due to such reasons as dental makeover. Wherein, while there is no limitation as to the age of a human from whom a tooth is extracted, a tooth extracted from a human of 10 to 50 years old is preferred, and a tooth extracted from a human of 15 to 45 years is more preferred. Besides, there is no limitation as to the gender of a human from whom a tooth is obtained.

A tooth from which dental pulp is to be obtained is used preferably within 72 hours after tooth extraction, and more preferably 24 hours after tooth extraction, and still more preferably 12 hours after tooth extraction. An extracted tooth is washed with bicarbonate Ringer's solution, and then disinfected with a disinfectant. While there is no limitation as to a disinfectant employed here, a disinfectant which shows antibacterial activity both to Gram-positive and Gram-negative bacteria is preferred, and as such, 1% chlorohexidine hydrochloride solution is employed, for example.

Dental pulp can be obtained from an extracted tooth by fracturing the tooth so as to expose the dental pulp. The dental pulp taken out form the extracted tooth is then minced with a tool such as scissors, and digested with a protease.

By the above protease digestion, individual cells are released from the dental pulp obtained from the extracted tooth. While serine protease, metalloprotease, or a mixture of these is a preferable protease used here, a protease is not limited to them but any protease that can release individual cells forming dental pulp, namely one that can decompose proteinous cell adhesion molecules, may be used.

Examples of metalloproteases suitably used here include matrix metalloproteases, neutral metalloproteases, or a mixture of these. Among matrix metalloproteases, collagenases, gelatinases, or a mixture of these can be suitably used. Among collagenases, collagenase I, collagenase II, or a mixture of these is preferred. Among neutral metalloproteases, thermolysin, Dispase™, or a mixture of these can be suitably used.

An example of preferable combination of proteases is a mixture of collagenase I, collagenase II, and thermolysin. When using this mixture as proteases, the protease activity in the reaction solution is adjusted preferably to 0.17-1.33 units/mL. Liberase (registered trademark) (Roche) can be suitably used as proteases, which contains collagenase I, collagenase II, and thermolysin.

In the case where a mixture solution of collagenase type II and Dispase is used, the concentration of collagenase type II and Dispase is preferably 1-2 mg/mL and 3000-7000 units/mL, respectively, and more preferably approximately 1.5 mg/mL and approximately 5000 units/mL, respectively.

When digesting dental pulp taken out of an extracted tooth, suitable reaction temperature is 35-37.5°C, and suitable reaction time is 30 minutes to 3 hours. For example, dental pulp is treated with proteases at 37°C for 2 hours. However, a reaction temperature and reaction time should be adjusted as desired according to the types and concentration of proteases.

Dental pulp digested with proteases can be disintegrated by a mechanical means such as pipetting so that each cell forming dental pulp may be released from the dental pulp. Wherein, it is preferable to inactivate the proteases prior to mechanical disintegration of dental pulp. This can be done by adding to the reaction solution a compound that inactivates the proteases, and such a compound is exemplified by a chelating agent such as EDTA, and a medium for cell culture containing FBS. Disintegration of dental pulp gives a suspension where the cells that formed the dental pulp are suspended in a solution. This suspension contains multipotent stem cells released from the dental pulp. In order to remove the proteases along with the supernatant, this suspension is preferable centrifuged once to settle undissolved substances such as cells. After removal of the supernatant, sedimented cells and the like are suspended again. As a solution to be used for this resuspending process may be a medium for cell culture containing FBS, for example. The dental pulp suspension thus prepared contains tissue debris of dental pulp, in addition to the cells released from the dental pulp.

The dental pulp suspension in which disintegrated dental pulp is dispersed in a medium for cell culture is added to a cell culture plate and cultured on it. A culture medium used for this is preferably Dulbecco's modified Eagle's medium supplemented with fetal bovine serum. The concentration (v/v %) of fetal bovine serum added to the medium is preferably 8-25%, more preferably 10-25%, and for example 20%. An example of detailed composition of Dulbecco's modified Eagle's medium (before addition of fetal bovine serum) is shown in Table 1. As desired, L-alanyl-L-glutamine may be added to the medium at 3-5 mM, and, e.g., 4 mM. Further, as desired, antibiotics such as streptomycin may be added to the medium. When streptomycin is added to the medium, adjustment is made so that its concentration in the medium may be 10 mg/L to 250 mg/L, and, e.g., 100 mg/L. Besides, each ingredient can be replaced with its equivalent, e.g., its salt.

When a suspension of dental pulp obtained from an extracted tooth, e.g., a third molar of an adult, is cultured on a cell culture plate, the culture area of the plate is preferably 0.3-4 cm$^2$, and more preferably 0.6-2 cm$^2$, and, e.g., 1 cm$^2$. The amount of the medium added to the cell culture plate, per 1 cm$^2$-culture area, is preferably 250 μL-1 mL, and, e.g., 500 μL. For example, dental pulp suspension prepared from one of the third molars of an adult in 500 μL of a medium is added to a cell culture plate having 1 cm$^2$-culture areas, and cultured

Table 1. Formulation of Dulbecco's modified Eagle medium

| Ingredients | mM (Range) | mM (Suitable example) |
|---|---|---|
| Glycine (aminoacetic acid) | 0.2-0.6 | 0.4 |
| L-Arginine hydrochloride | 0.3-0.5 | 0.398 |
| L-Cystine dihydrochloride | 0.15-0.25 | 0.201 |
| L-Glutamine | 3-5 | 4 |
| L-Histidine hydrochloride monohydride | 0.15-0.25 | 0.2 |
| L-Isoleucine | 0.65-0.95 | 0.802 |
| L-Leucine | 0.65-0.95 | 0.802 |
| L-Lysine hydrochloride | 0.65-0.95 | 0.798 |
| L-Methionine | 0.15-0.25 | 0.201 |
| L-Phenylalanine | 0.2-0.6 | 0.4 |
| L-Serine | 0.2-0.6 | 0.4 |
| L-Threonine | 0.65-0.95 | 0.798 |
| L-Tryptophan | 0.06-0.1 | 0.0784 |
| L-Tyrosine disodium dihydrate | 0.2-0.6 | 0.398 |
| L-Valine | 0.65-0.95 | 0.803 |
| Choline chloride | 0.02-0.04 | 0.0286 |
| D-Calcium pantothenate | 0.007-0.009 | 0.00839 |
| Folic acid | 0.008-0.01 | 0.00907 |
| Nicotinamide | 0.03-0.05 | 0.0328 |
| Pyridoxine hydrochloride | 0.015-0.025 | 0.0196 |
| Riboflavin | 0.0008-0.0012 | 0.00106 |
| Thiamine hydrochloride | 0.01-0.014 | 0.0119 |
| i-Inositol | 0.03-0.05 | 0.04 |
| Calcium chloride (anhydrous) | 1.5-2.1 | 1.8 |
| Ferric nitrate nonahydrate | 0.0002-0.0004 | 0.000248 |
| Magnesium sulfate | 0.65-0.95 | 0.814 |
| Potassium chloride | 5-6 | 5.33 |
| Sodium bicarbonate | 40-48 | 44.05 |

(continued)

| Ingredients | mM (Range) | mM (Suitable example) |
|---|---|---|
| Sodium chloride | 100-120 | 110.34 |
| Sodium dihydrogen phosphate monobasic monohydrate | 0.65-0.95 | 0.906 |
| D-Glucose | 5-7 | 5.56 |
| Phenol red | 0.03-0.05 | 0.0399 |
| Sodium pyruvate | 0.8-1.2 | 1 |

The temperature at which a dental pulp suspension is cultured on a cell culture plate is preferably 35-37.5°C, and, e.g., 37°C. Culture of a dental pulp suspension is continued until, for example, colonies of visible sizes are formed on the plate due to cell growth. Such colonies generally look round when viewed from above, with their diameter about 1-3 mm, but colonies are not limited to those of such a shape and size and may, in some cases, be of an indefinite shape, for example. During the culture of a dental pulp suspension, the medium is replaced with a fresh one so as to maintain the ingredients within a certain range. Replacement of the medium is carried out at an interval of once in 1-6 days, 2-4 days, 1 day, 2 days, 3 days, 4 days, for example. When the medium is replaced, either the whole medium may be replaced with a fresh one, or only part of the medium may be replaced. When only part of the medium is replaced with a fresh one, 1/4 to 4/5, 1/4, 1/3, 1/2 or 4/5 of the used medium is removed and an equal amount of fresh medium is added. In this process of medium replacement, it is also possible to remove floating cells and tissue debris that were contained in the dental pulp suspension.

The cells that formed colonies on the cell culture plate by culturing dental pulp suspension can be collected by treating them with a protease so as to detach them from the plate. While there is no particular limitation as to the proteases employed here as far as they can decompose proteinous cell adhesion molecules, serine protease is preferred, and trypsin is more preferred. When using trypsin, its concentration is preferably 0.1-0.5% (w/v), and more preferably 0.25% (w/v).

Cells recovered from the above plate are once suspended in a medium for cell culture, and added to a cell culture plate, and culture is started on the plate. This is called "initial cell culture". The medium employed here is preferably Dulbecco's modified Eagle's medium supplemented with fetal bovine serum. The concentration (v/v %) of fetal bovine serum added to the medium is preferably 8-25%, more preferably 8-20%, and, e.g., 10%. An example of detailed composition of Dulbecco's modified Eagle's medium (before addition of fetal bovine serum) is shown in Table 1. L-alanyl-L-glutamine may be added to the medium as desired at 3-5 mM, and e.g., 4 mM. Further, as desired, antibiotics such as streptomycin may be added to the medium, though antibiotics are generally not added to the medium. When it is added to the medium, streptomycin is added so that its concentration may be at 10 mg/L to 250 mg/L, and, e.g., at 100 mg/L. Further, each ingredient can be replaced with its equivalent, e.g., its salt.

At the start of the initial cell culture mentioned above, the cells are added so that the density of viable cells on the cell culture plate may be preferably 2000-30000 cells/cm$^2$, more preferably 3000-20000 cells/cm$^2$, e.g., 3000 cells/cm$^2$, 5000 cells/cm$^2$, 10000 cells/cm$^2$, or 20000 cells/cm2. During the cell culture, the medium is replaced so as to maintain the ingredients within a certain range. Wherein, the medium is replaced, once in 1-6 days, 2-4 days, 1 day, 2 days, 3 days, or 4 days, for example. When the medium is replaced, either the entire medium may be replaced with a fresh one, or only part of the medium may be replaced. When only part of the medium is replaced with a fresh one, 1/4 to 4/5, 1/4, 1/3, 1/2 or 4/5 of the used medium is removed and an equal amount of fresh medium is added. In this process of medium replacement, it is also possible to remove floating cells and tissue debris that were contained in the dental pulp suspension, and as a result, those cells adhering to the cell culture plate are selected.

The initial cell culture is carried out until the cells on the cell culture plate come to be almost confluent. For example, culture is carried out until a state is reached in which the cells on the cell culture plate occupy not less than 85%, not less than 90%, or not less than 95% of the area where cells can adhere. The cells cultured until they come to be almost confluent on the cell culture plate can be recovered by protease treatment so as to detach them from the plate. While there is no particular limitation as to the proteases employed here as far as they can decompose proteinous cell adhesion molecules, serine protease is preferred, and trypsin is more preferred. When trypsin is employed, its concentration is preferably 0.1-0.5% (w/v), and more preferably 0.25% (w/v).

Cells recovered from the above plate are suspended again in the medium for cell culture, added then to a cell culture plate, and cultured on the plate. The medium employed here is preferably Dulbecco's modified Eagle's medium supplemented with fetal bovine serum. The concentration (v/v %) of fetal bovine serum added to the medium is preferably 8-25%, more preferably 8-20%, and, e.g., 10%. An example of detailed composition of Dulbecco's modified Eagle's medium (before addition of fetal bovine serum) is shown in Table 1. L-alanyl-L-glutamine may be added to the medium as desired at 3-5 mM, and e.g., 4 mM. Further, as desired, antibiotics such as streptomycin may be added to the medium, though antibiotics are generally not added to the medium. When it is added to the medium, streptomycin is added so that its concentration may be at 10 mg/L to 250 mg/L, and, e.g., at 100 mg/L. Further, each ingredient can be replaced with its equivalent, e.g., its salt. Cell culture is carried out until the cells on the cell culture plate come to be almost confluent. For example, culture is carried out until a state is reached where the cells on the cell culture plate occupy not less than 85%, not less than 90%, or not less than 95% of the area where cells can adhere.

The number of the dental pulp-derived multipotent stem cells can be increased by repeating the process of recovering the cells from a cell culture plate and culturing them on a cell culture plate. Further, by replacing the medium during culture, floating cells and tissue debris are removed, and thereby those cells adhering to the cell culture plate are preferentially collected (selected, as a result). As dental pulp-derived multipotent stem cells are prone to adhere to a cell culture plate, repetition of this process will lead to enrichment of multipotent stem cells. Namely, cells having undergone a process of culturing of a dental pulp suspension on a cell culture plate, and a process of recovering them from the plate after culture, are multipotent stem cell-enriched dental pulp-derived cells. Thus, by repeating recovery of cells from a cell culture plate and their culture which follows, the cells are further enriched in multipotent stem cells.

Recovery of cells from the cell culture plate and culture of them is repeated preferably 3-20 times, more preferably 3-8 times, still more preferably 4-8 times, and, e.g., 4 times, 5 times, 6 times, 7 times, or 8 times, including the initial cell culture. While many of the cells obtained after their growth on a cell culture plate are generally spindle-shaped dental pulp-derived multipotent stem cells, other cells are also present at the time of completion of the initial culture. By repeating 3 cycles of recovery of cells from the cell culture plate and their culture, the cells become enriched in dental pulp-derived multipotent stem cells until they consist almost entirely of dental pulp-derived multipotent stem cells. When the process of recovery of cells from the cell culture plate and their culture is repeated 5 times or more, the cells thus obtained have been further enriched in dental pulp-derived multipotent stem cells, to the point that when observed under an optical microscope, other cells than dental pulp-derived multipotent stem cells having a spindle shape can hardly be recognized. Thus, through 5 times or more repetition of the process of recovery of cells from the cell culture plate and their culture, dental pulp-derived multipotent stem cells are substantially isolated.

The ratio of dental pulp-derived multipotent stem cells to all the cells contained in the multipotent stem cell-enriched dental pulp-derived cells obtained as above is preferably not less than 99%, more preferably not less than 99.5%, still more preferably not less than 99.9%, and still more preferably not less than 99.95%. The ratio of dental pulp-derived multipotent stem cells can be determined by, e.g., dividing the number of generally spindle-shaped cells by the number of all the cells observed under an optical microscope.

The multipotent stem cell-enriched dental pulp-derived cells thus obtained can be cryopreserved as a suspension in a cryopreservation solution. To be subjected to cryopreservation, the mean doubling time of the cells on the cell culture plate is preferably not more than 96 hours, more preferably not more than 84 hours, still more preferably not more than 72 hours, still more preferably not more than 48 hours, and most preferably not more than 36 hours or not more than 24 hours, for example. It is possible to set a certain criterion as to a mean doubling time, so that only those cells which exhibit a mean doubling time falling within a certain time range may be cryopreserved for later use. A criterion may be set as desired, such that a mean doubling time must be not more than 84 hours, not more than 72 hours, or not more than 48 hours, for example. By discarding those cells which fail to meet the criterion without subjecting them to cryopreservation, only those cells having an ability above a certain level to undergo cell division can be selectively stored. The shorter the mean doubling time is, the higher the ability to undergo cell divisions the cells are deemed to have is.

There is no particular limitation as to the composition of a cryopreservation solution employed here as far as the solution can be used in freezing and thawing multipotent stem cell-enriched dental pulp-derived cells of a mammalian, and of human, in particular, without resulting their death. A cryopreservation solution contains a cell protective agent. A cell protective agent is, for example, a compound that serves to suppress formation ice crystals within the cells during they are being frozen, which otherwise could destroy cells. Preferable examples of cell protective agents include dimethyl sulfoxide (DMSO), ethylene glycol, propylene glycol, sericin, and glycerol. A combination of two of these may also be used. When dimethyl sulfoxide is employed as a cell protective agent, its concentration is preferably 5-15% (v/v), more preferably 9-11% (v/v), and, e.g., 10% (v/v). A cryopreservation solution may contain a buffering agent. A buffering agent capable of adjusting the pH of a solution to 6-8, and, e.g., 6.8-7.8 is preferred. Examples of such buffering agents include those containing carbonate ion, citrate ion, and sodium ion, for example. A cryopreservation solution may further contain human serum albumin. When human serum albumin is employed, its concentration is preferably 40-100 g/L, and more preferably 46-56 g/L, and, e.g., 51 g/L. A bicarbonate Ringer's solution supplemented with human serum albumin and dimethyl sulfoxide mentioned later is an example of preferred cryopreservation solution. Besides, either a cryopreservation solution for intermediate cells or a cryopreservation solution for cells as a preparation described later is a cryopreservation solution. Where a step of freezing intermediate cells is placed in the process of production, the former is called a first cryopreservation solution (cryopreservation solution for intermediate cells) and the latter is called a second cryopreservation solution (cryopreservation solution for cells as a preparation), for convenience.

Cryopreserved multipotent stem cell-enriched dental pulp-derived cells can be used as a dental pulp-derived multipotent stem cell preparation described later, and also as intermediate cells (which are preserved to let them further grow later). Cryopreservation of multipotent stem cell-enriched dental pulp-derived cells as intermediate cells is described in detail below.

Where multipotent stem cell-enriched dental pulp-derived cells are to be cryopreserved as intermediate cells (hereinafter referred to "where freezing cells as intermediate cells"), recovery of cells from the cell culture plate and their culture, including the initial cell culture are repeated preferably 3-8 times, more preferably 4-8 times, and, e.g., 4 times, 5 times, 6 times, 7 times, or 8 times.

Further, where cells are to be frozen as intermediate cells, the cells that are recovered from colonies formed on the cell culture plate after culturing a dental pulp suspension on a cell culture plate are allowed to undergo cell divisions preferably not less than 10 times, more preferably not less than 15 times. For example, those cells which have undergone cell divisions 13-20 times, 13-23 times, or 14-20 times are cryopreserved. Theoretically, through 15 times of cell divisions, the number of cells increases more than $3 \times 10^4$ times.

Further, where cells are to be frozen as intermediate cells, the cells are generally allowed to grow until the number of cells obtained from one extracted tooth (e.g., a single third molars) expand preferably not less than $3\times10^5$ cells, more preferably not less than $1\times10^6$ cells, and still more preferably not less than $1\times10^9$ cells.

Further, to be subjected to cryopreservation as intermediate cells, the mean doubling time of the cells on the cell culture plate is preferably not more than 96 hours, more preferably not more than 84 hours, still more preferably not more than 72 hours, still more preferably not more than 48 hours, and most preferably, e.g., not more than 24 hours, or not more than 36 ours. It is possible to set a certain criterion as to a mean doubling time, so that only those cells which exhibit a mean doubling time falling within a certain time range are cryopreserved for later use. A criterion may be set as desired, such that a mean doubling time should be not more than 84 hours, not more than 72 hours, or not more than 48 hours, for example. By discarding those cells which fail to meet the criterion without subjecting them to cryopreservation, only those cells having more than a certain level of ability to undergo cell divisions can be selectively stored. The shorter the mean doubling time is, the higher ability to undergo cell divisions the cells are deemed to have is.

Where freezing cells as intermediate cells, the multipotent stem cell-enriched dental pulp-derived cells detached from the cell culture plate by a protease treatment and recovered are washed with a wash solution prior to their cryopreservation. A wash solution used here is for removal of the medium and the protease, and there is no particular limitation as to its composition, and while physiological saline, phosphate-buffered saline, acetate Ringer's solution, bicarbonate Ringer's solution, and the like can be used, a solution prepared by adding human serum albumin to bicarbonate Ringer's solution is preferred. A commercially available acetate Ringer's solution and Bicarbonate Ringer's solution may be used. For example, PLA SMA-LYTE (registered trademark) A (Baxter), and Physio (registered trademark) 140 (Otsuka Pharmaceutical) may be employed as an acetate Ringer's solution, and Bicabon (registered trademark) (Ajinomoto) may be employed as a bicarbonate Ringer's solution. Preferable examples of composition and electrolyte content of bicarbonate Ringer's solution are shown in Table 2 and Table 3, respectively.

Table 2. Composition of bicarbonate Ringer's solution

| Ingredients | mM (Range) | mM (Suitable example) |
|---|---|---|
| Sodium chloride | 94.5-116 | 105 |
| Potassium chloride | 3.62-4.42 | 4.02 |
| Calcium chloride dihydrate | 1.35-1.65 | 1.5 |
| Magnesium chloride hexahydrate | 0.45-0.55 | 0.5 |
| Sodium bicarbonate | 22.5-27.5 | 25 |
| Sodium citrate dihydrate | 1.5-1.84 | 1.67 |
| pH 6.8-7.8, Osmotic pressure ratio to physiological saline: 0.9-1.0 | | |

Table 3. Electrolytes contained in bicarbonate Ringer solution

| Electrolytes | mEq/L (Range) | mEq/L (Suitable example) |
|---|---|---|
| $Na^+$ | 122-149 | 135 |
| $K^+$ | 3.6-4.4 | 4 |
| $Ca^{2+}$ | 2.7-3.3 | 3 |

(continued)

| Electrolytes | mEq/L (Range) | mEq/L (Suitable example) |
|---|---|---|
| $Mg^{2+}$ | 0.9-1.1 | 1 |
| $Cl^-$ | 102-124 | 113 |
| $HCO_3^-$ | 22.5-27.5 | 25 |
| $Citrate^{3-}$ | 4.5-5.5 | 5 |

Further, a suitable example of the composition of wash solution prepared by adding human serum albumin to bicarbonate Ringer's solution is shown in Table 4. Furthermore, a suitable example of electrolytes' concentrations in a wash solution prepared by adding human serum albumin to bicarbonate Ringer's solution is shown in Table 5. Besides, among the ingredients shown in Table 4 and Table 5, acetyltryptophan sodium and sodium caprylate may be omitted.

Table. 4 Composition of wash solution

| Ingredients | mM (Range) | mM (Suitable example) |
|---|---|---|
| Sodium chloride | 90-110 | 100 |
| Potassium chloride | 3.45-4.21 | 3.83 |
| Calcium chloride dihydrate | 1.29-1.57 | 1.43 |
| Magnesium chloride hexahydrate | 0.429-0.525 | 0.477 |
| Sodium bicarbonate | 21.4-26.2 | 23.8 |
| Sodium citrate dihydrate | 1.43-1.75 | 1.59 |
| Human serum albumin | (10.7-13.1) | (11.9) |
| Acetyltryptophan sodium | 0.870-1.06 | 0.967 |
| Sodium caprylate | 0.874-1.07 | 0.971 |
| (Note) In the table, the unit of concentration of human serum albumin is g/L. | | |

Table 5. Concentration of electrolytes (except albumin) in wash solution

| Ingredients | mEq/L (Range) | mEq/L (Suitable example) |
|---|---|---|
| $Na^+$ | 117.5-143.6 | 130.5 |
| $K^+$ | 3.45-4.21 | 3.83 |
| $Ca^{2+}$ | 2.57-3.15 | 2.86 |
| $Mg^{2+}$ | 0.86-1.05 | 0.95 |
| $Cl^-$ | 96.8-118 | 107.6 |
| $HCO_3^-$ | 21.4-26.2 | 23.8 |
| $Citrate^{3-}$ | 2.48-5.24 | 4.76 |
| Acetyltryptophan ion | 0.870-1.06 | 0.967 |
| Caprylate ion | 0.874-1.07 | 0.971 |

When freezing cells as intermediate cells, following their washing with a wash solution, the multipotent stem cell-enriched dental pulp-derived cells, are once recovered by a method such as centrifugation. The cells recovered then are cryopreserved in the form of a suspension (intermediate cell suspension). However, it is also possible to prepare an intermediate cell suspension not by recovering the cells but by replacing the composition of the solution in which the cells are suspended using ultrafiltration membrane or the like, and by cryopreserving the resulting suspension. There is no particular limitation as to the composition of the solution part of the intermediate cell suspension (cryopreservation solution for intermediate cells) as far as the solution can be used in freezing and thawing multipotent stem cell-enriched dental pulp-derived cells of a mammalian, and of human, in particular, without resulting their death. A cryopreservation solution for intermediate cells contains a cell protective agent. A cell protective agent is, for example, a compound that serves to suppress formation ice crystals within the cells during they are being frozen, which otherwise could destroy cells. Preferable examples of cell protective agents include dimethyl sulfoxide (DMSO), ethylene glycol, propylene glycol, sericin, and glycerol. A combination of two or more of these may also be used. Where dimethyl sulfoxide is employed as a cell protective agent, its concentration is preferably 5-15% (v/v), more preferably 9-11% (v/v), and, e.g., 10% (v/v). A cryopreservation solution may contain a buffering agent. A buffering agent capable of adjusting the pH of a solution to 6-8, and, e.g., 6.8-7.8 is preferred. Examples of such buffering agents include those containing carbonate ion, bicarbonate ion, citrate ion, and sodium ion, for example. A cryopreservation solution for intermediate cells may further contain human serum albumin. When human serum albumin is employed, its concentration is preferably 40-100 g/L, and more preferably 46-56 g/L, and, e.g., 51 g/L.

A bicarbonate Ringer's solution supplemented with human serum albumin and dimethyl sulfoxide is an example of preferred cryopreservation solution for intermediate cells. A cryopreservation solution for intermediate cells preferably contains 59-80.4 mM sodium chloride, 2.3-3.08 mM potassium chloride, 0.85-1.16 mM calcium chloride dihydrate, 0.28-0.385 mM magnesium chloride hexahydrate, 14-19.2 mM sodium bicarbonate, 0.94-1.28 mM sodium citrate dihydrate, 46-56 g/L human sedum albumin, 3.73-4.55 mM acetyltryptophan sodium, 3.74-4.58 mM sodium caprylate, and 9-11% (v/v) DMSO. Preferred examples of the composition of cryopreservation solution for intermediate cells are shown in Table 6. Namely, the composition of the cryopreservation solutions for intermediate cells generally contains 65.8-80.4 mM sodium chloride, 2.52-3.08 mM potassium chloride, 0.95-1.16 mM calcium chloride dihydrate, 0.315-0.385 mM magnesium chloride hexahydrate, 15.7-19.2 mM sodium bicarbonate, 1.04-1.28 mM sodium citrate dihydrate, 46-56 g/L human sedum albumin, 3.73-4.55 mM acetyltryptophan sodium, 3.74-4.58 mM sodium caprylate, and 9-11% (v/v) DMSO. Among these, acetyltryptophan sodium and sodium caprylate may be omitted. The osmotic pressure ratio of the cryopreservation solution for intermediate cells to physiological saline is preferably 0.9-1.1.

Further, a solution containing sodium ion, potassium ion, calcium ion, magnesium ion, bicarbonate ion, citrate ion, human serum albumin, and dimethyl sulfoxide can be suitably used as a cryopreservation solution for intermediate cells. A preferable example is a solution containing 91-113 mM sodium ion, 2.52-3.08 mM potassium ion, 0.95-1.16 mM calcium ion, 0.315-0.385 mM magnesium ion, 15.6-19.2 mM bicarbonate ion, 1.04-1.28 mM citrate ion, 46-56 g/L human serum albumin, and 9-11% (v/v) dimethyl sulfoxide. Furthermore, another preferable example is a solution containing 100-102 mM sodium ion, 2.71-2.77 mM potassium ion, 1.01-1.03 mM calcium ion, 0.335-0.345 mM magnesium ion, 16.9-17.2 mM bicarbonate ion, 1.13-1.15 mM citrate ion, 52.2-54.3 g/L human serum albumin, and 10.3-10.9% (v/v) dimethyl sulfoxide. Furthermore, still another preferable example is a solution containing 102 mM sodium ion, 2.80 mM potassium ion, 1.05 mM calcium ion, 0.35 mM magnesium ion, 17.4 mM bicarbonate ion, 1.16 mM citrate ion, 51 g/L human serum albumin, and 10% (v/v) dimethyl sulfoxide. Furthermore, a preferable example is a solution containing 101 mM sodium ion, 2.77 mM potassium ion, 1.03 mM calcium ion, 34 mM magnesium ion, 17.2 mM bicarbonate ion, 1.15 mM citrate ion, 52 g/L human serum albumin, and 10% (v/v) dimethylsulfoxide. Where acetyltryptophan or its salt is contained, its concentration is preferably 3.73-4.55 mM, or 4.24-4.41 mM, and, e.g., 4.14 mM, or 4.24 mM. Where caprylic acid or its salt is contained, its concentration is preferably 3.74-4.58 mM, or 4.25-4.43 mM, and, e.g., 4.16 mM, or 4.25 mM.

Table 6. Composition of cryopreservation solution for intermediate cells

| Ingredients | mM (Suitable range 1) | mM (Suitable range 2) | mM (Suitable example 1) | mM (Suitable example 2) |
|---|---|---|---|---|
| Sodium chloride | 65.8-80.4 | 70.8-72.3 | 73.1 | 72.3 |
| Potassium chloride | 2.52-3.08 | 2.71-2.77 | 2.80 | 2.77 |
| Calcium chloride dihydrate | 0.95-1.16 | 1.01-1.03 | 1.05 | 1.03 |
| Magnesium chloride hexa hydrate | 0.315-0.385 | 0.335-0.345 | 0.35 | 0.34 |
| Sodium bicarbonate | 15.67-19.15 | 16.9-17.2 | 17.41 | 17.2 |
| Sodium citrate dihydrate | 1.04-1.28 | 1.13-1.15 | 1.16 | 1.15 |
| Human serum albumin | (46-56) | (52.2-54.3) | (51) | (52.2) |
| Acetyltryptophan sodium | 3.73-4.55 | 4.24-4.41 | 4.14 | 4.24 |
| Sodium caprylate | 3.74-4.58 | 4.25-4.43 | 4.16 | 4.25 |

(continued)

| Ingredients | mM (Suitable range 1) | mM (Suitable range 2) | mM (Suitable example 1) | mM (Suitable example 2) |
|---|---|---|---|---|
| DMSO | (9-11) | (10.3-10.9) | (10) | (10) |
| (Note) The unit of concentration of human serum albumin is g/L, and that of DMSO is %(v/v). | | | | |

Where freezing cells as intermediate cells, while there is no particular limitation as to the density of the cells in a suspension in which multipotent stem cell-enriched dental pulp-derived cells are suspended in a cryopreservation solution for intermediate cells, it is preferably $2 \times 10^6$-$8 \times 10^7$ cells/mL, more preferably $4 \times 10^6$-$3 \times 10^7$ cells/mL, still more preferably $8 \times 10^6$-$2 \times 10^7$ cells/mL, and, e.g., $1.1 \times 10^7$ cells/mL. The multipotent stem cell-enriched dental pulp-derived cells suspended in a cryopreservation solution for intermediate cells are dispensed into cell cryopreservation containers in aliquots, and cryopreserved.

In the above, while the amount of the cell suspension that is dispensed into each cell cryopreservation container is to be adjusted as desired in accordance with its intended use, it is preferably 1-20 mL. Further, the number of cells dispensed into a cell cryopreservation container is preferably $5 \times 10^6$-$9.2 \times 10^8$ cells.

There is no particular limitation as to the container employed as a cell cryopreservation container as far as its material is durable at low temperatures and is not damaged during freezing and thawing of its content. Those commercially available as containers for cryopreservation of cells may also be used. For example, Cryotubes made of glass or plastics (e.g., of polyolefin resins) can be suitably used as cell cryopreservation containers. As glass-made containers here, vials made of borosilicate glass, in particular, can be used suitably. And the polyolefin resins above include polyethylene, high-density polyethylene, low-density polyethylene, polypropylene, ethylenepropylene copolymers, thermoplastic elastomers. Freezing of cells is preferably conducted by reducing the temperature stepwise after dispensing the cell suspension into cell cryopreservation containers in aliquots and sealing them. To freeze the cells, a method, for example, may be employed in which the temperature is lowered at a rate of 1°C per minute until it reaches -6°C, and then the cells are frozen rapidly. The cells are preserved in a frozen state, in which the temperature at which the cells are cryopreserved is preferably not higher than -130°C, more preferably not higher than -170°C, and still more preferably not higher than -180°C. The cells may be preserved, for example, in cell cryopreservation containers immersed in liquid nitrogen (boiling point: -196°C).

Before use, the cells cryopreserved as intermediate cells described above are thawed and subjected to the following step for production of a dental pulp-derived cell preparation.

In the process of production of multipotent stem cell-enriched dental pulp-derived cells, it is of great importance in the aspect of process control to place a step in which cells having been allowed to grow are once preserved as intermediate cells. In the case when a desired amount of teeth cannot be obtained at a time, for example, if production process is started with the very obtained extracted teeth and run up to the preparation of the final product, then the size of a lot of the final product would be relatively small, which would make lot management cumbersome. In contrast, preservation of intermediate cells following their production allows accumulation of intermediated cells up to a certain amount and then starting the production step which follows using them as a single batch, which makes lot management easier.

Further, placing a step where the intermediate cells are preserved allows quality inspection of the cells for their conditions at an intermediate stage of the whole production process. Thus, it permits the cells obtained as the intermediate cells to be subjected to the next step of production process only when they meet a desirable quality criteria. Namely, that make possible to remove such cells as fail to meet the desirable quality criteria before or after completion of the process producing intermediate cells. As a result, those cells which fail to meet the desired criteria are prevented from being put into use in the next step, and this increases the probability for the final product meeting the desired quality criteria to be produced. Thus, the production yield can be increased, which then can lower the production cost.

Cryopreserving cells in a production process generally involves a risk of change in cells' shapes between before and after their freezing. Further, some cells may degenerate in their proliferation ability after cryopreservation. In the present invention, in contrast, the cells obtained as intermediate cells show neither change in cells' shapes between before and after their freezing, nor degeneration in proliferation ability after cryopreservation.

It is preferred that the multipotent stem cell-enriched dental pulp-derived cells to be preserved as intermediate cells contain viable cells at a high ratio. The ratio of viable cells before cryopreservation (viable cell ratio) is preferably not less than 50%, more preferably not less than 60%, still preferably not less than 70%, and, e.g., not less than 80%, not less than 90%, or not less than 95%. It is also possible to set a desired criterion so that only those cells may be preserved as intermediate cells which have a cell viability ratio equal to or above a certain value before cryopreservation.

The multipotent stem cell-enriched dental pulp-derived cells to be preserved as intermediate cells are those having undergone cell divisions in an in vitro environment preferably not less than 10 times, and, e.g., not less than 15 times, not less than 16 times, or not less than 17 times, and showing their mean time per cell division during the period of their cell divisions not more than 48 hours.

Besides, in the present invention, that "the cells are allowed to divide in an in vitro environment" means, for example, to let the cells divide under a condition in which they are added to a culture container, such as a flask, together with a medium for cell culture. The cultivation temperature herein is preferably 34-38°C, more preferably 36-38°C, and, e.g., 37°C. And the cells are cultivated in a humid environment in the presence of 5% $CO_2$. There is no particular limitation as to the medium for cell culture employed here as far as it is a medium that can be used in culturing mammalian cells, and it may be, for example, Dulbecco's modified Eagle's medium (DMEM) supplemented with fetal bovine serum (FBS). The concentration of FBS in the medium is preferably 2-20%, and more preferably 5-20%, still more preferably 8-15%, and .e.g., 10%. And the concentration of glucose in the DMEM is 5-20 mM, and, e.g., 5 mM. A preferable set of culture conditions consists of cultivation temperature of 37°C, a humid environment in the presence of 5% $CO_2$, and DMEM as a medium containing approximately 5 mM glucose and 10% FBS. The culturing method for dental pulp-derived cells as described in Example 5 (i.e., on a cell culture plate, in DMEM medium supplemented with 10% FBS and containing 5.56 mM glucose, in the presence of 5% $CO_2$, at 37°C) is an example of suitable methods for letting the cells divide in an in vitro environment.

Where the cells are allowed to divide in an in vitro environment, passage culture of cells is conducted first by detaching them from the culture container and then seeding them in a fresh culture container so that 1/20-1/30 of the bottom area of the container may be covered with the cells. Passage culture is repeated when not less than 70%, not less than 80%, or not less than 90% of the bottom area of the culture container has covered by the cells,.

In an embodiment, when the multipotent stem cell-enriched dental pulp-derived cells to be preserved as intermediate cells are cultured in a medium containing a compound known to induce differentiation into chondrocytes, an increase in expression levels of aggrecans is observed as a whole. Further, when multipotent stem cell-enriched dental pulp-derived cells to be preserved as intermediate cells are cultured in a medium containing a compound known to induce differentiation into osteocytes, an increase in the amount of accumulated calcium in the cells is observed as a whole. Thus, the multipotent stem cell-enriched dental pulp-derived cells have abilities to differentiate into chondrocytes and osteocytes, when observed as a whole.

In an embodiment, when observed as a whole with respect to their expression pattern of surface antigen markers, the multipotent stem cell-enriched dental pulp-derived cells to be preserved as intermediate cells are positive for at least one of CD73, CD90, CD105, and CD166, while negative for at least one of CD34 and CD45. For example, when observed as a whole with respect to their expression pattern of surface antigen markers, the cells, are positive for CD73 and CD90, while negative for CD34. Further, for example, when observed as a whole with respect to their expression pattern of surface antigen markers, the cells, are positive for CD73, CD90, CD105, and CD166, while negative for CD34 and CD45.

In an embodiment, when observed as a whole with respect to their expression pattern of surface antigen markers, the multipotent stem cell-enriched dental pulp-derived cells to be preserved as intermediate cells, are negative, e.g., for at least one of CD40, CD80, CD86, and MHC-class II antigen. These cells, when observed as a whole with respect to their expression pattern of surface antigen markers, are negative, e.g., for CD40, CD80, CD86, and MHC-class II antigen. It is known that those cells which are positive for these surface antigen markers, when implanted in an allogeneic individual, tend to be excluded from the living body by being recognized as an antigen. Being negative for at least one of these surface antigen markers indicates that the multipotent stem cell-enriched dental pulp-derived cells to be preserved as intermediate cells have correspondingly less antigenicity, and therefore less tend to be excluded from the living body when implanted in an allogenic individual. Furthermore, with respect to their expression pattern of surface antigen markers, when these cells are stimulated with IFNγ, CD40, CD80, and CD86 remain negative, while MHC-class II antigen may turn positive. For example, with respect to their expression pattern of surface antigen markers, when these cells are stimulated with IFNγ, CD40, CD80, and CD86 remain negative, while MHC-class II antigen turns positive.

In an embodiment, when observed as a whole with respect to their expression pattern of surface antigen markers, the multipotent stem cell-enriched dental pulp-derived cells to be preserved as intermediate cells are positive, e.g., for CD73, CD90, CD105, and CD166, while negative for CD34, CD40, CD45, CD80, CD86, and MHC-class II antigen. Further, when the cells are stimulated with IFN-γ, CD40, CD80, and CD86 remain negative, while MHC-class II antigen turns positive.

In an embodiment, the multipotent stem cell-enriched dental pulp-derived cells to be preserved as intermediate cells are

(b-1) when observed as a whole with respect to their expression pattern of surface antigen markers, positive for at least one of, and preferably all of CD47, CD81, CD90, CD147, and HLA-A, B, C. Wherein, when observation is made cell-by-cell in the whole cell population, preferably not less than 80%, more preferably not less than 90%, and still more preferably not less than 95% of observed cells are positive for these antigens.

Further, in an embodiment, the multipotent stem cell-enriched dental pulp-derived cells to be preserved as intermediate cells are

(b-2) when observed as a whole with respect to their expression pattern of surface antigen markers, positive for at least one of, and preferably all of CD29, CD46, CD55, CD59, CD73, and CD140b. Wherein, when observation is made cell-by-cell in the whole cell population, preferably not less than 70%, more preferably not less than 80%, and still more preferably not less than 90% of observed cells are positive for these antigens.

Further, in an embodiment, multipotent stem cell-enriched dental pulp-derived cells to be preserved as intermediate cells are

(b-3) when observed as a whole with respect to their expression pattern of surface antigen markers, positive for at least one of, and preferably all of CD9, CD44, CD49b, CD49c, CD98, and EGF-R. Wherein, when observation is made cell-by-cell in the whole cell population, preferably not less than 65%, more preferably not less than 75%, and still more preferably not less than 80% of observed cells are positive for these antigens.

Further, in an embodiment, the multipotent stem cell-enriched dental pulp-derived cells to be preserved as intermediate cells are

(b-4) when observed as a whole with respect to their expression pattern of surface antigen markers, positive for at least one of, and preferably all of CD49f, and CD166. Wherein, when observation is made cell-by-cell in the whole cell population, preferably not less than 60%, preferably not less than 70%, and still more preferably not less than 75% of observed cells are positive for these antigens.

Further, in an embodiment, the multipotent stem cell-enriched dental pulp-derived cells to be preserved as intermediate cells are

(b-5) when observed as a whole with respect to their expression pattern of surface antigen markers, positive for at least one of, and preferably all of CD10, CD13, CD58, CD63, CD105, CD151, and CD164. Wherein, when observation is made cell-by-cell in the whole cell population, preferably not less than 60%, more preferably not less than 65%, and still more preferably not less than 70% of observed cells are positive for these antigens..

In an embodiment, the multipotent stem cell-enriched dental pulp-derived cells to be preserved as intermediate cells have preferably not less than 2, more preferably not less than 3, and still more preferably all of the characteristics (b-1), (b-2), (b-3), (b-4) and (b-5) defined above. For example, cells having the characteristics (b-1) and (b-2) defined above are one of the preferable embodiments of the present invention.

In an embodiment, the multipotent stem cell-enriched dental pulp-derived cells to be preserved as intermediate cells are

(b-6) when observed as a whole with respect to their expression pattern of surface antigen markers, negative for at least one of, and preferably all of CD120b, CD132, CD158a, CD161, CD184, CD195, CD206, CD210, CD212, CD226, CD244, CD267, CD278, CD279, CD282, CD294, NKB1, SSEA-1, TRA-1-60, TRA-1-81 ,Vβ23,SSEA-3, CLA, and integrin β7. Wherein, when observation is made cell-by-cell in the whole cell population, preferably only not more than 10%, more preferably not more than 5%, still more preferably not more than 2%, and still more preferably not more than 1% of observed cells are positive for these antigens.

In an embodiment, the multipotent stem cell-enriched dental pulp-derived cells to be preserved as intermediate cells are

(b-7) when observed as a whole with respect to their expression pattern of surface antigen markers, negative for at least one of, and preferably all of CD8b, CD11b, CD15s, CD16, CD19, CD24, CD31, CD32, CD62E, CD62P, CD66f, CD86, CD88, CD94, CD100, CD103, CD104, CD114, CD117, CD118, CD121b, CD122, CD123, CD124, CD126, CD127, CD128b, CD135, CD137, CD137 ligand, CD150, CD163, CD172b, CD177, CD178, CD180, CD197, CD220, CD229, CD231, CD255, CD268, CD305, CD314, CD321, CDw327, CDw328, CD329, CD335, CD336, BLTR-1, CLIP, CMRF-44, CMRF-56, fMLP-R, Vβ8, Invariant NKT, and γδTCR. Wherein, when observation is made cell-by-cell in the whole cell population, preferably only not more than 10%, more preferably not more than 5%, and still more preferably not more than 2% of observed cells are positive for these antigens.

In an embodiment, the multipotent stem cell-enriched dental pulp-derived cells to be preserved as intermediate cells are

(b-8) when observed as a whole with respect to their expression pattern of surface antigen markers, negative for at least one of, and preferably for all of CD1a, CD1b, CD1d, CD2, CD3, CD5, CD6, CD7, CD8a, CD11c, CD15, CD18, CD21, CD22, CD23, CD25, CD26, CD27, CD28, CD33, CD34, CD35, CD37, CD38, CD40, CD41a, CD41b, CD42b, CD45, CD45RB, CD45RO, CD48, CD50, CD53, CD62L, CD64, CD66(a, c, d, e), CD69, CD70, CD72, CD74, CD80, CD84, CD85, CD87, CD89, CDw93, CD97, CD106, CD134, CD138, CD141, CD144, CD154, CD158b, CD162, CD183, CD205, CD235a, CD271, CD309, CD326, CD337, αβTCR, and MHC-class II antigen. Wherein, when observation is made cell-by-cell in the whole cell population, preferably only not more than 10%, and preferably not more than 5% of observed cells are positive for these antigens.

In an embodiment, the multipotent stem cell-enriched dental pulp-derived cells to be preserved as intermediate cells have preferably not less than 2, more preferably not less than 3, and still more preferably all of the characteristics (b-1), (b-2), (b-3), (b-4), (b-5), (b-6), (b-7), and (b-8) defined above. For example, cells having the characteristics (b-1) and (b-6) defined above are one of the preferable examples of the present invention.

In an embodiment, the multipotent stem cell-enriched dental pulp-derived cells to be preserved as intermediate cells, when observed as a whole, express prostaglandin $E_2$ ($PGE_2$) and/or vascular endothelial growth factor (VEGF), for example, wherein expression levels of prostaglandin $E_2$ ($PGE_2$) increases when the cells are stimulated with TNF$\alpha$.

In an embodiment of the present invention, the multipotent stem cell-enriched dental pulp-derived cells to be preserved as intermediate cells secrete an increased amount of kynurenine when cultured in the presence of IFN-$\gamma$.

In an embodiment of the present invention, the multipotent stem cell-enriched dental pulp-derived cells to be preserved as intermediate cells, when observed as a whole with respect to their expression pattern of surface antigen markers, are positive for at least one of, and preferably all of CD47, CD81, and CD147, while negative for at least one of, or all of CD19, CD34, and CD206. In another embodiment, the cells are positive for at least one of, or all of CD47, CD81, and CD147, while negative for at least one of, or all of CD19, CD31, CD33, CD34, CD38, CD45, CD206, CD235a, and SSEA-1.

In an embodiment of the present invention, the multipotent stem cell-enriched dental pulp-derived cells to be preserved as intermediate cells, when observed as a whole with respect to their expression pattern of surface antigen markers, are positive for, e.g., one of CD73, CD90, CD105, and CD166, while negative for at least one of CD34 and CD45.

In an embodiment, the multipotent stem cell-enriched dental pulp-derived cells to be preserved as intermediate cells, when observed as a whole with respect to their expression pattern of surface antigen markers, are positive for CD73, CD90, CD105, and CD166, while negative for CD34, CD40, CD45, CD80, CD86, and MHC-class II antigen. Wherein, when the cells are stimulated with IFN-$\gamma$, CD40, CD80, and CD86, for example, remain negative, while MHC-class II antigen turns positive. Further, they express prostaglandin $E_2$ ($PGE_2$) and/or vascular endothelial growth factor (VEGF), wherein the amount of secreted prostaglandin $E_2$ ($PGE_2$) increases when the cells are stimulated with TNF$\alpha$.

In an embodiment, the multipotent stem cell-enriched dental pulp-derived cells to be preserved as intermediate cells, when observed as a whole with respect to their expression pattern of surface antigen markers, express increased levels of aggrecans when cultured in the presence of a compound known to induce differentiation into chondrocytes. Further, they exhibit increase in the amount of accumulated calcium in the cells. Thus, the multipotent stem cell-enriched dental pulp-derived cells, when observed as a whole, have abilities to differentiate into chondrocytes and osteocytes.

It is also possible to employ a method in management of production process in which only those cells are preserved as intermediate cells which show one or more particular expression patterns as to surface antigen markers, by applying a criterion set as desired based on the above-described expression patterns of surface antigen markers that multipotent stem cell-enriched dental pulp-derived cells to be preserved as intermediate cells must exhibit and/or expression patterns of other genes.

The multipotent stem cell-enriched dental pulp-derived cells to be preserved as intermediate cells consist almost entirely of multipotent cells that are derived from dental pulp (dental pulp-derived multipotent stem cells). In plate culture, they are observed as generally spindle-shaped adhering cells under an optical microscope, and the ratio of spindle-shaped cells to all the cells is preferably not less than 99%, more preferably not less than 99.5%, still preferably not less than 99.9%, and still more preferably not less than 99.95%. The ratio of dental pulp-derived multipotent stem cells can be determined by dividing the number of generally spindle-shaped cells by the number of all the cells observed under an optical microscope. Based on a criterion set as desired as mentioned above, it is also possible to employ a method in management of production process by which only those groups of cells are preserved as intermediate cells whose ratio of generally spindle-shaped cells is equal to or greater than a certain standard.

In an embodiment, the multipotent stem cell-enriched dental pulp-derived cells to be preserved as intermediate cells, when observed as a whole, preferably have an ability to differentiate into osteocytes and an ability to differentiate into chondrocytes. Namely, the intermediated cells, when cultured in a medium containing a compound known to induce differentiation into chondrocytes, exhibit increased levels of aggrecan expression. Further, the intermediate cells, when cultured in a medium containing a compound known to induce differentiation into osteocytes, exhibit an increase in the amount of accumulated calcium in the cells. That the intermediate cells have abilities to differentiate into chondrocytes and osteocytes can be determined by the methods described in Example 20 and Example 19, respectively. Using those methods, it is possible to make it a rule that only those groups of cells which are shown to have abilities to differentiate into chondrocytes and osteocytes are determined as the intermediate cells that are to be preserved. However, a method to examine the presence of those differentiation abilities is not limited to those described in Example 20 and Example 19. Using other proper methods, it is also possible to make it a rule that, only those groups of cells showing to have abilities to differentiate into chondrocytes and osteocytes are to be preserved as the intermediate cells.

In an embodiment, the multipotent stem cell-enriched dental pulp-derived cells to be preserved as intermediate cells may be those having the same, or substantially the same properties as those intermediated cells to be subjected to a further cultivation step described below in detail.

It is preferred for the cells cryopreserved as intermediate cells to maintain their properties when compared between before freezing and after thawing. Namely, it is preferred that multipotent stem cell-enriched dental pulp-derived cells cryopreserved as intermediate cells are substantially the same after being thawed as the cells before being frozen, in viable cell ratio, expression pattern of surface antigen markers, the shape of cells, differentiation abilities, and the like. By examining the intermediate cells for their properties after they are thawed or thawed and cultured, only those cells in which their properties are maintained as compared between before and after their thawing can be subjected to a later culturing step.

It is preferred that the cells cryopreserved as intermediate cells show, after they are thawed, viable cell ratio of preferably not less than 50%, more preferably not less than 60%, still more preferably not less than 70%, and the viable cell ratio is preferably, e.g., not less than 80%, not less than 90%, or not less than 95%. It is possible to make it a rule that following criteria set as desired, only those cells which after thawed, show a viable cell ratio equal to or more than a certain numerical value, can be subjected to a later culturing step. The viable cell ratio after they are thawed is generally 80%-98%, or 85%-95%.

After they are thawed and cultured, the cells that were cryopreserved as intermediate cells have an ability to undergo cell divisions of preferably not less than 10 times, more preferably not less than 15 times, and have, e.g., an ability to undergo cell divisions of not less than 14 times. Further, after they are thawed and cultured, the cells that were cryopreserved as intermediate cells exhibit their mean doubling time, on a cell culture plate, of preferably not more than 96 hours, more preferably not more than 84 hours, still more preferably not more than 72 hours, and still more preferably not more than 48 hours, and still more preferably not more than 36 hours. It is also possible, for example, that by setting a criterion for their ability to undergo cell divisions and mean doubling time, only those cells which exhibit an ability to undergo cell divisions equal to or above a certain level and mean doubling time equal to or less than a certain length of time, are subjected to later use. Such a criterion can be set as desired, namely for example, having an ability to undergo cell divisions not less than 10 times and mean doubling time of not more than 96 hours, having an ability to undergo cell divisions not less than 14 times and mean doubling time of not more than 72 hours, having an ability to undergo cell divisions not less than 15 times and mean doubling time of not more than 72 hours, and the like. By not using cells that fail to meet these criteria, it becomes possible so that only those intermediate cells having an ability to undergo cell divisions above a certain level are subjected to a later cultivation step. The culture conditions in the later step may be the same as or equivalent to those culture conditions described above for multipotent stem cell-enriched dental pulp-derived cells.

The cells to be cryopreserved as intermediate cells are those having undergone cell divisions in an in vitro environment preferably not less than 10 times, and, e.g., not less than 15 times, not less than 16 times, or not less than 17 times, and preferably showing their mean time per cell division over the period of their cell divisions of not more than 48 hours.

When they are cultured in a medium containing a compound known to induce differentiation into chondrocytes, intermediate cells to be subjected to a further cultivation step are observed as a whole to exhibit increased levels of aggrecan expression. Further, when cultured in a medium containing a compound known to induce differentiation into osteocytes, intermediate cells to be subjected to a further cultivation step are observed as a whole to exhibit an increase in the amount of accumulated calcium in the cells. Those cells, when observed as a whole, have abilities to differentiate into chondrocytes and osteocytes.

In an embodiment, when observed as a whole with respect to the expression pattern of surface antigen markers after they are thawed or thawed and cultured, intermediate cells to be subjected to a further cultivation step are positive for at least one of CD73, CD90, CD105, and CD166, while negative for at least one of CD34 and CD45. For example, those cells are positive for CD73, and CD90, while negative for CD34. Further, for example, they are positive for CD73, and CD90, while negative for CD34. The cells are positive for CD73, CD90, CD105, and CD166, while negative for CD34, and CD45.

Further, in an embodiment, when observed as a whole with respect to the expression pattern of surface antigen markers immediately after they are thawed or thawed and cultured, intermediate cells to be subjected to a further cultivation step are negative for at least one of CD40, CD80, CD86, and MHC-class II antigen. For example, they are negative for CD40, CD80, CD86, and MHC-class II antigen. It is known that those cells which are positive for these surface antigen markers, when implanted in an allogeneic individual, tend to be excluded from the living body by being recognized as an antigen. Being negative for these surface antigen markers indicates that the intermediate cells to be subjected to a further cultivation step are correspondingly less antigenic and therefore less tend to be excluded from the living body when implanted in an allogenic individual. Further, with respect to the expression pattern of surface antigen markers, when the cells are stimulated with IFN-γ, CD40, CD80, and CD86 remain negative, while MHC-class II antigen may turn positive. For example, with respect to the expression pattern of surface antigen markers, when the cells are stimulated with IFN-γ, CD40, CD80, and CD86 remain negative, while MHC-class II antigen turns positive.

In an embodiment, when observed as a whole with respect to the expression pattern of surface antigen markers immediately after they are thawed or thawed and cultured, intermediate cells to be subjected to a further cultivation step are positive for CD73, CD90, CD105, and CD166, while negative for CD34, CD40, CD45, CD80, CD86, and MHC-class II antigen. Further, when the cells are stimulated with IFN-γ, CD40, CD80, and CD86 remain negative, while MHC-class II antigen turns positive.

In an embodiment, intermediate cells to be subjected to a further cultivation step are;

(c-1) when observed as a whole with respect to the expression pattern of surface antigen markers, are positive for at least one of, and preferably all of CD47, CD81, CD90, CD147, and HLA-A, B, C. Wherein, when observation is made cell-by-cell in the whole cell population, preferably not less than 80%, more preferably not less than 90%, still more preferably not less than 95% of observed cells are positive for these antigens.

In an embodiment, intermediate cells to be subjected to a further cultivation step are;

(c-2) when observed as a whole with respect to the expression pattern of surface antigen markers, are positive for at least one of, and preferably all of CD29, CD46, CD55, CD59, CD73, and CD140b. Wherein, when observation is made cell-by-cell in the whole cell population, preferably not less than 70%, more preferably not less than 80%, and still more preferably not less than 90% of observed cells are positive for these antigens.

Further, in an embodiment, intermediate cells to be subjected to a further cultivation step are;

(c-3) when observed as a whole with respect to the expression pattern of surface antigen markers, are positive for at least one of, and preferably all of CD9, CD44, CD49b, CD49c, CD98, and EGF-R. Wherein, when observation is made cell-by-cell in the whole cell population, preferably not less than 65%, more preferably not less than 75%, and still more preferably not less than 80% of observed cells are positive for these antigens.

Further, in an embodiment, intermediate cells to be subjected to a further cultivation step are;

(c-4) when observed as a whole with respect to the expression pattern of surface antigen markers, are positive for at least one of, and preferably all of CD49f, and CD166. Wherein, when observation is made cell-by-cell in the whole cell population, preferably not less than 60%, more preferably not less than 70%, and still more preferably not less than 75% of observed cells are positive for these antigens.

Further, in an embodiment, intermediate cells to be subjected to a further cultivation step are;

(c-5) when observed as a whole with respect to the expression pattern of surface antigen markers, positive for at least one of, and preferably all of CD10, CD13, CD58, CD63, CD105, CD151, and CD164. Wherein, when observation is made cell-by-cell in the whole cell population, preferably not less than 60%, more preferably not less than 65%, and still more preferably not less than 70% of observed cells are positive for these antigens.

In an embodiment, intermediate cells to be subjected to a further cultivation step have preferably not less than 2, more preferably not less than 3, and still more preferably all of the characteristics (c-1), (c-2), (c-3), (c-4), and (c-5) defined above. For example, cells having the characteristics (c-1) and (c-2) defined above are one of the preferable examples of the present invention.

In an embodiment, intermediate cells to be subjected to a further cultivation step are;

(c-6) when observed as a whole with respect to the expression pattern of surface antigen markers, negative for at least one of, and preferably all of CD120b, CD132, CD158a, CD161, CD184, CD195, CD206, CD210, CD212, CD226, CD244, CD267, CD278, CD279, CD282, CD294, NKB1, SSEA-1, TRA-1-60, TRA-1-81, Vβ23, SSEA-3, CLA, and integrin β7. Wherein, when observation is made cell-by-cell in the whole cell population, preferably only not more than 10%, more preferably not more than 5%, still preferably not more than 2%, and still more preferably not more than 1% of the observed cells are positive for these antigens.

In an embodiment, intermediate cells to be subjected to a further cultivation step are

(c-7) when observed as a whole with respect to the expression pattern of surface antigen markers, negative for at least one of, and preferably for all of CD8b, CD11b, CD15s, CD16, CD19, CD24, CD31, CD32, CD62E, CD62P, CD66f, CD86, CD88, CD94, CD100, CD103, CD104, CD114, CD117, CD118, CD121b, CD122, CD123, CD124, CD126, CD127, CD128b, CD135, CD137, CD137 ligand, CD150, CD163, CD172b, CD177, CD178, CD180, CD197, CD220, CD229, CD231, CD255, CD268, CD305, CD314, CD321, CDw327, CDw328, CD329, CD335, CD336, BLTR-1, CLIP, CMRF-44, CMRF-56, fMLP-R, Vβ8, Invariant NKT, and γδTCR. Wherein, when observation is made cell-by-cell in the whole cell population, preferably only not more than 10%, more preferably not more than 5%, and still more preferably not more than 2% of the observed cells are positive for these antigens.

In an embodiment, intermediate cells to be subjected to a further cultivation step are;

(c-8) when observed as a whole with respect to the expression pattern of surface antigen markers, are negative for at least one of, and preferably all of CD1a, CD1b, CD1d, CD2, CD3, CD5, CD6, CD7, CD8a, CD11c, CD15, CD18, CD21, CD22, CD23, CD25, CD26, CD27, CD28, CD33, CD34, CD35, CD37, CD38, CD40, CD41a, CD41b, CD42b, CD45, CD45RB, CD45RO, CD48, CD50, CD53, CD62L, CD64, CD66 (a, c, d, e), CD69, CD70, CD72, CD74, CD80, CD84, CD85, CD87, CD89, CDw93, CD97, CD106, CD134, CD138, CD141, CD144, CD154, CD158b, CD162, CD183, CD205, CD235a, CD271, CD309, CD326, CD337, αβTCR, and MHC-class II antigen. Wherein, when observation is made cell-by-cell in the whole cell population, preferably only not more than 10%, more preferably not more than 5% are positive for these antigens.

In an embodiment, intermediate cells to be subjected to a further cultivation step have preferably not less than 2, more preferably not less than 3, and still more preferably all of the characteristics (c-1), (c-2), (c-3), (c-4), (c-5), (c-6), (c-7), and (c-8) defined above. For example, cells having the characteristics (c-1) and (c-6) defined above are one of the preferable examples of the present invention.

Further, intermediate cells to be subjected to a further cultivation step, when observed as a whole, express prostaglandin $E_2$ ($PGE_2$) and/or vascular endothelial growth factor (VEGF), wherein the expression levels of prostaglandin $E_2$ ($PGE_2$) increases when the cells are stimulated with TNF$\alpha$.

In an embodiment of the present invention, intermediate cells to be subjected to a further cultivation step, when cultured in the presence of IFN-$\gamma$, exhibit an increase in the amount of secreted kynurenine.

In an embodiment of the present invention, intermediate cells to be subjected to a further cultivation step, when observed as a whole with respect to their expression pattern of surface antigen markers, are positive for at least one of, or preferably for all of CD47, CD81, and CD147, while negative for at least one of, or all of CD19, CD34, and CD206. Further, in an embodiment of the present invention, multipotent stem cell-enriched dental pulp-derived cells, when observed as a whole with respect to their expression pattern of surface antigen markers, are positive for at least one of, or all of CD47, CD81, and CD147, while negative for at least one of, or all of CD19, CD31, CD33, CD34, CD38, CD45, CD206, CD235a, and SSEA-1.

In an embodiment, intermediate cells to be subjected to a further cultivation step, when observed as a whole with respect to their expression pattern of surface antigen markers, are negative for at least one of, or all of CD19, CD26, CD106, CD117, and CD271.

In an embodiment, intermediate cells to be subjected to a further cultivation step, when observed as a whole with respect to their expression pattern of surface antigen markers, are positive for at least one of, and, e.g., all of CD140b and HLA-A, B, C.

In an embodiment of the present invention, intermediate cells to be subjected to a further cultivation step, when observed as a whole with respect to their expression pattern of surface antigen markers, are positive for at least one of, or all of CD46, CD47, CD55, CD58, and CD59.

In an embodiment of the present invention, intermediate cells to be subjected to a further cultivation step, when observed as a whole with respect to their expression pattern of surface antigen markers, are, for example, positive at least one of CD73, CD90, CD105, and CD166, while negative for at least one of CD34 and CD45.

Further, in an embodiment, intermediate cells to be subjected to a further cultivation step, when observed as a whole with respect to their expression pattern of surface antigen markers, are positive for CD73, CD90, CD105, and CD166, while negative for CD34, CD40, CD45, CD80, CD86, and MHC-class II antigen. Wherein when the cells are stimulated with IFN-$\gamma$, CD40, CD80, and CD86 remain negative, while MHC-class II antigen turns positive. Further, the cells express prostaglandin $E_2$ ($PGE_2$) and/or vascular endothelial growth factor (VEGF), wherein the amount of secreted prostaglandin $E_2$ ($PGE_2$) increases when the cells are stimulated with TNF$\alpha$. Further, when the cells are stimulated with IFN-$\gamma$, the amount of secreted kynurenine increases.

Intermediate cells to be subjected to a further cultivation step, secrete various humoral factors when they are cultured.

(c-9) In an embodiment, intermediate cells to be subjected to a further cultivation step, when cultured, express at least one of, and preferably all of MMP-2, IGFBP-4, and cystatin C.

(c-10) In an embodiment, intermediate cells to be subjected to a further cultivation step, when cultured, express at least one of, and preferably all of IL-6, IL-11, MCP-1, IL-8, GRO$\alpha$, HGF, VEGF, VCAM-1, TIMP-3, TIMP-2, and TIMP-1.

(c-11) Further, in an embodiment, intermediate cells to be subjected to a further cultivation step, when cultured, express at least one of, and preferably all of IL-23, TNF-$\alpha$, IL-18, IL-33, IL-27, TARC, ENA-78, MIP-3$\alpha$, MIP-1$\beta$, IP-10 (CXCL10), SCF, and ICAM-1.

(c-12) Further, in an embodiment, intermediate cells to be subjected to a further cultivation step, when cultured, express no, or almost no IL-21, IFN-$\alpha$, Eotaxin, MIP-1$\alpha$, MIG, I-TAC, or GM-CSF.

(c-13) Further, in an embodiment, intermediate cells to be subjected to a further cultivation step, when cultured in the presence of TNF-$\alpha$ or IFN-$\gamma$, express increased levels of IL-6, as compared with their culture in the absence of these.

(c-14) Further, in an embodiment, intermediate cells to be subjected to a further cultivation step, when cultured in the presence of TNF-$\alpha$ or IFN-$\gamma$, express increased levels of IL-11, as compared with their culture in the absence of these.

(c-15) Further, in an embodiment, intermediate cells to be subjected to a further cultivation step, when cultured in the presence of TNF-$\alpha$ or IFN-$\gamma$, express increased levels of IP-10 (CXCL 10) as compared with their culture in the absence of these.

(c-16) Further, in an embodiment, intermediate cells to be subjected to a further cultivation step, when cultured in the presence of TNF-$\alpha$ or IFN-$\gamma$, express increased levels of MCP-1 as compared with their culture in the absence of these.

(c-17) Further, in an embodiment, intermediate cells to be subjected to a further cultivation step, when cultured in the presence of TNF-$\alpha$, are induced to express GM-CSF, while expression of GM-CSF is not induced when the cells are cultured in the presence of IFN-$\gamma$.

(c-18) Further, in an embodiment, intermediate cells to be subjected to a further cultivation step, when cultured in the presence of TNF-$\alpha$, show a decrease in the expression levels of HGF as compared with their culture in its absence, while they exhibit increased levels of HGF when cultured in the presence of IFN-$\gamma$ as compared with their culture in its absence.

(c-19) Further, in an embodiment, intermediate cells to be subjected to a further cultivation step, when cultured in the presence of TNF-$\alpha$, express increased levels of IL-8 as compared their culture in its absence.

(c-20) Further, in an embodiment, intermediate cells to be subjected to a further cultivation step, when cultured in the presence of TNF-$\alpha$, are induced to express G-CSF, while expression of G-CSF is not induced when they are cultured in the presence of IFN-$\gamma$.

In an embodiment, intermediate cells to be subjected to a further cultivation step have not less than 2, preferably not less than 3, more preferably not less than 4, and still more preferably all of the characteristics (c-9)-(c20) defined above. For example, cells having characteristics (c-9), (c-15) and (c-20) defined above and cells having characteristics (c-9), (c-14), (c-15) and (c-20) defied above are preferred embodiments of the present invention.

Further, intermediate cells to be subjected to a further cultivation step, when cultured immediately after they are thawed, or thawed and cultured, have abilities to differentiate into chondrocytes and osteocytes when observed as a whole. Namely, they express increased levels of aggrecans when cultured in the presence of a compound known to induce differentiation into chondrocytes, and they exhibit an increase in the amount of accumulated calcium in the cells when cultured in a medium containing a compound known to induce differentiation into osteocytes. That the intermediate cells have abilities to differentiate into chondrocytes and osteocytes can be determined by the methods described in Example 20 and Example 19, respectively. Using those methods, it is possible to make it a rule that only those groups of cells showing to have abilities to differentiate into chondrocytes and osteocytes are determined as the intermediate cells to be subjected to a further cultivation step. However, a method to examine the presence of those differentiation abilities is not limited to what are described in those Examples. It is also possible to make it a rule that, using other proper methods, that only those groups of cells showing to have abilities to differentiate into chondrocytes and osteocytes are to be subjected to a further cultivation step.

Based on the above expression pattern of surface antigen markers and/or other genes that intermediate cells to be subjected to a further cultivation step exhibit, it is also possible to employ a method in which only those cells having certain expression patterns are determined as being intermediate cells that may be subjected to a further cultivation step, as a manner of management of the production process.

Further, intermediate cells that are to be subjected to a further cultivation step, when observed as a whole immediately after they are thawed, or thawed and cultured, consist almost entirely of dental pulp-derived multipotent stem cells. In plate culture, they are observed as generally spindle-shaped adhering cells under an optical microscope, and the ratio of spindle-shaped cells to all the cells is preferably not less than 99%, more preferably not less than 99.5%, still more preferably not less than 99.9%, and still more preferably not less than 99.95%. Based on a criterion set as desired as mentioned above, it is also possible to make it a rule that only those groups of cells whose ratio of generally spindle-shaped cells are equal to or greater than a certain standard is to be subjected to a later cultivation process.

Further, intermediate cells that are to be subjected to a further cultivation step, when thawed and cultured, have an ability to undergo cell divisions of preferably not less than 10 times, more preferably not less than 15 times, and have, e.g., an ability to undergo cell divisions not less than 14 times. Furthermore, intermediate cells that are to be subjected to a further cultivation step, when thawed and cultured on a cell culture plate, exhibit their mean doubling time of preferably not more than 96 hours, more preferably not more than 84 hours, still more preferably not more than 72 hours, and still more preferably not more than 48 hours, and still more preferably not more than 36 hours. A set of culture conditions here is the same or substantially the same as the culture conditions for multipotent stem cell-enriched dental pulp-derived cells as described hereinbefore, and for example, the conditions described in Example 5 (i.e., on a cell culture plate, in DMEM medium supplemented with 10% FBS and containing 5.56 mM glucose, in the presence of 5% $CO_2$, at 37°C) are employed. Based on a criterion set as desired according to the above, it is possible to make it a rule that only those cells showing to have an ability to undergo cell divisions that is not lower than a certain level are to be subjected to a later cultivation step as intermediated cells. Further using a certain criterion set as desired, it is also possible to make it a rule that only those groups of cells exhibiting their mean doubling time falling within a certain length of time are subjected to a later cultivation step as intermediated cells. Such criteria can be set as desired, namely for example, having an ability to undergo cell divisions not less than 10 times and mean doubling time of not more than 96 hours, having an ability to undergo cell divisions not less than 14 times and mean doubling time of not more than 72 hours, having an ability to undergo cell divisions not less than 15 times and mean doubling time of not more than 72 hours.

Namely, intermediate cells to be subjected to a further cultivation step are such cells that have the properties of stem cells, namely a high ability to undergo cell divisions and an ability to differentiate into two or more different kinds of cells. It may be said that Intermediate cells to be subjected to a further cultivation step are cells having a high colony-forming ability. The term "colony-forming ability" herein means a cell's ability to divide and form colonies when seeded on a plate and cultured.

When thawed and suspended in a solution, e.g., a culture medium, intermediate cells have a mean diameter preferably of not more than 20 $\mu$m, not more than 18 $\mu$m, and, e.g., 10-20 $\mu$m, 10-18 $\mu$m, 12-20 $\mu$m, 14-20 $\mu$m, 16-20 $\mu$m.

Examples of quality inspection items performed at the completion of, or before and after the step of, production of intermediate cells are listed as quality inspections a-k. Quality inspections are performed on one or more of those items. To perform inspections on all of the items is also allowed. Cells to be subjected to an inspection are any one of (1) part of cells dispensed before they are suspended in a cryopreservation solution as intermediate cells, (2) part of cells which are dispensed before they are suspended in a cryopreservation solution as intermediate cells, and which have undergone passage culture, (3) cells suspended but not yet frozen in a cryopreservation solution to be cryopreserved as intermediate cell, (4) cells prepared immediately after thawing of those cells which were once frozen as intermediate cells, or (5) cells obtained by culturing the cells that were prepared by thawing cells which were once frozen as intermediate cells. Unless specified otherwise, the same quality inspection may be performed on 2 or more groups of cells identified in (1)-(5) above.

(Quality Inspection a) Verification that, when observed under an optical microscope, the ratio of generally spindle-shaped cells to all the cells is not less than 99%, not less than 99.5%, not less than 99.9%, or not less than 99.95%.

(Quality Inspection b) Verification that the viable cell ratio is not less than 50%, not less than 60%, not less than 70%, not less than 80%, not less than 90%, or not less than 95%.

(Quality Inspection c) Verification that with respect to their expression pattern of surface antigen markers, the cells are positive for CD73, CD90, CD105, and CD166, while negative for CD34 and CD45, or
verification that with respect to their expression pattern of surface antigen markers, the cells are positive for at least one of CD73 and CD90, while negative for CD34.

(Quality Inspection d) Verification that with respect to their expression pattern of surface antigen markers, the cells are negative for CD40, CD80, CD86, and MHC-class II antigen, or
verification that with respect to their expression pattern of surface antigen markers, the cells are negative for at least one of CD40, CD80, CD86, and MHC-class II antigen.

(Quality Inspection e) Verification that with respect to their expression pattern of surface antigen markers, when the cells are stimulated with IFN-$\gamma$, CD40, CD80, and CD86 remain negative, while MHC-class II antigen turns positive, or at least one verification that with respect to their expression pattern of surface antigen markers, when the cells are stimulated with IFN-$\gamma$, the cells are negative for CD40, negative for CD80, negative for CD86, or positive for MHC-class II antigen.

(Quality Inspection f) Verification that the cells express prostaglandin $E_2$ ($PGE_2$) and/or vascular endothelial growth factor (VEGF), and the expression levels of prostaglandin $E_2$ ($PGE_2$) increases when the cells are stimulated with TNF$\alpha$.

(Quality Inspection g) Verification that when cultured in a medium containing a compound known to induce differentiation into chondrocytes, the cells exhibit increased levels of aggrecan expression.

(Quality Inspection h) Verification that when cultured in a medium containing a compound known to induce differentiation into osteocytes, the cells exhibit an increase in the amount of accumulated calcium in the cells.

(Quality Inspection i) Verification that the cells have an ability to undergo cell divisions on a cell culture plate not less than 10 times, not less than 14, or not less than 15 times.

(Quality Inspection j) Verification that during the above inspection i, the cells' mean doubling time on a cell culture plate is not more than 96 hours, not more than 84 hours, not more than 72 hours, not more than 48 hours, or not more than36 hours.

(Quality Inspection k) Verification that the mean diameter of the cells, when they are suspended in a culture medium, is not more than 20 $\mu$m, not more than 18 $\mu$m, 10-20 $\mu$m, 10-18 $\mu$m, 12-20 $\mu$m, 14-20 $\mu$m, or 16-20 $\mu$m.

It is permitted that quality inspection is performed on 10 items of (Quality Inspection a) to (Quality Inspection j). Further it is also permitted that quality inspection is performed on 9 items chosen arbitrarily from the above 10. When 9 items are chosen to perform quality inspection, Quality Inspections a, b, c, d, e, f, g, i, and j may be chosen, for example but without limitation. Further it is also permitted to perform quality inspections on 8 items chosen arbitrarily from the above 10. When quality inspection is performed on 8 items chosen, Quality Inspections a, b, c, d, e, f, i, and j may be chosen, for example but without limitation. Further it is also permitted to perform quality inspections on 7 items chosen arbitrarily from the above 10. When 7 items are chosen to perform quality inspection, Quality Inspections a, b, c, d, e, i, and j may be chosen, for example but without limitation. Further, it is also permitted to perform quality inspection on 6 items chosen arbitrarily from the above 10. When 6 items are chosen to perform quality inspection, Quality Inspections a, b, c, d, i, and j may be chosen, for example but without limitation. Further, it is also permitted to perform a quality inspection on 5 items chosen arbitrarily from the above 10. When 5 items are chosen to perform quality inspection, Quality Inspection a, b, c, i, and j may be chosen, for example but without limitation. Furthermore, it is also permitted to perform a quality inspection on 4 items chosen arbitrarily from the above 10. When 4 items are chosen to perform quality inspection, Quality Inspection a, b, c, and j may be chosen, for example but without limitation. Furthermore, it is also permitted to perform quality inspection on 3 items chosen arbitrarily from the above 10. When 3 items are chosen to perform quality inspection, Quality Inspection a, b, and c may be chosen, for example but without limitation. Still farther, it is also permitted to perform a quality inspection on 2 items chosen arbitrarily from the above 10. When 2 items are chosen to perform quality inspection, quality inspection may be performed, for example without limitation, on Quality Inspections a and b, Quality Inspections a and c, Quality inspections a and d, Quality Inspections a and e, Quality Inspections a and f, Quality Inspections a and g, Quality Inspections a and h, Quality Inspections a and i, Quality Inspections a and j, Quality Inspections b and c, Quality Inspections b and d, Quality Inspections b and e, Quality Inspections b and f, Quality Inspections b and g, Quality Inspections b and h, Quality Inspections b and i, Quality Inspections b and j, Quality Inspections c and d, Quality Inspections c and e, Quality Inspections c and f, Quality Inspections c and g, Quality Inspections c and h, Quality Inspections c and i, Quality Inspections c and j, Quality Inspections d and e, Quality Inspections d and f, Quality Inspections d and g, Quality Inspections d and h, Quality Inspections d and i, Quality Inspections d and j, Quality Inspections e and f, Quality Inspections e and g, Quality Inspections e and h, Quality Inspections e and i, Quality Inspections e and j, Quality Inspections f and i, Quality Inspections f and j, or Quality Inspections i and j, for example but without limitation.

(Production Culture)

A further cultivation step performed with intermediate cells is a step that ends in the production of multipotent stem cell-enriched dental pulp-derived cells as the final product, and this step is called "production culture".

A medium employed in a production culture is preferably a Dulbecco's modified Eagle's medium supplemented with fetal bovine serum. The concentration (v/v %) of fetal bovine serum with which the medium is supplemented is preferably 8-25%, more preferably 8-20%, and, e.g., 10%. Dulbecco's modified Eagle's medium (before addition of fetal bovine serum) is shown in Table 1. As desired, L-alanyl-L-glutamine may be added to the medium at 3-5 mM, e.g., at 4 mM. Further, in general, no antibiotics is added in general to the medium in a production culture. However, an antibiotic such as streptomycin or the like may be added to the medium. When streptomycin is added to the medium, its addition is made so that its concentration in the medium may come to be 10 mg/L - 250 mg/L, e.g., 100 mg/L. Further, each ingredient of the medium may be replaced with its equivalent, e.g., its salt.

In a production culture, cultivation may be carried out either on a cell culture plate or on microcarriers in a cell cultivation apparatus, such as a bioreactor, for example. Further, a production culture may be carried out by letting the cells grow first on a cell culture plate, and then on microcarriers in a cell cultivation apparatus, such as a bioreactor, for example. Furthermore, when culturing cells on microcarriers, shake flasks may also be used instead of a bioreactor. When culturing cells on a cell culture plate, the conditions for culture are the same as or similar to the aforementioned conditions employed for culturing those cells which are obtained following cultivation of a dental pulp suspension. Namely, starting with the thawed intermediate cells, it is done to increase the number of the cells by repeating cycles of cultivation on a cell culture plate and their recovery. Besides, as a bioreactor, one of those marketed by such companies as GE Healthcare, Merck, Sartorius, for example, can be suitably used. A detailed explanation is made below about a method of cell culture using a bioreactor as a cell cultivation apparatus.

In a production culture, when culturing cells on microcarriers using a bioreactor, microcarriers with adhered cells are put in the bioreactor, and the cells are cultured while stirring the microcarriers in a medium. Microcarriers are particles (small particles) of a material to which cells can adhere and used to culture cells on their surfaces (including the surfaces of pores in the case of a porous material). In this sense, microcarriers can be called carrier particles or fine carrier particles. The diameter (particle size) of microcarriers during culture (i.e., when hydrated) is preferably 50-400 $\mu$m, e.g., 80-300 $\mu$m, 80-250 $\mu$m, 100-200 $\mu$m, or 130-180 $\mu$m. Microcarriers may be porous particles with pores extending from their surfaces to their interior. In the case of porous microcarriers, the diameter of the pores (pore size) during cell culture is preferably 3-40 $\mu$m, e.g., 5-25 $\mu$m, 10-20 $\mu$m, and 5-15 $\mu$m.

There is no particular limitation as to the material of which microcarriers are made, and any material that allows cells to be cultured, with the cells adhering to its surfaces, may be used. There are such microcarriers as polymer compounds formed into particles, ceramic particles, glass particles, and the like. Examples of such polymer compounds include, e.g., vinyl resins, epoxy resins, polystyrene, polymethylmethacrylate polyesters, polyamides, silicone resins, phenol resins, melamine resins, urea resins, aniline resins, ionomer resins, polycarbonates, collagen, dextran gelatin, cellulose, alginic acid, and a mixture of them. Microcarriers may be further modified on their surfaces with a cell adhesion molecule. Examples of such cell adhesion molecules include, e.g., gelatin, keratin, elastin, heparan sulfate, dextran, dextran sulfate, chondroitin sulfate, sodium hyaluronate, n-isopropylacrylamide, collagen I to XIX, fibronectin, vitronectin, laminin-1 to 12, tenascin, thrombospondin, osteopontin, fibrinogen, poly-D-lysine, chitin, chitosan, sepharose, laminin, entactin I, or a fragment or a peptide containing the cell adhesion domain of these cell adhesion molecules. The specific weight of microcarriers, relative to the medium, is preferably close to 1, e.g., preferably 0.9-1.2, e.g., 0.9-1.1, and approximately 1.

Particles of gelatin matrix having a dimeter of 130-180 $\mu$m with a pore size of 10-20 $\mu$m (or 5-15 $\mu$m) during cell culture can be suitably used as microcarriers.

Adhesion of cells to microcarriers in production culture is performed by mixing the cells and microcarriers in a solution with stirring. This step is called an adhesion step of cells to microcarriers. In the adhesion step, those cells come into contact with the microcarriers during stirring adhere to their surfaces in sequence. The solution employed in this step is preferably a medium for cell culture, but is not limited to it. Stirring of cells with microcarriers in the adhesion step may also be carried out intermittently. In such a case, the microcarrier are stirred for 1 minute to 20 minutes, and then left to stand for 10 minutes to 2 hours, for example.

In the case where an adhesion step is carried out using a medium for cell culture as a solution, the step may also be performed in a bioreactor. In the case where an adhesion step is carried out in a bioreactor, a medium for cell culture, cells, and microcarriers are put into a bioreactor, and stirred to let the cells adhere to the microcarriers. After the cells adhered, cultivation of the cell may simply be continued in the bioreactor. Stirring of cells and microcarriers in the bioreactor may also be done intermittently. In this case, they are stirred for 1 minute to 20 minutes, and then left to stand for 10 minutes to 2 hours, for example.

The term "bioreactors" generally means biochemical reactors such as immobilized enzyme-reaction apparatuses and biological/microbiological reaction apparatuses such as fermenters and biological liquid waste-treating apparatuses. The bioreactor used in the examples of the present invention described later is suited to cultivation of multipotent stem cell-enriched dental pulp-derived cells adhering to microcarriers in a medium with stirring. The bioreactor is provided with a fermenter for cultivation of cells, a stirrer device for stirring a medium, a gas phase inlet port, a liquid phase inlet port, a drain outlet port, and a sensor part. The fermenter is a container which defines a generally cylindrical cavity. Multipotent stem cell-enriched dental pulp-derived cells adhering to microcarriers can be cultured in the fermenter. The cavity of the fermenter is provided with a stirrer device for a medium in the fermenter. The stirrer device is an impeller, for example, with whose rotation the medium is stirred. Stirring of the medium with the impeller is adjusted so that most of the microcarriers with adhered cells may be suspended in the medium without settling on the bottom of the fermenter's cavity. While the rotation rate of the impeller is to be selected as desired in accordance with the shape of the impeller, the type of the microcarriers, and the like, its examples include 30-100 rpm, 50-80 rpm, and approximately 70 rpm. The gas phase inlet port is for use in supplying a gas phase, such as air, oxygen and carbon dioxide into the fermenter. The gas phase inlet port may be positioned so that its opening inside the cavity lies below the fluid levels of the medium solution, and in this case a gas phase is introduced into the medium in the form of bubbles. The liquid phase inlet port is for use in supplying various solutions, such as a medium, or a glucose solution, into the fermenter. The drain outlet port is for use in discharging the medium in the fermenter, and the medium is discharged through the drain outlet port when the medium is replaced. The drain outlet port is also used to withdraw microcarriers with adhered cells along with the medium. The drain outlet port may be used either for sampling of microcarriers during culture or to recover microcarriers following completion of the culture. The sensor part includes sensors for the measurement of temperature, pH, and dissolved oxygen concentration of the medium in the fermenter. The sensor part may also include sensors for the measurement of glucose concentration, and lactic acid concentration. Sensors for the measurement of temperature, pH, dissolved oxygen concentration, glucose concentration, and lactic acid concentration in the solution may be selected and put in place as desired from those well known in the art.

The fermenter of the bioreactor is either fixed, or removably placed, in the bioreactor. Removably placing the fermenter makes the fermenter easier to wash. In general, the fermenter is made of metal or rigid resin, and repeatedly used after washing following each use. Instead of such a fermenter, it is also possible to set a culture bag made of a flexible resin in the bioreactor, and put the medium and cells in the bag to carry out culture in it. A culture bag is supported in the bioreactor so that it can be handled in the same manner as common fermenters. As they can be disposed of and thus no washing process is needed, culture bags serve to improve efficiency of cultivation process.

In the production culture, when the glucose concentration in the medium comes down below a certain value, the medium is supplemented with glucose. For example, when the glucose concentration comes down to or below 0.1 mM, glucose is added so that its final concentration may come to be 5-7 mM. Further, in the production culture, when the concentration of lactic acid goes up to or over a certain value, the medium is replaced either partly of in its entirety. For example, when the concentration of lactic acid goes up to or over 20 mM, the medium is replaced either in its entirety or part of the medium is replaced so that the concentration of lactic acids comes down to or below 5 mM, or to or below 2 mM, for example.

During culture in the bioreactor, oxygen, air, and $CO_2$ is supplied through the gas phase inlet port. The amount of oxygen supplied is regulated so that the dissolved oxygen concentration in the medium may be kept at 50-100% of saturation concentration, e.g., at 50% or 100%. Further, the flow of $CO_2$ is supplied so that the pH of the medium may be kept at 7.0-7.8 to let cells grow well. As dissolved oxygen concentration and pH are continuously monitored by the senser part, when their values monitored deviate from certain predetermined values, the amount of oxygen and $CO_2$ supplied is adjusted correspondingly to keep the dissolved oxygen concentration and pH within a certain range. It is also possible to supply an additional amount of microcarriers to the bioreactor in accordance with the growth of the cells.

Cultivation in a bioreactor is made until the surfaces of the microcarriers are covered with the cells up to a certain ratio. The ratio may be set as desired, like, e.g., 20%, 40%, 85%, 95%, and 98%. Further, Cultivation in a bioreactor is made until the cell density on the microcarriers' surfaces reaches a certain value. This cell density may be set as desired, like, e.g., 3000, 5000, 10000, 20000, 22000 cells/cm$^2$.

The cells to be frozen as a dental pulp-derived cell preparation following the completion of production culture are preferably such cells that have undergone cell divisions not less than 15 times, and more preferably not less than 20 times since their recovery as those cells which formed colonies on a cell culture plate by cultivation of a dental pulp suspension. For example, those cells are cryopreserved which have undergone cell divisions 15-30 times, 15-23 times, 15-20 times, 20-30 times, 20-28 times, or 23-26 times.

The cells to be frozen as a dental pulp-derived cell preparation following the completion of a production culture via intermediate cells are such cells that have undergone divisions preferably not less than 10 times, and more preferably not less than 15 times since their recovery as those cells which formed colonies on a cell culture plate in cultivation of a dental pulp suspension until they are obtained as intermediate cells. Those cells are cryopreserved as intermediate cells which have undergone divisions, e.g., 13-20 times, 13-23 times, or 14-20 times. And in a production culture, in which intermediate cell are thawed and their culture is started, the cells undergo divisions preferably not less than 5 times, e.g., preferably not less than 8 times or 10 times, from the start of their culture to completion of a dental pulp-derived cell preparation. Where microcarriers are used in a production culture, in particular, the cells undergo divisions preferably 2-5 times, e.g., 2-3 times.

Where cells are cultured on a cell culture plate, the mean doubling time of the cells from starting the culture of intermediate cells to obtainment of a dental pulp-derived cell preparation, is preferably not more than 96 hours, more preferably not more than 84 hours, still more preferably not more than 72 hours, still more preferably not more than 48 hours, and still more preferably not more than 36 hours. Where cells are cultured using microcarriers, a mean doubling time of cells from starting the culture of intermediate cells to obtainment of a dental pulp-derived cell preparation, is preferably not more than 8 days, more preferably not more than 6 days, still more preferably not more than 5 days, still preferably note more than 4 days, and, e.g., 2-8 days, 2-7 days, 2-6 days, 2.5-6 days, or 2.5-5.5 days.

By setting a criterion of mean doubling time of the cells over the entire length of their production culture or during culture using microcarriers, it is also possible to make it a rule that only those groups of cells which show their mean doubling time falling within the criterion are to be cryopreserved for later use. Such a criterion may be set as desired, like not more than 84 hours, not more than 72 house, not more than 48 hours, or not more than 36 hours, for example where cultivation is performed on a cell culture plate, and not more than 8 days, not more than 6 days, not more than 5 days, not more than 4 days, or not more than 3 days where cultivation is performed using microcarriers. Discarding and not cryopreserving those cells which fail to meet this criterion allows that only those cells which have an ability to undergo cell divisions above a certain level to be selected and cryopreserved as a dental pulp-derived cell preparation.

In a production culture, the cells are allowed to grow until a dental pulp-derived cell preparation is obtained, so that the number of multipotent stem cells obtained from an extracted tooth (e.g., a third molar) may expand preferably to not less than $1 \times 10^6$, more preferably not less than $1 \times 10^7$, still more preferably not less than $3 \times 10^7$, still more preferably not less than $1 \times 10^8$, still more preferably not less than $1 \times 10^9$, and, e.g., $1 \times 10^1$ - $1 \times 10^{10}$. Theoretically, the number of cells expands not less than $1 \times 10^6$ times, not less than $3 \times 10^7$ times, and not less than $1 \times 10^9$ times.

(Production of Preparation)

Following the completion of a production culture, microcarriers are recovered with cells adhering to them and washed. While there is no particular limitation as to a solution usable as a wash solution to wash the microcarriers with as far as it does not damage the cells nor inhibit the enzyme activity of serine protease and metalloprotease, in particular trypsin, preferred solutions are physiological saline, PBS, DMEM Low Glucose medium (free of serum), and a wash solution having a composition shown in Table 4, and DMEM Low Glucose medium (free of serum) may be used, for example.

Following washing, the microcarriers are treated with a protease. While there is no particular limitation as to a protease employed here as far as it can decompose proteinous adhesion molecules, preferred are serine protease, metalloprotease, or a mixture of these, among which trypsin is a preferred serin protease, and gelatinase is a preferred metalloprotease. Treated with a protease, the cells are detached from the microcarriers. Further, where the microcarriers are made of a protein such as gelatin, protease treatment will decompose the microcarriers. In this case, if the microcarriers are porous, those cells adhering to inner surfaces of the pores also are detached from the microcarriers. If the microcarriers are made of porous gelatin matrix, protease treatment will decompose gelatin and gives a suspension containing liberated cells and decomposition products of gelatin. In this case, trypsin, gelatinase, or a mixture of these are particularly preferred as proteases.

In the case where the microcarriers are made of a protein, such as gelatin, protease treatment will give a suspension containing cells liberated from the microcarriers and decomposition products of the protein (decomposition products of gelatin if the microcarriers are made of gelatin). To process the liberated dental pulp-derived cells into the form of a preparation, it is preferred to wash the cells so as to remove contaminants such as proteases and decomposition produces of microcarrier materials from the suspension. The process of washing the cells where the microcarriers are made of gelatin is described below in detail.

A suspension obtained by protease treatment can be washed by repeating cycles of recovery following centrifugation and resuspension using a wash solution. Proteases and fine decomposition products of gelatin can be removed utilizing centrifugation. Some of decomposition products of gelatin, however, are debris having sizes with which they settle along with the cells in centrifugation. Such coarse debris can be removed by means of membrane filtration and the like. With this respect, when performing membrane filtration, the pore sizes of a filter membrane is preferably 20-80 μm. For example, filter membranes with their pore sizes of 25 μm, 26 μm, 27 μm, 28 μm, 30 μm, 50 μm, 70 μm, or 80 μm can be used. By first employing a filter membrane having a larger pore size and then a filter membrane having a smaller pore size, filtration using filter membranes can be carried out efficiently while avoiding clogging of pores. For example, the first filtration may be made with a filter membrane having a pore size of 60-100 μm, which is followed by the second filtration with a filter membrane having a pore size of 25-50 μm, or the first filtration may be made with a filter membrane having a pore size of 60-75 μm, which is followed by the second filtration with a filter membrane having a pore size of 25-30 μm. It is also possible to perform filtration only with a filter membrane having a small pore size. The pose size of a filter membrane employed in such a case is preferably 25-50 μm, more preferably 25-30 μm, and, e.g., a filter membrane having a pore size of 25 μm, 26 μm, 27 μm, 28 μm, or 30 μm. By this, coarse debris of gelatin are unable to pass through the filter membrane and left behind, while the cells are recovered in the filtrate. However, it is also possible to prepare unfrozen suspension of cells for preparation, not through recovering the cells but by replacing the solution in which the cells are suspended with a cryopreservation solution described later (cryopreservation solution for cells for a preparation) using an ultrafiltration membrane or the like.

The wash solution employed in the above washing process is for removing the medium and proteases, and also for removing decomposition products where the microcarriers are made of a protein, such as gelatin. There is no particular limitation as to the composition of a wash solution, and while physiological saline, phosphate buffered saline, acetate Ringer's solution, and bicarbonate Ringer's solution may be employed, a bicarbonate Ringer's solution supplemented with human serum albumin is preferred. As acetate Ringer's solution, and bicarbonate Ringer's solution, those commercially available may be employed. For example, PLASMA-LYTE A (Baxter) and Physio 140 (Otsuka Pharmaceutical) may be employed as acetate Ringer's solution, and Bicabon Injection (Ajinomoto) may be used as bicarbonate Ringer's solution. An example of the composition and concentration of electrolytes of a suitable bicarbonate Ringer's solution is shown in Table 2 and Table 3.

A suitable example of composition of a wash solution is shown in Table 4. Further, a suitable example of electrolyte concentration in a wash solution is shown in Table 5. Besides, among the ingredients of a wash solution listed in Table 4 and Table 5, acetyltryptophan sodium and sodium caprylate may be omitted.

Besides, in the case where both centrifugation and membrane filtration are carried out to remove debris after a treatment with a protease, it is also possible either to carry out centrifugation first and then membrane filtration or membrane filtration first and then centrifugation, but it is preferred to carry out membrane filtration first and then centrifugation.

The cells recovered after washing with a wash solution then are suspended in a cryopreservation solution (cryopreservation solution for cells for a preparation). This suspension (unfrozen suspension of cells for preparation) is put in cryopreservation containers, sealed, and frozen to give the optimal form of dental pulp-derived cell preparation suited to preservation and transport. There is no particular limitation as to the composition of the liquid portion of an unfrozen suspension of cells for preparation (cryopreservation solution for cells for a preparation) so far as it enables freezing and thawing mammalian cells, particularly human multipotent stem cell-enriched dental pulp-derived cells, without causing their death. A cryopreservation solution for cells for a preparation contains a cell protective agent. A cell protective agent is, for example, a compound that serves to suppress formation ice crystals within the cells during they are being frozen, which otherwise could destroy cells. Preferable examples of cell protective agents include dimethyl sulfoxide (DMSO), ethylene glycol, propylene glycol, sericin, and glycerol. A combination of two or more of these may also be used. When dimethyl sulfoxide is employed as a cell protective agent, its concentration is preferably 5-15% (v/v), more preferably 9-11% (v/v), and, e.g., 10% (v/v). A cryopreservation solution may contain a buffering agent. A buffering agent capable of adjusting the pH of a solution to 6-8, and, e.g., 6.8-7.8 is preferred. Examples of such buffering agents include those containing carbonate ion, bicarbonate ion, citrate ion, and sodium ion, for example. Cryopreservation solution for cells for a preparation may also contain human serum albumin. Where human serum albumin is used, its concentration is preferably 40-100 g/L, more preferably 46-56 g/L, and, e.g., 51 g/L.

A bicarbonate Ringer's solution supplemented with human serum albumin and dimethylsulfoxide is a suitable example of a cryopreservation solution for cells for a preparation. A cryopreservation solution for cells for a preparation preferably contain 59-80.4 mM sodium chloride, 2.3-3.08 mM potassium chloride, 0.85-1.16 mM calcium chloride dihydrate, 0.28-0.385 mM magnesium chloride hexahydrate, 14-19.2 mM sodium bicarbonate, 0.94-1.28 mM sodium citrate dihydrate, 46-56 g/L human serum albumin, 3.73-4.55 mM acetyltryptophan sodium, 3.74-4.58 mM sodium caprylate, and 9-11% (v/v) DMSO. The cryopreservation solution for intermediate cells shown in Table 6 may be suitably used also as a cryopreservation solution for cells for a preparation.

Namely, a solution which can be used as a cryopreservation solution for intermediate cells generally can be suitably used as a cryopreservation solution for cells for a preparation. For example, its composition is as follows: 65.8-80.4 mM sodium chloride, 2.52-3.08 mM potassium chloride, 0.95-1.16 mM calcium chloride dihydrate, 0.315-0.385 mM magnesium chloride hexahydrate, 15.7-19.2 mM sodium bicarbonate, 1.04-1.28 mM sodium citrate dihydrate, 46-56 g/L human serum albumin, 3.73-4.55 mM acetyltryptophan sodium, 3.74-4.58 mM sodium caprylate, and 9-11% (v/v) DMSO. In addition to the composition shown in Table 6, 70.8-71.3 mM sodium chloride, 2.71-2.73 mM potassium chloride, 1.01-1.02 mM calcium chloride dihydrate, 0.335-0.345 mM magnesium chloride hexahydrate, 16.9-17.0 mM sodium bicarbonate, 1.12-1.14 mM sodium citrate dihydrate, 53.6-54.3 g/L human serum albumin, 4.36-4.41 mM acetyltryptophan sodium, 4.37-4.43 mM sodium caprylate, 10.6-10.9% (v/v) DMSO. Besides, among these, acetyltryptophan sodium and sodium caprylate may be omitted. The osmotic pressure ratio of the cryopreservation solution for cells for a preparation to physiological saline is preferably 0.9-1.1.

Further, a solution containing sodium ion, potassium ion, calcium ion, magnesium ion, bicarbonate ion, citrate ion, human serum albumin, and dimethylsulfoxide can be suitably used as cryopreservation solution for intermediate cells. For example, it is a suitable solution that contains 91-113 mM sodium ion, 2.52-3.08 mM potassium ion, 0.95-1.16 mM calcium ion, 0.315-0.385 mM magnesium ion, 15.6-19.2 mM bicarbonate ion, 1.04-1.28 mM citrate ion, 46-56 g/L human serum albumin, and 9-11% (v/v) dimethylsulfoxide. Further, it is an example of a suitable solution that contains 100-102 mM sodium ion, 2.71-2.77 mM potassium ion, 1.01-1.03 mM calcium ion, 0.335-0.345 mM magnesium ion, 16.9-17.2 mM bicarbonate ion, 1.13-1.15 mM citrate ion, 52.2-54.3 g/L human serum albumin, and 10.3-10.9% (v/v) dimethylsulfoxide. Furthermore, it is an example of a suitable solution that contains 102 mM sodium ion, 2.80 mM potassium ion, 1.05 mM calcium ion, 0.35 mM magnesium ion, 17.4 mM bicarbonate ion, 1.16 mM citrate ion, 51 g/L human serum albumin, and 10% (v/v) dimethylsulfoxide. Furthermore, it is an example of a suitable solution that contains 101 mM sodium ion, 2.77 mM potassium ion, 1.03 mM calcium ion, 0.34 mM magnesium ion, 17.2 mM bicarbonate ion, 1.15 mM citrate ion, 52 g/L human serum albumin, and 10% (v/v) dimethylsulfoxide. Furthermore, it is an example of a suitable solution that contains 100 mM sodium ion, 2.71 mM potassium ion, 1.01 mM calcium ion, 0.34 mM magnesium ion, 16.9 mM bicarbonate ion, 1.13 mM citrate ion, 53.6 g/L human serum albumin, and 10.7% (v/v) dimethylsulfoxide.

In the case where acetyltryptophan or its salt is contained in addition, its concentration is preferably, 3.73-4.55 mM, more preferably 4.24-4.41 mM, and, e.g., 4.14 mM, 4.24 mM, 4.36 mM or the like. In the case where caprylic acid or its salt is contained in addition, its concentration is preferably 3.74-4.58 mM, more preferably 4.25-4.43 mM, and, e.g., 4.16 mM, 4.25 mM, 4.37 mM, or the like.

While there is no particular limitation as to the density of the cells suspended in a cryopreservation solution for cells for a preparation, it is preferably $5 \times 10^6$-$8 \times 10^7$ cells/mL, $1.5 \times 10^7$-$3 \times 10^7$ cells/mL, more preferably $2.1 \times 10^7$-$3.0 \times 10^7$ cells/mL, and, e.g., $2.5 \times 10^7$ cells/mL. Multipotent stem cell-enriched dental pulp-derived cells in a suspension are dispensed in cell cryopreservation containers in aliquots, and then cryopreserved.

Though it varies depending on the density of the cells suspended, the composition of a cell suspension prepared by suspending multipotent stem cell-enriched dental pulp-derived in a cryopreservation solution for cells for a preparation generally contains 59-61 mM sodium chloride, 2.3-2.6 mM potassium chloride, 0.85-0.98 mM calcium chloride dihydrate, 0.28-0.32 mM magnesium chloride hexahydrate, 14-16 mM sodium bicarbonate, 0.94-1.08 mM sodium citrate dihydrate, 49-50 g/L human serum albumin, 4.0-4.1 mM acetyltryptophan sodium, 4.0-4.1 mM sodium caprylate, and 9-11% (v/v) DMSO.

While the amount of the cell suspension dispensed in a single cell cryopreservation container should be adjusted as desired, it is preferably 1-20 mL. And the number of cells dispensed in a single cell cryopreservation container is preferably $5 \times 10^6$-$9.2 \times 10^8$ cells.

A container suitable to cryopreservation of suspended cells in a cryopreservation solution for cells for a preparation, procedures and conditions in freezing and preservation are the same as those described regarding cryopreservation of intermediate cells.

The cells cryopreserved as described above can be used as a medicine containing multipotent stem cell-enriched dental pulp-derived cells as an active ingredient (dental pulp-derived cell preparation). In that case, the multipotent stem cell-enriched dental pulp-derived cells are transported in a frozen state, thawed before use, and administered to a patient

Immediately after thawing, the cells obtained through a production culture and then contained in the dental pulp-derived cell preparation shows a ratio of viable cells of preferably not less than 50%, more preferably not less than 60%, still more preferably not less than 70%, and, e.g., preferably not less than 80%, not less than 90%, or not less than 95%. Based on a criterion set as desired, it is possible to make it a rule that only those cells showing a ratio of viable cells not lower than a certain value are to be supplied as a dental pulp-derived cell preparation, i.e., as a medicine.

The cells contained in the dental pulp-derived cell preparation are observed as a whole to express increased levels of aggrecans when cultured in a medium containing a compound known to induce differentiation into chondrocytes. Further, the cells are observed as a whole to exhibit an increase in the amount of accumulated calcium in the cells when cultured in a medium containing a compound known to induce differentiation into osteocytes. Namely, the cells when observed as a whole have abilities to differentiate into chondrocytes and osteocytes.

In an embodiment of the present invention, the cells contained in the dental pulp-derived cell preparation express angiopoietin-1 (Ang-1) at levels of 86-161 pg /$10^4$ cells, which expression levels are preferably not less than 3 times, more preferably not less than 5 times, still more preferably not less than 6 times, and still more than 8 times, as compared with human bone marrow-derived mesenchymal stem cells, which also are multipotent stem cells. The expression levels of angiopoietin-1 were determined by a method described in Example 30, for example.

In an embodiment of the present invention, when the cells contained in the dental pulp-derived cell preparation and human bone marrow-derived mesenchymal stem cells are respectively cultured, the amount of angiopoietin-1 produced by the dental pulp-derived cells is preferably not less than 5 times, more preferably not less than 6 times, still more preferably note less than 8 times, as compared with the amount of angiopoietin-1 produced by human bone marrow-derived mesenchymal stem cells. The method used here to culture these cells is the one described in Example 30, for example.

In an embodiment, when observed as a whole with respect to their expression pattern of surface antigen markers, the cells contained in the dental pulp-derived cell preparation are positive for at least one of CD73, CD90, CD105, and CD166. In the same manner, the cells are negative for at least one of CD34 and CD45. For example, when observed as a whole with respect to their expression pattern of surface antigen markers immediately after they are thawed, or thawed and cultured, the cells contained in the dental pulp-derived cell preparation are positive for at least CD73 and CD90, while negative for CD34, or, for example, positive for CD73, CD90, CD105, and CD166, while negative for CD34 and CD45.

Further, in an embodiment, when observed as a whole with respect to their expression pattern of surface antigen markers, the cells contained in the dental pulp-derived cell preparation are negative at least one of CD40, CD80, CD86, and MHC-class II antigen. For example, when observed as a whole with respect to their expression pattern of surface antigen markers immediately after they are thawed, or thawed and cultured, the cells contained in the dental pulp-derived cell preparation are negative for CD40, CD80, CD86, and MHC-class II antigen. It is known that those cells which are positive for these surface antigen markers, when implanted in an allogeneic individual, tend to be excluded from the living body by being recognized as an antigen. Being negative for at least one of these surface antigen markers indicates that the multipotent stem cell-enriched dental pulp-derived cells are correspondingly less antigenic and therefore less tend to be excluded from the living body when implanted in an allogenic individual. Further, with respect to the expression pattern of surface antigen markers, when the cells are stimulated with IFN-$\gamma$, CD40, CD80, and CD86 remain negative, while MHC-class II antigen may turn positive. For example, when the cells are stimulated with IFN-$\gamma$, CD40, CD80, and CD86 remain negative, while MHC-class II antigen turns positive.

Further, in an embodiment, when observed as a whole with respect to their expression pattern of surface antigen markers immediately after they are thawed, or thawed and cultured, the cells contained in the dental pulp-derived cell preparation are positive, for example, for CD73, CD90, CD105, and CD166, while negative for CD34, CD40, CD45, CD80, CD86, and MHC-class II antigen. When the cells are stimulated with IFN-$\gamma$, CD40, CD80, and CD86 remain negative, while MHC-class II antigen turns positive.

In an embodiment, the cells contained in the dental pulp-derived cell preparation,

(d-1) when observed as a whole with respect to their expression pattern of surface antigen markers, are positive for at least one of, and preferably all of CD29, CD44, CD59, and CD164. Wherein, when observation is made cell-by-cell in the whole cell population, preferably not less than 80%, more preferably not less than 90%, and still more preferably not less than 95% of the observed cells are positive for these antigens.

Further, in an embodiment, the cells contained in the dental pulp-derived cell preparation, (d-2) when observed as a whole with respect to their expression pattern of surface antigen markers, are positive for at least one of, and preferably all of CD9, CD13, CD46, CD47, CD58, CD63, CD73, CD81, CD90, CD98, CD147, HLA-A, B, C, and EGF-R. Wherein, when observation is made cell-by-cell in the whole cell population, preferably not less than 70%, more preferably not less than 80%, and still more preferably not less than 90% of the observed cells are positive for these antigens.

Further, in an embodiment, the cells contained in the dental pulp-derived cell preparation, (d-3) when observed as a whole with respect to their expression pattern of surface antigen markers, are positive for at least one of, and preferably all of CD49b, CD49c, CD49e, CD55, CD95, CD151, and CD166. Wherein, when observation is made cell-by-cell in the whole cell population, preferably not less than 65%, more preferably not less than 75%, and still more preferably not less than 80% of the observed cells are positive for these antigens.

Further, in an embodiment, the cells contained in the dental pulp-derived cell preparation, (d-4) when observed as a whole with respect to their expression pattern of surface antigen markers, are positive for at least one of, and preferably all of CD10, CD49f, CD105, and CD140b. Wherein, when observation is made cell-by-cell in the whole cell population, preferably not less than 60%, more preferably not less than 70%, and more preferably not less than 75% of the observed cells are positive for these antigens.

In an embodiment, the cells contained in the dental pulp-derived cell preparation have preferably not less than 2, more preferably not less than 3, and still more preferably all of the characteristics (d-1), (d-2), (d-3), and (d-4) defined above. For example, cells having characteristics (d-1) and (d-2) defined above is one of suitable embodiments of the present invention.

In an embodiment, the cells contained in the dental pulp-derived cell preparation,

(d-5) when observed as a whole with respect to their expression pattern of surface antigen markers, are negative for at least one of, and preferably all of CD1a, CD1d, CD2, CD3, CD4, CD5, CD7, CD8a, CD8b, CD11b, CD11c, CD15, CD15s, CD16, CD18, CD19, CD20, CD21, CD22, CD23, CD24, CD25, CD28, CD30, CD31, CD32, CD33, CD34, CD35, CD37, CD38,CD41a, CD86, CD87, CD88, CD89, CDw93, CD94, CD100, CD102, CD103, CD114, CD117, CD118, CD120b, CD121b, CD122, CD123, CD124, CD126, CD127, CD128b, CD132, CD134, CD135, CD137, CD137 ligand, CD138, CD144, CD150, CD153, CD154, CD158a, CD158b, CD161, CD162, CD163, CD172b, CD177,CD178, CD180, CD184, CD195, CD196, CD197, CD205, CD206, CD210, CD212, CD220, CD226, CD229, CD231, CD235a, CD244, CD255, CD267, CD268, CD278, CD279, CD282, CD294, CD305, CD309, CD314, CD321, CDw327, CDw328, CD329, CD335, CD336, CD337, BLTR-1, CLIP, CMRF-44, CMRF-56, fMLP-R, SSEA-1, TRA-1-60, CLA, integrin $\beta$7, and Invariant NKT. Wherein, when observation is made cell-by-cell in the whole cell population, preferably not more than 10%, more preferably not more than 5%, still more preferably not more than 2%, and still more preferably only not more than 1% of the observed cells are positive for these antigens.

In an embodiment, the cells contained in the dental pulp-derived cell preparation,

(d-6) when observed as a whole with respect to their expression pattern of surface antigen markers, are negative for at least one of, and preferably all of CD1b, CD6, CD27, CD41b, CD42a, CD42b, CD43, CD45, CD45RB, CD48, CD50, CD53, CD57, CD62E, CD62L, CD62P, CD64, CD66b, CD66f, CD69,CD70, CD72, CD75, CD84, CD85, CD97, CD99R, CD183, CD193, SSEA-3, and $\gamma\delta$TCR. Wherein, when observation is made cell-by-cell in the whole cell population, preferably not more than 10%, more preferably not more than 5%, still more preferably only not more than 2% of the observed cells are positive for these antigens.

In an embodiment, the cells contained in the dental pulp-derived cell preparation,

(d-7) when observed as a whole with respect to their expression pattern of surface antigen markers, are negative for at least one of, and preferably all of CD4v4, CD14, CD36, CD45RA, CD45RO, CD66 (a, c, d, e), CD79b, CD83, CD106, CD152, CD209, CD271, CD275,CD326, MIC A/B, and $\alpha\beta$TCR. Wherein, when observation is made cell-by-cell in the whole cell population, preferably not more than 10%, and more preferably only not more than 5% of the observed cells are positive for these antigens.

In an embodiment, the cells contained in the dental pulp-derived cell preparation,

(d-8) when observed as a whole with respect to their expression pattern of surface antigen markers, are negative for at least one of, and preferably all of CD26, CD40, CD56, CD80, CD146, and MHC-class II antigen. Wherein, when observation is made cell-by-cell in the whole cell population, preferably not more than 20%, and more preferably only not more than 10% of the observed cells are positive for these antigens.

In an embodiment, the cells contained in the dental pulp-derived cell preparation have preferably not less than 2, more preferably not less than 3, and still more preferably all of the characteristics (d-1), (d-2), (d-3), (d-4), (d-5), (d-6), (d-7), and (d-8) defined above. For example, cells having the characteristics (d-1) and (d-5) defined above are one of the preferable embodiments of the present invention.

Further, when observed as a whole, the cells contained in the dental pulp-derived cell preparation immediately after they are thawed, or thawed and cultured, express prostaglandin $E_2$ (PGE$_2$) and/or vascular endothelial growth factor (VEGF), wherein the expression levels of prostaglandin $E_2$ (PGE$_2$) increases when the cells are stimulated with TNF$\alpha$.

Further, in an embodiment, when cultured in the presence of IFN-yt, the cells contained in the dental pulp-derived cell preparation exhibit an increase in the amount of kynurenine secreted.

In an embodiment, when observed as a whole with respect to their expression pattern of surface antigen markers, the cells contained in the dental pulp-derived cell preparation are positive for at least one of, or all of CD47, CD81, and CD147, while negative for at least one, or preferably all of CD19, CD34, and CD206. Further, in an embodiment of the present invention, when observed as a whole with respect to their expression pattern of surface antigen markers, the cells contained in the dental pulp-derived cell preparation are positive for at least one of, or all of CD47, CD81, and CD147, while negative for at least one of, or for all of CD19, CD31, CD33, CD34, CD38, CD45, CD206, CD235a, and SSEA-1.

In one embodiment, when observed as a whole with respect to their expression pattern of surface antigen markers, the cells contained in the dental pulp-derived cell preparation are negative for at least one of, and, e.g., for all of CD19, CD26, CD34, CD56, CD106, CD117, CD146, and CD271.

In an embodiment, when observed as a whole with respect to their expression pattern of surface antigen markers, the cells contained in the dental pulp-derived cell preparation are positive for at least one of, and, e.g., for all of CD140b and HLA-A, B, C.

In an embodiment, when observed as a whole with respect to their expression pattern of surface antigen markers, the cells contained in the dental pulp-derived cell preparation are positive for at least one of, and, e.g., for all of CD10, CD49e and CD95.

In an embodiment of the present invention, when observed as a whole with respect to their expression pattern of surface antigen markers, the cells contained in the dental pulp-derived cell preparation are positive for at least one of, and, e.g., for all of CD46, CD47, CD55, CD58, and CD59.

In an embodiment of the present invention, when observed as a whole with respect to their expression pattern of surface antigen markers, the cells contained in the dental pulp-derived cell preparation are, for example, positive for at least one of CD73, CD90, CD105, and CD166, while negative for at least one of CD34 and CD45.

Further, when observed as a whole immediately after they are thawed, or thawed and cultured, the cells contained in the dental pulp-derived cell preparation are positive for CD73, CD90, CD105, and CD166, while negative for CD34, CD40, CD45, CD80, CD86, and MHC-class II antigen. Further, when the cells are stimulated with IFN-γ, CD40, CD80, and CD86, for example remain negative, while MHC-class II antigen turns positive. Further, they express prostaglandin $E_2$ ($PGE_2$) and/or vascular endothelial growth factor (VEGF), wherein the amount of prostaglandin $E_2$ ($PGE_2$) secreted increases when the cells are stimulated with TNFα.

When cultured, the cells contained in the dental pulp-derived cell preparation secrete various humoral factors.

(d-9) In an embodiment, when cultured, the cells contained in the dental pulp-derived cell preparation express at least one of, and preferably all of MMP-2, IGFBP-4, and cystatin C.

(d-10) Further, in an embodiment, when cultured, the cells contained in the dental pulp-derived cell preparation express at least one of, and preferably all of IL-6, IL-11, MCP-1, IL-8, GROα, HGF, VEGF, VCAM-1, TIMP-3, TIMP-2, and TIMP-1.

(d-11) Further, in an embodiment, when cultured, the cells contained in the dental pulp-derived cell preparation express at least one of, and preferably all of IL-23, IFN-α, TNF-α, IL-18, IL-27, TARC, ENA-78, MIP-3α, MIP-1β, IP-10 (CXCL10), SCF, and ICAM-1.

(d-12) Further, in an embodiment, when cultured, the cells contained in the dental pulp-derived cell preparation does not express, or scarcely express, at least one of, or any of IL-21, Eotaxin, MIP-1α, MIG, I-TAC, IP-10 (CXCL10), and GM-CSF.

(d-13) Further, in an embodiment, when cultured, the cells contained in the dental pulp-derived cell preparation show increased levels of expression of at least one of IL-6, IL-23, IL-11, MCP-1, ENA-78, HGF, VEGF, MMP-2, IGFBP-4, cystatin C, TIMP-3, TIMP-2, and TIMP-1, as compared with their expression levels observed in intermediate cells. For example, they show increased levels of expression of IL-6, IL-11, HGF, TIMP-3, and TIMP-1, as compared with their expression levels observed in intermediate cells.

(d-14) Further, in an embodiment, when cultured in the presence of TNF-α, or in the presence of IFN-γ, the cells contained in the dental pulp-derived cell preparation shows an increased level of expression of IL-6, as compared with when cultured in their absence.

(d-15) Further, in an embodiment, when cultured in the presence of TNF-α, or in the presence of IFN-γ, the cells contained in the dental pulp-derived cell preparation shows an increased level of expression of IL-11, as compared with when cultured in their absence.

(d-16) Further, in an embodiment, when cultured in the presence of TNF-α, or in the presence of IFN-γ, the cells contained in the dental pulp-derived cell preparation shows an increased level of expression of IP-10 (CXCL 10), as compared with when cultured in their absence.

(d-17) Further, in an embodiment, when cultured in the presence of TNF-α, or in the presence of IFN-γ, the cells contained in the dental pulp-derived cell preparation shows an increased level of expression of MCP-1, as compared with when cultured in their absence.

(d-18) Further, in an embodiment, when cultured in the presence of TNF-α, the cells contained in the dental pulp-derived cell preparation are induced to express GM-CSF, whereas no induction of GM-CSF expression is shown when they are cultured in the presence of IFN-γ.

(d-19) Further, in an embodiment, when cultured in the presence of TNF-α, the cells contained in the dental pulp-derived cell preparation show a deceased level of HGF expression as compared with when they are cultured in its absence, whereas they show an increased level of HGF expression when cultured in the presence of as compared with IFN-γ, as compared when they are cultured in its absence.

(d-20) Further, in an embodiment, when cultured in the presence of TNF-α, the cells contained in the dental pulp-derived cell preparation show an increased level of IL-8 as compared with when they are cultured in its absence.

(d-21) Further, in an embodiment, when cultured in the presence of TNF-α, the cells contained in the dental pulp-derived cell preparation are induced to express G-CSF, whereas no induction of GM-CSF expression is shown when they are cultured in the presence of IFN-γ.

In an embodiment, the cells contained in the dental pulp-derived cell preparation have not less than 2, preferably not less than 3, more preferably not less than 4, and still more preferably all of the characteristics (d-9)-(d-21) defined above. For example, cells having characteristics (d-9), (d-16), and (d-21) defined above, and cells having characteristics (d-9), (d-15), (d-16) and (d-21) defined above, are preferred embodiments of the present invention.

Further, when observed as a whole immediately after they are thawed, or thawed and cultured, the cells contained in the dental pulp-derived cell preparation show an increased expression level of aggrecans where the culture is carried out in a medium containing a compound known to induce differentiation into chondrocytes. Further, when observed as a whole, the cells contained in the dental pulp-derived cell preparation, show an increase in the amount of accumulated calcium in the cells where the culture is carried out in a medium containing a compound known to induce differentiation into osteocytes. Namely, the cells contained in the dental pulp-derived cell preparation have abilities to differentiate into chondrocytes and osteocytes. That the cells contained in the dental pulp-derived cell preparation have abilities to differentiate into chondrocytes and osteocytes can be determined by the methods described in Example 20 and Example 19, respectively. Using those methods, it is possible to make it a rule that only those groups of cells confirmed to have abilities to differentiate into chondrocytes and osteocytes may be supplied to medical facilities as the product.

Based on a criterion set as desired, it is possible to make it a rule that only those groups cells which exhibit a predetermined expression pattern of surface antigen markers and/or expression pattern of genes may be preserved as a dental pulp-derived cell preparation.

Further, when observed as a whole immediately after they are thawed, or thawed and cultured, the cells contained in the dental pulp-derived cell preparation consist almost entirely of dental pulp-derived multipotent stem cells. Under an optical microscope during plate culture, they are observed as generally spindle-shaped adhering cells. When observed under an optical microscope during plate culture, the ratio of spindle-shaped cells to all the cells is preferably not less than 99%, more preferably not less than 99.5%, still more preferably not less than 99.9%, and still more preferably not less than 99.95%. Based on a criterion set as desired as mentioned above, it is also possible to make it a rule that only those dental pulp-derived cell preparation whose ratio of generally spindle-shaped cells are equal to or greater than a certain standard is to be supplied to medical facilities as a product.

When thawed and cultured, the cells contained in the dental pulp-derived cell preparation have an ability to undergo cell divisions not less than 3 times, preferably not less than 4 times, still more preferably not less than 5 times, and still more preferably not less than 10 times. Further, when thawed and cultured on a cell culture plate, the cells contained in the dental pulp-derived cell preparation exhibit their mean doubling time of preferably not more than 96 hours, more preferably not more than 84 hours, still more preferably not more than 72 hours, still more preferably not more than 48 hours, and still more preferably not more than 36 hours. A preferable set of cell culture conditions here is the same or substantially same as those for multipotent stem cell-enriched dental pulp-derived cells as described hereinbefore, and for example, the conditions described in Example 5 are employed. Based on a criterion set as desired, it is possible to make it a rule that only those dental pulp-derived cell preparation showing to have an ability to undergo cell divisions that is not lower than a certain level is to be supplied to medical facilities as a product. Further, based on a criterion set as desired, it is possible to make it a rule that only those dental pulp-derived cell preparation showing a mean doubling time falling within a certain range are to be supplied to medical facilities as a product. Such criteria can be set as desired, namely for example, having an ability to undergo cell divisions not less than 3 times and mean doubling time of not more than 72 hours; an ability to undergo cell division not less than 4 times and mean doubling time of not more than 72 hours, and an ability to undergo cell divisions not less than 5 times and mean doubling time of not more than 72 hours.

Thus, the cells contained in the dental pulp-derived cell preparation have properties of stem cells, i.e., a high ability to undergo cell divisions and ability to differentiate two or more different kinds of cells. The cells contained in the dental pulp-derived cell preparation can be said as cells having a high colony-forming ability.

The cells contained in the dental pulp-derived cell preparation are those having undergone cell divisions in an in vitro environment preferably not less than 16 times, and for example, not less than 21 times, not less than 22 times, or not less than 23 times.

The cells contained in the dental pulp-derived cell preparation, when thawed and suspended in a solution, e.g., a culture medium, have their mean diameter preferably not more than 20 $\mu$m, not more than 18 $\mu$m, and, e.g., 10-20 $\mu$m, 10-18 $\mu$m, 12-20 $\mu$m, 14-20 $\mu$m, 16-20 $\mu$m.

Examples of items of quality inspections performed on the cells contained in the dental pulp-derived cell preparation are listed as Quality Inspections a'-k'. Quality inspections are performed on one or more of those items. To perform inspections on all the items is also allowed. Cells to be subjected to an inspection are any one of (1) part of cells dispensed before they are suspended in a cryopreservation solution as the dental pulp-derived cell preparation, (2) part of cells which are dispensed before they are suspended in a cryopreservation solution as the dental pulp-derived cell preparation, (3) cells suspended but not frozen in a cryopreservation solution to be cryopreserved as the dental pulp-derived cell preparation, (4) cells prepared immediately after thawing of those cells once frozen as the dental pulp-derived cell preparation, or (5) cells obtained by culturing the cells that were prepared by thawing cells once frozen as the dental pulp-derived cell preparation. Unless specified otherwise, the same quality inspections may be performed on 2 or more groups of cells identified in (1)-(5) above.

(Quality Inspection a') Verification that, when observed under an optical microscope, the ratio of generally spindle-shaped cells to all the cells is not less than 99%, not less than 99.5%, not less than 99.9%, or not less than 99.95%.

(Quality Inspection b') Verification that the viable cell ratio is not less than 50%, not less than 60%, not less than 70%, not less than 80%, not less than 90%, or not less than 95%:

(Quality Inspection c') Verification that with respect to their expression pattern of surface antigen markers, the cells are positive for CD73, CD90, CD105, and CD166, while negative for CD34 and CD45, or verification that with respect to their expression pattern of surface antigen markers, the cells are positive for at least one of CD73 and CD90, while negative for CD34.

(Quality Inspection d') Verification that with respect to their expression pattern of surface antigen markers, the cells are negative for CD40, CD80, CD86, and MHC-class II antigen, or verification that with respect to their expression pattern of surface antigen markers, the cells are negative for at least one of CD40, CD80, CD86, and MHC-class II antigen.

(Quality Inspection e') Verification that with respect to their expression pattern of surface antigen markers, when the cells are stimulated with IFN-$\gamma$, CD40, CD80, and CD86 remain negative, while MHC-class II antigen turns positive, or at least one verification that with respect to their expression pattern of surface antigen markers, when the cells are stimulated with IFN-$\gamma$, the cells are negative for CD40, negative for CD80, negative for CD86, negative for MHC-class II antigen.

(Quality Inspection f') Verification that the cells express prostaglandin $E_2$ ($PGE_2$) and/or vascular endothelial growth factor (VEGF), and the expression levels of prostaglandin $E_2$ ($PGE_2$) increases when the cells are stimulated with TNF$\alpha$.

(Quality Inspection g') Verification that when cultured in a medium containing a compound known to induce differentiation into chondrocytes, the cells exhibit increased levels of aggrecan expression.

(Quality Inspection h') Verification that when cultured in a medium containing a compound known to induce differentiation into osteocytes, the cells exhibit an increase in the amount of accumulated calcium in the cells.

(Quality Inspection i') Verification that the cells have an ability to undergo cell divisions on a cell culture plate not less than 3 times, not less than 4 times, or not less than 5 times.

(Quality Inspection j') Verification that during the above inspection i', the cells' mean doubling time on a cell culture plate is not more than 96 hours, not more than 84 hours, not more than 72 hours, not more than 48 hours, or not more than36 hours.

(Quality Inspection k') Verification that the mean diameter of the cells, when they are suspended in a culture medium, is not more than 20 $\mu$m, not more than 18 $\mu$m, 10-20 $\mu$m, 10-18$\mu$m, 12-20 $\mu$m, 14-20 $\mu$m, or 16-20 $\mu$m.

It is permitted that quality inspection is performed on arbitrary 10 items of (Quality Inspection a') to (Quality Inspection j'). Further, It is also permitted that quality inspection is performed on 9 items chosen arbitrarily from the above 10 items. When 9 items are chosen to perform quality inspection, Quality Inspections a', b', c', d', e', f', g', i', and j' may be chosen, for example but without limitation. Further, it is also permitted to perform quality inspections on 8 items chosen arbitrarily from the above 10. When quality inspection is performed on 8 items chosen, Quality Inspections a', b', c', d', e', f', i', and j' may be chosen, for example but without limitation. Further it is also permitted to perform quality inspections on 7 items chosen arbitrarily from the above 10. When 7 items are chosen to perform quality inspection, Quality Inspections a', b', c', d', e', i', and j' may be chosen, for example but without limitation. Further, it is also permitted to perform quality inspection on 6 items chosen arbitrarily from the above 10. When 6 items are chosen to perform quality inspection, Quality Inspections a', b', c', d', i', and j' may be chosen, for example but without limitation. Further, it is also permitted to perform a quality inspection on 5 items chosen arbitrarily from the above 10. When 5 items are chosen to perform quality inspection, Quality Inspection a', b', c', i', and j' may be chosen, for example but without limitation. Furthermore, it is also permitted to perform a quality inspection on 4 items chosen arbitrarily from the above 10. When 4 items are chosen to perform quality inspection, Quality Inspection a', b', c', and j' may be chosen for example but without limitation. Furthermore, it is also permitted to perform quality inspection on 3 items chosen arbitrarily from the above 10. When 3 items are chosen to perform quality inspection, Quality Inspection a', b', and c' may be chosen, for example but without limitation. Still farther, it is also permitted to perform a quality inspection on 2 items chosen arbitrarily from the above 10. When 2 items are chosen to perform quality inspection, quality inspection may be performed, for example without limitation, on Quality Inspections a' and b', Quality Inspections a' and c', Quality Inspections a' and d', Quality Inspections a' and e', Quality Inspections a' and f', Quality Inspections a' and g', Quality Inspections a' and h', Quality Inspections a' and i', Quality Inspections a' and j', Quality Inspections b' and c', Quality Inspections b' and d', Quality Inspections b' and e', Quality Inspections b' and f', Quality Inspections b' and g', Quality Inspections b' and h', Quality Inspections b' and i', Quality Inspections b' and j', Quality Inspections c' and d', Quality Inspections c' and e', Quality Inspections c' and f', Quality Inspections c' and g', Quality Inspections c' and h', Quality Inspections c' and i', Quality Inspections c' and j', Quality Inspections d' and e', Quality Inspections d' and f', Quality Inspections d' and g', Quality Inspections d' and h', Quality Inspections d' and i', Quality Inspections d' and j', Quality Inspections e' and f,'

Quality Inspections e' and g', Quality Inspections e' and h', Quality Inspections e' and i', Quality Inspections e' and j', Quality Inspections f' and i', Quality Inspections f' and j', or Quality Inspections i' and j,' for example but without limitation.

The method for production of the dental pulp-derived cells enriched in multipotent stem cells of the present invention comprises culturing multipotent stem dells contained in a dental pulp, cryopreserving the multipotent stem cells thus proliferated as intermediate cells, and thawing and further culturing them to let them grow into a dental pulp-derived cell preparation. The process starting with intermediate cells to obtainment of a dental pulp-derived cell preparation include a step of culturing the multipotent stem cells, with the cells adhering to the surfaces of particles. In an embodiment, by inclusion of this step, the cells contained in a dental pulp-derived cell preparation comes to have properties which are different from those of intermediate cells.

The cells contained in the dental pulp-derived cell preparation secrete various humoral factors. In an embodiment, the cells contained in the dental pulp-derived cell preparation express angiopoietin-1 in an amount of 86-161 pg/$10^4$ cells, and the amount of expression is preferably not less than 3 times, more preferably not less than 5 times, still more preferably not less than 6 times, and still more preferably not less than 8 times greater than human bone marrow-derived mesenchymal stem cells, which also are multipotent stem cells. The amount of angeopoietin-1 expression here is measured by e.g., the method described in Example 30.

Further, in an embodiment of the present invention, the amount of angiopoietin-1 produced by the cells contained in the dental pulp-derived cell preparation is preferably not less than 3 times, more preferably not less than 5 times, and still more preferably not less than 8 times greater than the amount of angiopoietin-1 produced by human bone marrow-derived mesenchymal stem cells, when the cells contained in the dental pulp-derived cell preparation and the human bone marrow-derived mesenchymal stem cells are respectively cultured. The method for culturing those cells employed here is the one described in, e.g., Example 30.

The pharmaceutical composition according to the present invention preferably have at least one of the characteristics as defined in (1) to (8) below.

(1) That in an assay of action on vascular permeability utilizing human umbilical vein endothelial cells (HUVEC), when the dental pulp-derived cells are cocultured with HUVEC at a mixing ratio of 50%, the supernatant thus obtained preferably differs significantly from the supernatant obtained from a culture of HUVEC alone, in the rate of an HUVEC monolayer's permeability to a fluorescent compound (FITC-dextran), and more preferably the rate of permeation is not more than 70%.

(2) That in an assay of action on neutrophil infiltration into the lungs using a lipopolysaccharide (LPS)-induced acute lung injury mouse model, the dental pulp-derived cells administered at a dose of $1.5 \times 10^3$ cells suppress, preferably significantly, the infiltration as compared with a medium administration (bicarbonate Ringer's solution supplemented with human serum albumin and DMSO).

(3) That in an assay of action on VCAM-1 protein expression in the lungs using a lipopolysaccharide (LPS)-induced acute lung injury mouse model, the relative intensity of signals coming from VCAM-1, defined in comparison with the value of 1 as the mean recorded in a non-pathological group is preferably significantly smaller in the group administered with the dental pulp-derived cells at a dose of $1.5 \times 10^4$ cells than a group administered with a medium (bicarbonate Ringer's solution supplemented with human serum albumin and DMSO), and more preferably, is comparable to a non-pathological group.

(4) That when it is administered to cerebral infarction model rats, the number of MPO-positive cells observed at the site of infarction is significantly smaller than a group administered with a medium.

(5) That it exhibits a therapeutic effect when administered to a cerebral infarction patient in whom the concentration of angiopoietin-1 in the blood is not more than 10 ng/mL

(6) That when administered to a cerebral infarction patient, and in particular to a patient with lacunar infarction, it exhibits a prophylactic and therapeutic effect.

(7) That when administered to a cerebral infarction patient, and in particular to a patient with hemorrhagic cerebral infarction, it exhibits a prophylactic and therapeutic effect.

(8) That when administered to a patient with sepsis, and in particular to a patient complicated with an acute lung injury, whose angiopoietin-1 concentration in the blood is not more than 10 ng/mL, it exhibits a therapeutic effect.

For example, as to the positive ratio of surface antigens, when observed as a whole in their expression pattern of surface antigen markers, the cells contained in the dental pulp-derived cell preparation show high positive ratio for at least one of, or all of CD39, CD49a, CD61, CD107a, CD107b, and CD143, as compared with intermediate cells. For example, the cells contained in the dental pulp-derived cell preparation show high positive ratio for CD107b. The positive ratio of the cells contained in the dental pulp-derived cell preparation for these antigens are preferably not less than 10% higher, and more preferably not less than 20% higher than intermediate cells. Further, the cells contained in the dental pulp-derived cell preparation show a lower positive ratio for CD146. The cells contained in the dental pulp-derived cell preparation show a lower positive ratio for CD146 by not less than 20%, or not less than 50%, as compared with the intermediate cells.

Further, for example, as to the expression levels of humoral factors, the cells contained in the dental pulp-derived cell preparation show high expression levels of at least one of, or all of IL-6, IL-23, IL-11, MCP-1, ENA-78, HGF, VEGF, MMP-2, IGFBP-4, cystatin C, TIMP-3, TIMP-2, and TIMP-1 as compared with intermediate cells. In particular, the cells contained in the dental pulp-derived cell preparation show high expression levels of at least one of, or all of IL-6, IL-11, HGF, IGFBP-4, TIMP-3, and TIMP-1, as compared with the intermediate cells. In particular, the cells contained in the dental pulp-derived cell preparation show high expression levels of at least one of, or all of IL-6 and HGF, as compared with intermediate cells.

The intermediate cells and the cells contained in the dental pulp-derived cell preparation have properties of stem cells, i.e., a high ability to undergo cell divisions and ability to differentiate two or more different kinds of cells. These cells can be said as cells having a high colony-forming ability.

When observed as a whole with respect to their expression pattern of surface antigen markers, intermediate cells and the cells contained in the dental pulp-derived cell preparation are, for example, positive for at least one of CD73, CD90, CD105, and CD166, whereas negative for at least one of CD34 and CD45. Further, with respect to their expression pattern of surface antigen markers, the intermediate cells and the cells contained in the dental pulp-derived cell preparation are positive for CD73 and CD90, whereas negative for CD34. This expression pattern is common with mesenchymal stem cells. However, they also have characteristic properties.

In an embodiment of the present invention, when observed as a whole with respect to their expression pattern of surface antigen markers, intermediate cells and the cells contained in the dental pulp-derived cell preparation are positive for at least one of, or all of CD47, CD81, and CD147, whereas negative for at least one of, or all of CD19, CD34, CD206. Further, in an embodiment of the present invention, when observed as a whole with respect to their expression pattern of surface antigen markers, these cells are positive for at least one of, or all of CD47, and CD81, CD14, whereas negative for at least one of, or all of CD19, CD31, CD33, CD34, CD38, CD45, CD206, CD235a, and SSEA-1.

In an embodiment, when observed as a whole with respect to their expression pattern of surface antigen markers, intermediate cells and the cells contained in the dental pulp-derived cell preparation are negative for at least one of CD19, CD26, CD34, CD106, CD117, and CD271, and, for example, negative for all of these. Further, the cells contained in the dental pulp-derived cell preparation are negative for at least one of CD19, CD26, CD34, CD56, CD106, CD117, CD146, and CD271, and, for example, negative for all of these. Though its function is not well understood, CD106 among these is considered to take part in the activation of inflammatory signals because it is expressed in vascular endothelial cells in sites of inflammation. Although the expression pattern of these surface antigens differs from the patterns generally known in those stem cells which have a high colony forming ability, it is still surprising that the intermediate cells and the cells contained in the dental pulp-derived cell preparation have a high colony forming ability.

In an embodiment, when observed as a whole with respect to their expression pattern of surface antigen markers, intermediate cells and the cells contained in the dental pulp-derived cell preparation are positive for at least one of, and, for example, all of CD140b, and HLA-A, B, C.

In an embodiment, when observed as a whole with respect to their expression pattern of surface antigen markers, the cells contained in the dental pulp-derived cell preparation are positive for at least one of, and, for example, all of CD49e and CD95.

In an embodiment, when observed as a whole with respect to their expression pattern of surface antigen markers, intermediate cells and the cells contained in the dental pulp-derived cell preparation are positive for CD10. Though its function is not well known, CD10 may take part in termination of inflammatory reactions by decomposing inflammatory compounds such as enkephalin and substance P and inflammation-related peptides.

Further in an embodiment, when observed as a whole with respect to their expression pattern of surface antigen markers, the cells contained in the dental pulp-derived cell preparation have a higher positive ratio for CD39 then intermediate cells. In addition, those cells are all positive for CD73. Cooperating with CD73, CD39 converts ATP to adenosine and thereby causes an increase in the concentration of extracellular adenosine. Adenosine functions so as to suppresses inflammation by negatively controlling inflammatory reactions. This function is considered to be stronger in a dental pulp-derived cell preparation, which have a higher positive ratio for CD39, than intermediate cells.

Further, in an embodiment, when observed as a whole with respect to their expression pattern of surface antigen markers, intermediate cells and the cells contained in the dental pulp-derived cell preparation are positive for CD46. CD46 is considered to take part in evasion complement-dependent cellular injury by suppressing the activity of a complement (C3).

Further, in an embodiment, when observed as a whole with respect to their expression pattern of surface antigen markers, these cells are positive for CD47. CD47 provides cells with resistance to phagocytosis by macrophages.

Further, in an embodiment, when observed as a whole with respect to their expression pattern of surface antigen markers, these cells are positive for CD55. CD55 has a function to modulate the classical complement pathway or the alternative pathway and is considered to take part in evasion of the complement mediated cellular injury.

Further, in an embodiment, when observed as a whole with respect to their expression pattern of surface antigen markers, these cells are positive for CD58. Though the function of CD58 is not fully understood, it is suggested that CD58 contributes to the termination of inflammatory reactions through induction of regulatory T cells (Treg cells).

Further, in an embodiment, these cells are positive for CD59. CD59 is considered to take part in evasion of complement mediated cellular injury by acting on a complement (C9) and thereby inhibiting formation of membrane-attack complex.

The intermediate cells and the cell contained in the dental pulp-derived cell preparation secrete a variety of humoral factors. In an embodiment, the intermediate cells and the cell contained in the dental pulp-derived cell preparation secrete at least one of, and, e.g., all of MMP-2, IGFBP-4, cystatin C, IL-6, IL-11, MCP-1, IL-8, HGF, VEGF (vascular endothelial cell growth factor), TIMP-1, TIMP-2, and TIMP-3. In addition to these, the intermediate cells and the cell contained in the dental pulp-derived cell preparation secrete at least one of, and, e.g., all of GROα, VCAM-I, and IP-10 (CXCL 10).

In an embodiment, the intermediate cells and the cell contained in the dental pulp-derived cell preparation secrete IL-6. The amount of IL-6 secreted is increased by TNF-α stimulation, and by IFN-γ stimulation. Although IL-6 is also known as an inflammatory cytokine, it is considered to have an antiinflammatory activity, for aggravation of inflammatory reactions in the liver is known in an IL-6-deficient mouse,

The intermediate cells and the cell contained in the dental pulp-derived cell preparation secrete a variety of humoral factors. In an embodiment, the intermediate cells and the cell contained in the dental pulp-derived cell preparation secrete IL-11. The amount of IL-11 secreted is increased by TNF-α stimulation, and by IFN-γ stimulation. IL-11 is considered to have an antiinflammatory activity by suppressing secretion of inflammatory cytokines.

The intermediate cells and the cell contained in the dental pulp-derived cell preparation secrete a variety of humoral factors. In an embodiment, the intermediate cells and the cells contained in the dental pulp-derived cell preparation secrete IP-10 (CXCL 10). The amount of IP-10 secreted is increased by TNF-α stimulation, and by IFN-γ stimulation. IP-10 is considered to have an antiinflammatory activity by suppressing secretion of inflammatory cytokines.

Dental pulp-derived cells have functions such as antiinflammatory ones. The functions are exerted through a cooperation between some or all of the above surface antigens, humoral factors, and the like. For example, angiopoietin-1 expressed in dental pulp-derived cells suppresses expression of adhesion factors such as VCAM-1 on the surfaces of vascular endothelial cells, thereby suppressing accumulation and infiltration of neutrocytes, lymphocytes, and/or monocytes. Accumulation and infiltration of neutrocytes are particularly suppressed.

A pharmaceutical composition of the present invention comprising as an active ingredient the dental pulp-derived cell preparation having functions such as antiinflammatory activity as mentioned above can be used as a therapeutic drug for various disorders. For example, the dental pulp-derived cell preparation can be used for the treatment or prophylaxis of a disorder selected from the group comprising autoimmune diseases, inflammatory diseases, rheumatoid arthritis, Crohn's disease, chronic inflammatory bowel diseases, myocardial infarction, chronic inflammatory pulmonary disease, cerebral infarction (including chronic phase cerebral infarction and acute phase infarction), chronic inflammatory demyelinating multiple neuritis, multiple sclerosis, cirrhosis (including decompensated cirrhosis), sepsis, osteoarthritis, psoriasis, and organ transplant rejection. Chronic inflammatory pulmonary diseases include cystic fibrosis (also abridged as CF), chronic obstructive pulmonary disease (also abridged as COPD), lung dysplasia, idiopathic pulmonary fibrosis (also abridged as IPF), and newborn chronic pulmonary disease. Among these chronic pulmonary diseases, the above pharmaceutical product can be used particularly as a therapeutic or prophylactic drug for chronic pulmonary diseases in newborns or premature babies (e.g., bronchopulmonary dysplasia in newborns and premature babies). Chronic pulmonary diseases in newborns and premature babies are caused by, e.g., the use of artificial ventilators. Thus, the pharmaceutical product can be used as a therapeutic drug for newborns or premature babies who got chronic pulmonary diseases by, e.g., the use of artificial ventilators, or as a prophylactic drug for it. There are known cell preparations containing stems cells, such as mesenchymal stem cells, which can be used for the treatment of autoimmune diseases. However, known cell preparations do not exhibit a remarkable effect in every patient, and their way of use is limited, too. The dental pulp-derived cell preparation of the present invention adds to diversity of cell preparations usable as therapeutic drugs for autoimmune diseases. For example, the dental pulp-derived cell preparation of the present invention can, as a therapeutic preparation, provide a new chance of treatment to patients in whom no therapeutic effect has been obtained with currently known cell preparations, as well as to patients who are suffering from a disease on which no therapeutic effect has ever been proved with currently available cell preparations.

The dental pulp-derived cell preparation is administered to patients with the above disorders by such a route of administration as intravenous drip infusion, topical injection, or the like.

The dental pulp-derived cell preparation is thawed before use. Before use, the dental pulp-derived cell preparation is taken out of a liquid nitrogen preservation container and thawed. A process of thawing is carried out by, e.g., warming it in a 36.5-37.5°C water bath. The dental pulp-derived cell preparation thawed can be administered to a human as a pharmaceutical product by a route of intravenous drip infusion, topical injection, and the like. In the case where intravenous drip infusion is made, the dental pulp-derived cell preparation is transferred from the vial to an infusion bag, from which it is administered to a patient by intravenous drip infusion. In the case where topical injection is made, the dental pulp-derived cell preparation is transferred from the vial to an infusion bag, from which it is administered to a patient by topical injection.

Thawing before use of the dental pulp-derived cell preparation is expected to be carried out in medical facilities. The dental pulp-derived cell preparation is supplied to medical facilities in a manner described below, for example but without limitation. The dental pulp-derived cell preparation, cryopreserved in a depository of the maker or a distributor, is shipped out in the frozen condition in accordance with an order from medical facilities and transported to them. The frozen state preparation brought in the medical facilities is thawed immediately before its administration, and then administered to a patient by intravenous injection or the like. As an apparatus for handling a dental pulp-derived cell preparation keeping in its frozen state, a mobile low-temperature worktable (WO2017/099105) can be used suitably.

EXAMPLES

While the present invention is explained in further detail with reference to examples, it is not intended that the present invention be limited to the examples.

[Example 1: Preparation of mediums and reagents]

DMEM (10% FBS) medium: DMEM Low Glucose (glucose concentration: 5.56 mM, GE healthcare) supplemented with FBS (GE healthcare) to the final concentration of 10%

DMEM (20% FBS) medium: DMEM Low Glucose (glucose concentration: 5.56 mM, GE healthcare) supplemented with FBS (GE healthcare) to the final concentration of 20%

Streptomycin aqueous solution: a solution prepared by dissolving streptomycin sulfate in purified water to the final concentration of 0.01 g/mL

Protease solution: a solution of Liberase (Roche) prepared at the final concentration of 2.5 mg/mL by addition of DMEM Low Glucose. It is a solution containing collagenase I, collagenase II and thermolysin as proteases.

DMEM (20% FBS) streptomycin medium: a 100:1 mixture by volume of DMEM (20% FBS) medium and the streptomycin solution

Trypsin-EDTA solution: 0.25% trypsin-EDTA 溶液 (Thermo Fisher Scientific)

One % chlorohexidine gluconate solution: a solution prepared by 5 times dilution of 5% (w/v) Fermajin solution (Sioe Pharmaceutical) with water for injection

Bicarbonate Ringer's solution: Bicarbon Intravenous Solution (AY Pharma) of the composition shown in Table 7 and Table 8 below

Table 7. Composition of bicarbonate Ringer's solution

| Ingredients | Concentration (g/L) | Concentration (mM) |
|---|---|---|
| Sodium chloride | 6.14 | 105 |
| Potassium chloride | 0.3 | 4.02 |
| Calcium chloride dihydrate | 0.22 | 1.5 |
| Magnesium chloride hexahydrate | 0.102 | 0.501 |
| Sodium bicarbonate | 2.1 | 25 |
| Sodium citrate dihydrate | 0.49 | 1.67 |
| pH 6.8-7.8, Osmotic pressure ratio to physiological saline: 0.9-1.0 | | |

Table 8. Electrolyte concentration in bicarbonate Ringer solution

| Electrolytes | Concentration (mEq/L) |
|---|---|
| $Na^+$ | 135 |
| $K^+$ | 4 |
| $Ca^{2+}$ | 3 |
| $Mg^{2+}$ | 1 |

(continued)

| Electrolytes | Concentration (mEq/L) |
|---|---|
| Cl$^-$ | 113 |
| HCO$_3^-$ | 25 |
| Citrate$^{3-}$ | 5 |

Human serum albumin solution: donated albumin 25-Nichiyaku (Nihon Pharmaceutical) having the composition shown in Table 8 below

Table 9. Composition of human serum albumin solution

| Ingredients | Concentration (mg/mL) | Concentration (mM) |
|---|---|---|
| Human serum albumin | 250 | - |
| Acetyltryptophan sodium | 5.472 | 20.3 |
| Sodium caprylate | 3.395 | 20.4 |
| (Note) Sodium ion concentration in the solution: 40.7 mEq/L | | |

Wash solution: a solution prepared by adding 50 mL of 25% (w/v) human serum albumin solution (Nihon Pharmaceutical) to 1000 mL of the bicarbonate Ringer's solution (Bicarbon Intravenous Solution, AY Pharma) to the final concentration of human serum albumin of 1.19% (w/v). Table 10 and Table 11 show the composition of the wash solution.

Table 10. Composition of wash solution

| Ingredients | Concentration (g/L) | Concentration (mM) |
|---|---|---|
| Sodium chloride | 5.85 | 100 |
| Potassium chloride | 0.286 | 3.83 |
| Calcium chloride dihydrate | 0.21 | 1.43 |
| Magnesium chloride hexahydrate | 0.097 | 0.477 |
| Sodium bicarbonate | 2.00 | 23.8 |
| Sodium citrate dihydrate | 0.467 | 1.59 |
| Human serum albumin | 11.9 | - |
| Acetyltryptophan sodium | 0.261 | 0.967 |
| Sodium caprylate | 0.162 | 0.971 |

Table 11. Concentration of electrolytes (except albumin) in wash solution

| Ingredients | Concentration (mEq/L) |
|---|---|
| Na$^+$ | 130.5 |
| K$^+$ | 3.83 |
| Ca$^{2+}$ | 2.86 |
| Mg$^{2+}$ | 0.952 |
| Cl$^-$ | 107.6 |
| HCO$_3^-$ | 23.8 |
| Citrate$^{3-}$ | 4.76 |

(continued)

| Ingredients | Concentration (mEq/L) |
|---|---|
| Acetyltryptophan ion | 0.967 |
| Caprylate ion | 0.971 |

Cell protection solution: a solution prepared by mixing bicarbonate Ringer's solution (Bicarbon Intravenous solution, AY Pharma), the 25% human serum albumin and dimethylsulfoxide (DMSO, Mylan) at a volume ratio of 11:9:5. Table 12 shows the composition of the cell protection solution.

Table 12. Composition of cell protection solution

| Ingredients | mM |
|---|---|
| Sodium chloride | 46.2 |
| Potassium chloride | 1.77 |
| Calcium chloride dihydrate | 0.66 |
| Magnesium chloride hexahydrate | 0.22 |
| Sodium bicarbonate | 11 |
| Sodium citrate dihydrate | 0.73 |
| Human serum albumin | (90) |
| Acetyltryptophan sodium | 7.31 |
| Sodium caprylate | 7.34 |
| DMSO | (20) |
| (Note) The unit of concentration of human serum albumin is g/L, and that of DMSO is %(v/v). | |

[Example 2: Obtainment of dental pulp from extracted human tooth]

A human extracted tooth (a third molar), which was obtained on an informed consent, was rinsed with a bicarbonate Ringer's solution (Bicarbon Intravenous solution) and disinfected on its surfaces with 1% chlorhexidine gluconate solution. The extracted tooth then was fractured and the dental pulp was separated from other tissues.

[Example 3: Enzyme treatment of the dental pulp]

To the dental pulp obtained in Example 2 was added the protease solution which had been diluted approximately 10 times with DMEM Low Glucose, and was allowed to stand in a water bath adjusted to 37°C until it was visually confirmed that the tissue was fully disintegrated.
All the enzyme-treated cells then were transferred into a centrifuge tube, to which the DMEM (20% FBS) streptomycin medium was added to terminate the enzyme reaction, and tissue debris and cells were precipitated by centrifugation. The supernatant was removed, and to the precipitate thus obtained was added the DMEM (20% FBS) streptomycin medium so as to suspend the tissue debris and cells therein, and they were reprecipitated by centrifugation. To the precipitate was added DMEM (20% FBS) streptomycin medium, and by suspending the cells by mild pipetting, a dental pulp suspension containing debris and dental pulp-derived cells was obtained.

[Example 4: Culture of dental pulp suspension]

The dental pulp suspension prepared in Example 3 was added to a cell culture plate, and culture was started at 37°C in the presence of 5% $CO_2$. The culture was continued until colonies formed on the plate was visually confirmed while exchanging DMEM (20% FBS) streptomycin medium at intervals of 2-4 days.

[Example 5: Culture of dental pulp-derived cells]

The medium was removed from the cell culture plate on which formation of colonies was visually confirmed, and after addition of the trypsin-EDTA solution so as to cover the cells' surfaces with the solution, the plate was allowed to stand for 5-10 minutes at 37°C to detach the cells. To the cell culture plate then was added the DMEM (10% FBS) medium to terminate the reaction and suspend the cells. This cell suspension was recovered into a centrifuge tube. The recovered cells were centrifuged (300xg, 5 minutes) to spin them down, and the supernatant was removed. By adding the DMEM (10% FBS) medium to the centrifuge tube to suspend the cells, a cell suspension was prepared.

After counting the number of the viable cells contained in the cell suspension, the cells were seeded onto a cell culture plate so that cell density might come to be 3000-20000 cells/cm$^2$, and culture was started at 37°C in the presence of 5% $CO_2$. Culture was continued while exchanging DMEM (10% FBS) medium at intervals of 2-4 days until the cells became confluent on the plate.

After removing the medium from a cell culture plate on which the cells had come to be confluent, the trypsin-EDTA solution was added to the plate so that the surfaces of the cells were covered with the solution. The plate was allowed to stand for 5-10 minutes at 37°C to detach the cells. The DMEM (10% FBS) solution, in the same volume as that of the trypsin-EDTA solution, was then added to the plate to terminate the reaction and suspend the cells. This cell suspension was recovered into a centrifuge tube. The recovered cells were spun down (300xg, 5 minutes), and after removal of the supernatant, DMEM (10% FBS) medium was added to suspend the cells.

The above procedure of recovery of cells and their cultivation was repeated to let the cells grow until the number of viable cells comes to not less than $1 \times 10^8$. The number of cell divisions during this period (i.e., the number of cell divisions in the in vitro environment) was 16-17 times, and the cells' mean doubling time was not more than 2 days (48 hours) throughout the culture period.

[Example 6: Recovery of dental pulp-derived cells (intermediate cells)]

The medium was removed from the cell culture plate on which the cells became confluent in the final cycle of culture in Example 5, and the trypsin-EDTA solution was added so as to cover the cells' surfaces with the solution. The plate was allowed to stand for 5-10 minutes at 37°C to detach the cells. The DMEM (10% FBS) solution, in the same volume as that of the trypsin-EDTA solution, was then added to the plate to terminate the reaction and suspend the cells. This cell suspension was recovered into a container, and the cells were spun down by centrifugation, and the supernatant was removed. To wash the cells, the wash solution was added in an amount of 500 mL to 1000 mL to suspend the cells, and cells then were sedimented by re-centrifugation, and the supernatant was removed. This procedure of washing was repeated, and finally, the cells were recovered as a sediment following centrifugation. The cells thus obtained were designated the intermediate cells.

[Example 7: Freezing of dental pulp-derived cells (intermediate cells)]

To the sediment of cells prepared in Example 6 were added the wash solution and the cell protection solution so as to suspend the cells at a density of $11 \times 10^6$ cells/mL ($\pm 5\%$). This cell suspension thus prepared was dispensed into vials for cryopreservation (made of Cyclic olefin copolymer, Sterile Closed Vial, Aseptic Technologies), and the vials were sealed. This cell suspension was designated intermediate cell suspension. The ingredients contained in the solution part of the intermediate cell suspension (cryopreservation solution for intermediate cells) are shown in Table 13. The intermediate cell suspension, after their freezing in a program freezer, was transferred into a liquid nitrogen preservation container for preservation. This frozen intermediate cell suspension was designated "intermediate cell frozen".

Table 13. Composition of cryopreservation solution for intermediate cells

| Ingredients | mM (Range) | mM (Suitable example) |
|---|---|---|
| Sodium chloride | 65.8-80.4 | 73.1 |
| Potassium chloride | 2.52-3.08 | 2.80 |
| Calcium chloride dihydrate | 0.95-1.16 | 1.05 |
| Magnesium chloride hexahydrate | 0.315-0.385 | 0.35 |
| Sodium bicarbonate | 15.67-19.15 | 17.41 |
| Sodium citrate dihydrate | 1.04-1.28 | 1.16 |
| Human serum albumin | (46-56) | (51) |
| Acetyltryptophan sodium | 3.75-4.55 | 4.14 |
| Sodium caprylate | 3.74-4.58 | 4.16 |

(continued)

| Ingredients | mM (Range) | mM (Suitable example) |
|---|---|---|
| DMSO | (9-11) | (10) |
| (Note) The unit of concentration of human serum albumin is g/L, and that of DMSO is %(v/v). | | |

[Example 8: Thawing of dental pulp-derived cells (intermediate cells)]

Vials containing the dental pulp-derived cells (intermediate cells) prepared in Example 7 were taken out of the liquid nitrogen preservation container, and warmed in a 36.5-37.5°C water bath to thaw the cells. The cell suspension thus thawed was transferred into a centrifuge tube, and suspended in 30 mL DMEM (10% FBS) medium added. The cells were sedimented by centrifugation (300xg, 5 minutes), and the supernatant was removed. Twenty mL DMEM (10% FBS) medium then were added to suspend the intermediate cells.

[Example 9: Production culture of dental pulp-derived cells (preculture)]

After counting the number of viable cells contained in the intermediate cells contained in the cell suspension prepared in Example 8, the cells were seeded onto a cell culture plate to a density of not more than 20000 cells/cm$^2$, and culture was started at 37°C in the presence of 5% $CO_2$. Culture was continued while exchanging DMEM (10% FBS) medium at intervals of 2-4 days until the cells came to be confluent on the cell culture plate. Besides, the ratio of viable cells in the intermediate cells thawed, as calculated on the number of viable cells in the intermediate cells thawed (number of viable cells / number of total cells × 100%), was generally 85-95%.

The medium on the cell culture plate where the cells came to be confluent was removed, and the trypsin-EDTA solution was added so as to cover the cells' surfaces with the solution, and the plate was allowed to stand for 5-60 minutes at 37°C to detach the cells. The same volume of the DMEM (10% FBS) solution then was added to the cell culture plate to terminate the reaction and suspend the cells. This cell suspension was recovered in a container, the cells were sedimented by centrifugation, and the supernatant was removed. The DMEM (10% FBS) medium was added to suspend the cells, and cells then were sedimented by re-centrifugation, and the supernatant was removed. The cells were resuspended by addition of the DMEM (10% FBS) medium.

After measuring the number of viable cells contained in the cell suspension, the cells were seeded onto a cell culture plate to a density of not more than 20000 cells/cm$^2$, and culture was started at 37°C in the presence of 5% $CO_2$. Culture was continued while exchanging DMEM (10% FBS) medium at intervals of 2-4 days until the cells came to be confluent on the cell culture plate.

The above procedure of recovery of cells and their cultivation was repeated to let the cells grow until the number of viable cells came to be not less than $1 \times 10^9$. The number of cell divisions during this period of culture of intermediate cells on a cell culture plate was 5-6 times, and the cells' mean doubling time was not more than 2 days (48 hours) throughout the culture period.

The medium on the cell culture plate where the cells came to be confluent was removed, and the trypsin-EDTA solution was added. The plate was allowed to stand for 5-60 minutes at 37°C to detach the cells. The DMEM (10% FBS) solution then was added to the cell culture plate to terminate the reaction and to suspend the cells. The cell suspension was recovered in a container. The recovered cells were sedimented by centrifugation, and the supernatant was removed. The DMEM (10% FBS) medium, 500 mL-600 mL, was added to suspend the cells, and the cells then were sedimented by re-centrifugation, and the supernatant was removed. DMEM (10% FBS) medium, 500 mL-600 mL, was added to resuspend the cells.

[Example 10: Production culture of dental pulp-derived cells (provision of bioreactor)]

Fifty grams (±1.0 g) of microcarriers were weighed using an electronic analytical scale, and put into a reagent bottle, and after addition of 2000 mL PBS, sterilized in an autoclave. Besides, the microcarriers employed were 130-180 μm in particle size (when hydrated) and made of a gelatin for cell culture, which had given a thermostability so as to withstand autoclave sterilization.

A 50-L bag for bioreactor, a vent filter, and a sensor loop were set in a bioreactor, as well as a temperature sensor, a pH meter, and a dissolved oxygen meter. Ten L- DMEM (10% FBS) medium and 50 g (dry weight) of microcarriers were added to (the bag in) the bioreactor, and warmed up to 37°C while stirring. Stirring was performed with an impeller installed in the device.

[Example 11: Production culture of dental pulp-derived cells (adhesion step of cells to microcarriers)]

After measuring the number of viable cells contained in the cell's suspension prepared in Example 9, the cells were put in the bioreactor to a density of 1000-5000 cells/cm$^3$. After several minutes of stirring in the bioreactor, stirring was stopped, and the mixture was left to stand for at least one hour. This cycle of stirring and leaving to stand the mixture was repeated to let the cells adhere to the microcarriers.

[Example 12: Production culture of dental pulp-derived cells (cell culture step)]

Following the adhesion step, the contents in the bioreactor was stirred, and culture was started. During this culture period, the bioreactor was regularly checked in its inside, and when microcarriers were found having sedimented on the bottom of the bioreactor, the rate of stirring was increased so that the microcarriers would be kept suspended in the medium.

The production culture was continued until the number of viable cells came to be not less than $5 \times 10^9$ cells as counted in accordance with Example 13. The number of cell divisions during the period of culture on microcarriers was 2-3 times, and mean doubling time of the cells were about 3-6 days. The cells undergone the production culture were those which had undergone at least 23 times of cell divisions, and 23-27 times of cell divisions, under an in vitro environment.

[Example 13: Production culture of dental pulp-derived cells (counting of number of cells)]

Thirty to 40 mL of the suspension containing microcarriers were taken out of the reactor and transferred into centrifuge tubes and left to stand for at least 5 minutes to let the microcarriers sediment, and the supernatant was removed. PBS, in an amount of 25 mL, was added to suspend the cells, and this suspension was left to stand for at least 5 minutes, and the supernatant was removed. This procedure was repeated once again. After removal of the supernatant, the trypsin-EDTA solution was added to the microcarriers. After stirring for 5-10 minutes in a water bath at 37°C, DMEM (10% FBS) medium, in the same volume as the trypsin-EDTA solution, was added to terminate the reaction and suspend the cells. This cell suspension was centrifuged (300xg, 5 minutes) to sediment the cells, and the supernatant was removed. After addition of 1-5 mL of the DMEM (10% FBS) medium to suspend the cells, the number of viable cells contained in the cell suspension was counted.

[Example 14: Production culture of dental pulp-derived cells (measurement of glucose concentration and lactic acid concentration)]

From the reactor, 5-40 mL of the cell suspension during culture were taken and transferred into centrifuge tubes, left to stand for at least 5 minutes to let the microcarriers sediment, and part of the supernatant was transferred into a fresh 1.5-mL tube. Fifty µL of the supernatant were taken in a micro syringe and concentration of glucose and lactic acid in the supernatant was measured on a biosensor (BF-7D, Oji measuring equipment). When the glucose concentration was found lowered, either glucose was added so that its concentration would not come down below 0.1 mM or the medium was replaced entirely or partly. When the lactic acid concentration was found elevated, the medium was replaced entirely or partly so that the lactic acid concentration would not go up over 20 mM.

[Example 15: Recovery of dental pulp-derived cells obtained in production culture]

After confirming that the cells have proliferated to a predetermined number, stirring was stopped in the bioreactor and in the sensor loop, and these were left to stand for 10 minutes to let microcarriers sediment. The medium was removed as much as possible, and the cells were suspended in the remaining medium. This cell suspension was recovered in a 10-L bag (Thermo Fisher Scientific) and left undisturbed for at least 5 minutes to let the microcarriers sediment, and the supernatant was removed.

The microcarriers were suspended by addition of DMEM Low Glucose, and left undisturbed for at least 5 minutes to let the microcarriers sediment, and the supernatant was removed. This procedure was repeated several times. The supernatant then was removed, To the microcarriers left behind after the removal of the supernatant was added the trypsin-EDTA solution. Following the addition of the trypsin-EDTA solution, the cells were detached from the microcarriers and the microcarriers were decomposed, by stirring the mixture for 30-60 minutes in a 37°C water bath.

In order to remove fragments of the microcarriers, cell aggregates, and the like remaining in the cell suspension, recovered filtrate was passed through a filter having a pore size 20-35µm and the cells were recovered in the filtrate passing through the filter. The recovered filtrate was centrifuged to sediment the cells, and the supernatant was removed. In order to wash the cells, the wash solution prepared in Example 1 was added to suspend the cells, and after re-centrifugation to sediment the cells, the supernatant was removed. This washing cycle was repeated, and the cells were finally recovered as a sediment left after centrifugation.

[Example 16: Preparation of dental pulp-derived cells and cryopreservation]

The wash solution and cell protection solution were added to the sediment of cells that was recovered in Example 15 so as to suspend the cells at a density of $25\text{-}31\times10^6$/mL. The cell suspension thus obtained was dispensed into vials for cryopreservation (made of Cyclic olefin copolymer, Sterile Closed Vial, Aseptic Technologies) and sealed. This cell suspension was designated dental pulp derived cell preparation unfrozen. The ingredients contained in the solution part of the dental pulp derived cell preparation unfrozen (cryopreservation solution for cells for a preparation) are shown in Table 14.

Table 14. Composition of cryopreservation solution for cells for a preparation

| Ingredients | mM (Range) | mM (Suitable example) |
| --- | --- | --- |
| Sodium chloride | 65.8-80.4 | 70.8 |
| Potassium chloride | 2.52-3.08 | 2.71 |
| Calcium chloride dihydrate | 0.95-1.16 | 1.01 |
| Magnesium chloride hexahydrate | 0.315-0.385 | 0.34 |
| Sodium bicarbonate | 15.67-19.15 | 16.9 |
| Sodium citrate dihydrate | 1.04-1.28 | 1.13 |
| Human serum albumin | (46-56) | (53.6) |
| Acetyltryptophan sodium | 3.75-4.55 | 4.36 |
| Sodium caprylate | 3.74-4.58 | 4.37 |
| DMSO | (9-11) | (10.7) |
| (Note) The unit of concentration of human serum albumin is g/L, and that of DMSO is %(v/v). | | |

The dental pulp derived cell preparation unfrozen in the vials were frozen by a conventional method using a program freezer, and transferred into a liquid nitrogen preservation container and preserved in it. This frozen cell suspension was designated a preparation containing dental pulp-derived cells as the active ingredient (dental pulp-derived cell preparation).

[Example 17: Properties of dental pulp-derived cells(cell's morphology and the like)]

The intermediate cell frozen prepared in Example 7 and the dental pulp-derived cell preparation prepared in Example 16 were thawed, and after addition of 10 mL of the DMEM (10% FBS) and light shaking, the cells were sedimented by centrifugation (1500 rpm, 5 minutes). The supernatant was removed and 10 mL of the DMEM (10% FBS) medium was added to suspend the cells, and the cells were sedimented by re-centrifugation (1500 rpm, 5 minutes). The cells then were suspended in the DMEM (10% FBS) medium, and seeded onto a cell culture plate at a density of 5000-10000 cells/cm$^2$, and cultured until the cells came to be 90-100% confluent. The cells were observed under a phase-contrast microscope for their morphology on the cell culture plate. All the cells obtained by culturing the cells contained the intermediate cell frozen and the dental pulp-derived cell preparation were generally spindle-shaped cells, and no other differently shaped cells were contained. Besides, the ratio of viable cells in the cells prepared by thawing the intermediate cell frozen and the dental pulp-derived cell preparation (number of viable cells / number of total cells $\times$ 100%), as calculated from the number of viable cells, was generally 85-95%.

[Example 18: Properties of dental pulp-derived cell preparation (particle size)]

The intermediate cell frozen prepared in Example 7 and the dental pulp-derived cell preparation prepared in Example 16 were thawed, and respectively diluted with D-PBS (-) solution to prepare a diluted cell suspension containing approximately $5\times10^6$cells/mL. The particle size of the cells was measured by an image analysis method utilizing an automated cell viability analyzer (Vi-CellXR, Beckman Coulter). An image analysis method utilizing an automated cell viability analyzer is summarized in general below. Trypan blue is added to the diluted cell suspension to stain the cells, and this diluted cell suspension is sent through a thin, long flow path, and multiple magnified images of the flow path are taken with a microscope. Since dead cells, whose cell membrane is permeable to trypan blue, are stained with it, colored particles and transparent particles observed are counted as dead and live cells, respectively in each of the images. The particle size of the cells is calculated using the diameter of a particle measured on the image and the magnification factor of the microscope. A mean value is determined from the particle sizes in all of the images taken.

(Result)

The mean value of the particle size of the cells (cells' diameter) was 17.36 $\mu$m $\pm$ 1.40 $\mu$m (mean value $\pm$ SD, n=3) with the cells prepared by thawing frozen intermediate cells, and 16.98 $\pm$ 1.23 $\mu$m (mean value $\pm$ SD, n=3) with the cells prepared by thawing dental pulp-derived cell preparation frozen (Fig. 1).

[Example 19: Properties of dental pulp-derived cells (ability to differentiate into osteocytes)]

The ability to differentiate into osteocytes was examined as described below with reference to Pittenger MF., et al., Science. 284, 143-7 (1999),Colter DC., et al., Proc Natl Acad Sci USA. 98, 7841-5(2001).
The intermediate cell frozen prepared in Example 7 and the dental pulp-derived cell preparation prepared in Example 16 were thawed, and the cells were respectively sedimented by centrifugation (1500 rpm, 5 minutes). The cells then were suspended in DMEM (10% FBS) medium, and seeded on a cell culture plate at a density of 5000-15000 cells/cm$^2$, and cultured until the cells came to be 90-100% confluent. The cells were washed with PBS, and after addition of the trypsin-EDTA, the cells were detached by leaving them undisturbed for 5-10 minutes at 37°C. The DMEM (10% FBS) medium was added to suspend the cells, and the number of viable cells was counted. The cells were seeded on a 24-well cell culture plate coated with collagen I at the density in the DMEM (10% FBS) medium (15000-25000 cells/cm$^2$) and cultured for 3 days. The cells were divided into 2 groups, in one of which the medium was replaced with a medium for induction of osteocyte differentiation, which was a medium prepared by supplementing a basic medium for inducing osteocyte differentiation (Lonza) with a set of osteocyte differentiation supplements and factors (Lonza) containing dexamethasone, L-glutamine, an ascorbate, penicillin/streptomycin, mesenchymal cell growth supplement (MCGS), $\beta$-glycerophosphate, and differentiation culture was carried out for 2-3 weeks while exchanging the medium once in 3-4 days. This group was designated the osteocyte differentiation-induction group. In the other of the groups, the medium was replaced with fresh DMEM (10% FBS) medium, and culture was continued for 2-3 weeks exchanging the medium once in 3-4 days. This group was designated the control group. After the respective culture of cells in the osteocyte differentiation-induction group and the control group, the cells were washed once with PBS, and following addition of 0.4 mL of 10% formic acid to each well, the cells were left undisturbed for 1 hour at room temperature so that the accumulated calcium in the cells would be released from the cells. The concentration of calcium released was determined using E-Test Wako (Wako Pure Chemical).
Fig. 2 shows the result of the measurements. In comparison with the control group, the cells contained in the osteocyte differentiation-induction groups, either in the intermediate cells frozen or the dental pulp-derived cell preparation, exhibited an increase in the concentration of calcium in the cells. This result indicates that the cells contained either in the intermediate cells frozen or the dental pulp-derived cell preparation differentiated into osteocytes, indicating that the dental pulp-derived cells of the present invention have an ability to differentiate osteocytes.

[Example 20: Properties of dental pulp-derived cells (ability to differentiate into chondrocytes)]

The ability to differentiate into chondrocytes was examined as described below with reference to the Kiani C., et al. Cell Res. 12 19-32 (2002), Aung A., et al. Arthritis Rheum. 63 148-58 (2011).
The intermediate cell frozen prepared in Example 7 and the dental pulp-derived cell preparation prepared in Example 16 were thawed, and centrifuged (1500 rpm, 5 minutes) to sediment the cells. The cells then were suspended in DMEM (10% FBS) medium, seeded on a cell culture plate at the density of 5000-10000 cells/cm$^2$, and cultured until the cells came to be 90-100% confluent. The cells were washed with PBS, and after addition of the trypsin-EDTA, left undisturbed for 5-10 minutes so that they were detached. The DMEM (10% FBS) was added to suspend the cells, and the number of viable cells was counted. The cells were divided into 2 groups, and in one of which the cells were suspended at a density of $1\times10^6$ cells/mL in Stem MACS (registered trademark) ChondroDiff Medium (Miltenyi Biotec), which was a medium for induction of chondrocyte differentiation, and seeded 1 mL each in low adsorption containers (Stemfull (registered trademark), Sumitomo Bakelite) to let them form spheroids. The differentiation culture was continued for 2-3 weeks exchanging the medium once in 3-4 days. This was designated the chondrocyte differentiation-induction group. In the other of the groups, the cells were subjected to passage culture in the DMEM (10% FBS) medium, and this was designated the control group.
The cells were recovered after the above culture, treated with proteinase K for 30 minutes in a 55°C water bath to lyse them. A total RNA extract solution was prepared from the lysed cells using RNeasy (registered trademark) Plus Mini Kit (QIAGEN), and the concentration of RNA in the total RNA extract solution was measured using an absorptiometer (Denovix). After the measurement of RNA concentration, cDNAs were synthesized using QuantiTect (registered trademark) Reverse Transcription kit. Then, a PCR solution was prepared, and using the cDNAs in the respective groups as templates, real-time RT-PCR was performed under the conditions of (50°C/2 minutes)x1 cycle, (95°C/10 minutes)x1 cycle, (95°C/15 seconds, and 60°C/1 minute)x40 cycles, to multiply the aggrecan gene and the β-actin gene. As PCR primers, aggrecan probe (Applied Biosystems/Assay ID:Hs00153936_m1) and β-actin prove (Applied Biosystems/4310881E) were used, respectively.

Fig. 3 shows the result of measurement. It was found that compared with the control group, the Ct value (Threshold cycle) for aggrecans was low with the cells contained in the chondrocyte differentiation-induction groups, either in the intermediate cells frozen or the dental pulp-derived cell preparation. This result indicates that an increase was brough about in the expression levels of aggrecans, the main molecules for construction of extracellular matrix of cartilage, in the chondrocyte differentiation-induction groups, which means that the cells contained either in the intermediate cells frozen or the dental pulp-derived cell preparation have an ability to differentiate into chondrocytes.

[Example 21: Properties of dental pulp-derived cells (detection of surface antigens-1)]

The dental pulp-derived cells were observed to be generally spindle-shaped like human mesenchymal stem cells. Thus, presence/absence of expression of CD73, CD90, CD105, and CD166, which are surface antigen markers known to be positive in human mesenchymal stem cells, as well as CD34 and CD45, which are surface antigen markers known to be negative in mesenchymal stem cells, was examined using a flow cytometer.

Phosphate buffered saline pH 7.4, containing BSA, powder (Sigma) was dissolved in purified water, and passed through a 0.45 μm filter to prepare PBS-B (phosphate buffered saline 0.01 M (pH 7.4) containing 0.138 M sodium chloride, 0.0027 M potassium chloride, 1% (w/v) bovine serum albumin). IgG from Human (SIGMA) was dissolved in PBS (Life technologies) and passed through a 0.45 μm filter to prepare a 20 mg/mL IgG solution. To 18 mL of PBS-B was added 2 mL of the 20 mg/mL IgG solution to prepare a blocking solution.

And antibodies employed were FITC-labeled anti-human CD34 antibody (anti-CD34-FITC, BD) as anti-CD34 antibody, FITC-labeled anti-human CD45 (anti-CD45-FITC, Beckman Coulter) as anti-CD45 antibody, FITC-labeled anti-human CD73 antibody (anti-CD73-FITC, BD) as anti-CD73 antibody, FITC-labeled anti-human CD90 antibody (anti-CD90-FITC, BD) as anti-CD90 antibody, PE-labeled anti-human CD105 antibody (anti-CD105-R-PE, BD) as anti-CD105 antibody, PE-labeled anti-human CD166 antibody (anti-CD166-PE, Ancell) as CD166 antibody, and FITC-labeled anti-mouse IgG1 isotype control (anti-IgG1-FITC, Beckman Coulter) and PE-labeled anti-mouse IgG1 isotype control (IgG1-PE, Beckman Coulter) as a control antibodies.

(Method for staining cells)

The intermediate cell frozen prepared in Example 7 and the dental pulp-derived cell preparation prepared in Example 16 were thawed, and centrifuged (1500 rpm, 5 minutes) to sediment the cells. The supernatant was removed and 10 mL of DMEM (10% FBS) medium was added to suspend the cells and the number of viable cells was counted. Cells ($1\times10^7$ cells) were taken in a 50-mL centrifuge tube, and sedimented by centrifugation (1500 rpm, 5 minutes), and the supernatant was removed. After addition of PBS-B to adjust the total volume to 10 mL and suspend the cells, the cells were sedimented by re-centrifugation (1500 rpm, 5 minutes), and the supernatant was removed. The cells then were suspended in 1 mL of the blocking solution, and left undisturbed on ice for 1 hour. The antibody solutions were respectively added to 9 reaction tubes of 5-mL capacity (Nos. (1)-(9)) as shown in Table 15. To each tube then was added 100 μL of a cell suspension in the blocking solution, and following light shaking, left undisturbed on ice for 20 minutes to let the antibodies contained in the antibody solutions bind to the surface antigen markers expressed on cell surfaces. PBS-B then was added to the tubes 3 mL each, mixed, and centrifuged (1500 rpm, 5 minutes) to spun down the cells, and the antibodies unbound to the cells were removed by removing the supernatant. This procedure for removing antibodies was repeated 3 times. To the tubes were added PBS-B, 400 μL each, to suspend the cells.

Table 15. Type and amount of antibody solutions added to reaction tubes

| Tube numbers | Antibody solutions | Volume added (μL) |
| --- | --- | --- |
| (1) | PBS-B | - |
| (2) | IgG1-FITC | 20 |
| (3) | Anti-CD34-FITC | 20 |

(continued)

| Tube numbers | Antibody solutions | Volume added ($\mu$L) |
|---|---|---|
| (4) | Anti-CD45-FITC | 20 |
| (5) | Anti-CD73-FITC | 5 |
| (6) | Anti-CD90-FITC | 2 |
| (7) | IgG1-PE | 20 |
| (8) | Anti-CD 105-FITC | 2 |
| (9) | Anti-CD 166-FITC | 20 |

(Measurement and analysis)

The cells in each of the tubes Nos. (1)-(9) were measured for the amount of the fluorochromes FITC and PE using BD FACSVerse (registered trademark) (BD), and in comparison with a negative control, the amount of the fluorochromes was determined which bound to the cell surfaces via antibodies specifically bound to the surface antigens. Besides, tube (2) corresponds to the negative control for tubes (3)-(6), and tubes (7) corresponds to the negative control for tubes (8) and (9). Tube (1) corresponds to non-stained cells.

The results are shown in Fig. 4. It was found that in tubes Nos. (5), (6), (8), and (9) (corresponding to CD73, CD90, CD105, and CD166-stained cells, respectively), not less than 85% of the cells were found positive for these surface antigens, both in the cells contained in the intermediate cell frozen and the cells contained in the dental pulp-derived cell preparation. The cells in tube Nos. (3) and (4) (corresponding to CD34 and CD45-stained cells) were found negative for these surface antigens in most of the cells, both in the cells contained in the intermediate cell frozen and the cells contained in the dental pulp-derived cell preparation. These results indicate that dental pulp-derived cells are positive for CD73, CD90, CD105, and CD166, and negative for CD34 and CD45.

[Example 22: Properties of dental pulp-derived cells (detection of surface antigens-2)]

The intermediate cells frozen prepared in Example 7 and the dental pulp-derived cell preparation prepared in Example 16 were thawed, and the entire volume of respective cell suspensions was added to DMEM medium. Following centrifugation (300xg, 5 minutes, room temperature), the supernatant was removed, and DMEM medium was added to suspend the cells. The cells then were stained with antibodies to surface antigens using BD Lyoplate (Human Cell Screening Marker Screening Panel, BD). This antibody staining was performed following the protocol provided in Human cell screening panel (BD). The method for detection of surface antigens using Human cell screening panel is outlined here: The cell suspensions were dispensed into the wells of a 96-well plate. To the wells are added antibodies against various surface antigens as primary antibodies, and left undisturbed for 30 minutes to let the antibodies bind to the surface antigens. Following centrifugation to spun down the cells, the supernatant is removed. After washing the cells, antibodies to the primary antibodies are added to the wells as secondary antibodies, and left undisturbed to let the antibodies bind to the surface antigens. The secondary antibodies were those fluorescence-labelled with Alexa Fluor 647. After centrifugation to spun down the cells, the supernatant is removed. The cells are resuspended, and the cell suspensions are subjected to fluorescence detection by flow cytometry to detect the cells to which the secondary antibodies are bound as positive cells, and the ratio of positive cells for each surface antigen is determined. Nine lots of intermediate cells and 7 lots of the cells contained in the dental pulp-derived cell preparation were examined, and the positive ratio for each antigen was determined.

(Results)

Fig. 5 and Fig. 6 show those surface antigens which were found positive in the intermediate cells and the cells contained in the dental pulp-derived cell preparation, respectively.

The intermediate cells exhibited a positive ratio of not less than 95% for CD47, CD81, CD90, CD147, and HLA-A, B, C in all the lots. They exhibited a positive ratio of not less than 90% for CD29, CD46, CD55, CD59, CD73, and CD140b in all the lots. They exhibited a positive ratio of not less than 80% for CD9, CD44, CD49b, CD49c, CD98, and EGF-R in all the lots, for which the positive ratio was generally not less than 90%. Further they exhibited a positive ratio of not less than 70% for CD49f and CD166 in all the cells, for which the positive ratio was generally not less than 90%. Furthermore, they exhibited a positive ratio of not less than 60% for CD10, CD13, CD58, CD63, CD151, and CD164 in all the cells, for which the positive ratio was generally not less than 90% (Fig. 5). In addition, the intermediate cells were positive also for CD105 (Fig. 4).

On the other hand, the cells contained in the dental pulp-derived cell preparations exhibited a positive ratio of not less than 95% for CD29, CD59, CD44, and CD164 in all the lots, for which the positive ratio was generally not less than 98%. And they exhibited a positive ratio of not less than 90% for CD9, CD13, CD46, CD47, CD58, CD63, CD73, CD81, CD90, CD98, CD147, EGF-R, and HLA-A, B, C in all the lots, for which the positive ratio was generally not less than 95%. Further, they exhibited a positive ratio of not less than 80% for CD49b, CD49c, CD49e, CD55, CD95, CD151, and CD166 in all the lots, for which the positive ratio was generally not less than 90% except for CD95, CD151, and CD166. And they exhibited a positive ratio of not less than 70% for CD10, CD49f, and CD140b in all the lots, for which the positive ratio was generally not less than 80% (Fig. 6). In addition, the cells contained in the dental pulp-derived cell preparations are positive for CD105, too (Fig. 4).

Further, Table 16 and Table 17 show the surface antigens that were found negative in the intermediate cells and the cells contained in the dental pulp-derived cell preparations, respectively

As to the intermediate cells, their positive ratio was not more than 1% in all the lots for CD195, CD206, CD210, CD212, CD226, CD244, CD267, CD278, CD279, CD282, CD294, NKB1, SSEA-1, TRA-1-60, TRA-1-81, Vβ23, SSEA-3, CLA, and integrin β7. And their positive ratio was not more than 2% in all the lots for CD8b, CD11b, CD15s, CD16, CD19, CD24, CD31, CD32, CD62E, CD62P, CD66f, CD86, CD88, CD94, CD100, CD103, CD104, CD114, CD117, CD118, CD121b,CD122, CD123, CD124, CD126, CD127, CD128b, CD135, CD137, CD137 ligand, CD150, CD163, CD172b, CD177, CD178, CD180, CD197, CD220, CD229, CD231, CD255, CD268, CD305, CD314, CD321, CDw327, CDw328, CD329, CD335, CD336, BLTR-1, CLIP, CMRF-44, CMRF-56, fMLP-R, Vβ8, Invariant NKT, and γδTCR, for which their positive ratio was generally not more than 1%. Further, their positive ratio was not more than 5% in all the cells for CD1a, CD1b, CD1d, CD2, CD3, CD5, CD6, CD7, CD8a, CD11c, CD15, CD18, CD21, CD22, CD23, CD25, CD27, CD28, CD33, CD35, CD37, CD38, CD41a, CD41b, CD42b, CD45, CD45RB, CD45RO, CD48, CD50, CD53, CD62L, CD64, CD66 (a, c, d, e), CD69, CD70, CD72, CD74, CD84, CD85, CD87, CD89, CDw93, CD97, CD134, CD138, CD141, CD144, CD154, CD158b, CD162, CD183, CD205, CD235a, CD309, CD326, CD337, and aβTCR. Furthermore, though a positive ratio exceeding 5% was found in some lots for CD26, CD106, and CD271, their mean positive ratio was not more than 5% (Table 16A-Table 16D). In addition, the intermediate cells were found negative also for CD34, CD40, CD45, CD80, CD86, and MHC-class II antigen (Fig. 4, Fig. 11A).

As to the cells contained in the dental pulp-derived cell preparation, their positive ratio was not more than 1% in all the lots for CD1a, CD1d, CD2, CD3, CD4, CD5, CD7, CD8a, CD8b, CD11b, CD11c, CD15, CD15s, CD16, CD18, CD19, CD20, CD21, CD22, CD23, CD24, CD25, CD28, CD30, CD31, CD32, CD33, CD34, CD35, CD37, CD38, CD41a, CD86, CD87, CD88, CD89, CDw93, CD94, CD100, CD102, CD103, CD114, CD117, CD118, CD120b, CD121b, CD122, CD123, CD124, CD126, CD127, CD128b, CD132, CD134, CD135, CD137, CD137 ligand, CD138, CD144, CD150, CD153, CD154, CD158a, CD158b, CD161, CD162, CD163, CD172b, CD177, CD178, CD180, CD184, CD195, CD196, CD197, CD205, CD206, CD210, CD212, CD220, CD226, CD229, CD231, CD235a, CD244, CD255, CD267, CD268, CD278, CD279, CD282, CD294,C D305,CD309, CD314, CD321, CDw327, CDw328, CD329, CD335, CD336, CD337, BLTR-1, CLIP, CMRF-44, CMRF-56, fMLP-R, SSEA-1, TRA-1-60, CLA, integrin β7, and Invariant NKT. Further, their positive ratio was not more than 2% in all the lots for CD1b, CD6, CD27, CD41b, CD42a, CD42b, CD43, CD45, CD45RB, CD48, CD50, CD53, CD57, CD62E, CD62L, CD62P, CD64, CD66b, CD66f, CD69, CD70, CD72, CD75, CD84, CD85, CD97, CD99R, CD183, CD193, SSEA-3, and γδTCR, and their positive ratio was generally not more than 1%. Furthermore, their positive ratio was not more than 5% for all the lots of CD4v4, CD14, CD36, CD45RA, CD45RO, CD66 (a, c, d, e), CD79b, CD83, CD152, CD209, CD275, CD326, MIC A/B, and aβTCR, and their positive ratio was generally not more than 2%. Though a positive ratio exceeding 5% was found in some lots for CD106 and CD271, their mean positive ratio was not more than 5% (Table 17A - Table E). In addition to the above, the cells contained in the dental pulp-derived cell preparation were negative also for CD34, CD40, CD45, CD80, CD86, and MHC-class II antigen (Fig. 4, Fig. 11B).

Table 16A Surface antigens found negative in intermediate cells

| Surface antigens | Intermediates Mean | SE |
|---|---|---|
| CD1a | 0.49 | 0.32 |
| CD1b | 1.61 | 0.57 |
| CD1d | 0.89 | 0.32 |
| GCD2 | 0.79 | 0.33 |
| CD3 | 0.00 | 0.47 |
| CD5 | 0.00 | 0.49 |
| CD6 | 1.15 | 0.44 |

(continued)

| Surface antigens | Intermediates Mean | SE |
|---|---|---|
| CD7 | 0.64 | 0.30 |
| CD8a | 0.74 | 0.34 |
| CD8b | 0.00 | 0.55 |
| CD11b | 0.00 | 0.32 |
| CD11c | 0.81 | 0.29 |
| CD15 | 1.04 | 0.46 |
| CD15s | 0.58 | 0.18 |
| CD16 | 0.44 | 0.25 |
| CD18 | 0.67 | 0.37 |
| CD19 | 0.25 | 0.32 |
| CD21 | 0.65 | 0.38 |
| CD22 | 0.67 | 0.32 |
| CD23 | 0.70 | 0.29 |
| CD24 | 0.00 | 0.36 |
| CD25 | 0.86 | 0.33 |
| CD26 | 3.49 | 1.18 |
| CD27 | 0.90 | 0.48 |
| CD28 | 0.88 | 0.44 |
| CD31 | 0.25 | 0.32 |
| CD32 | 0.17 | 0.37 |
| CD33 | 0.57 | 0.37 |
| CD35 | 0.61 | 0.39 |
| CD37 | 0.58 | 0.32 |
| CD38 | 0.91 | 0.41 |
| CD41a | 0.40 | 0.35 |
| CD41b | 0.44 | 0.32 |
| CD42b | 0.39 | 0.32 |
| CD45 | 0.67 | 0.34 |
| CD45RB | 0.59 | 0.29 |

Table 16B Surface antigens found negative in intermediate cells

| Surface antigens | Intermediates Mean | SE |
|---|---|---|
| CD45RO | 0.94 | 0.37 |
| CD48 | 0.64 | 0.30 |
| CD50 | 0.84 | 0.50 |
| CD53 | 0.56 | 0.33 |
| CD62E | 0.14 | 0.33 |

(continued)

| Surface antigens | Intermediates Mean | SE |
|---|---|---|
| CD62L | 0.33 | 0.31 |
| CD62P | 0.14 | 0.19 |
| CD64 | 0.27 | 0.33 |
| CD66 (a,c,d,e) | 0.06 | 0.40 |
| CD66f | 0.17 | 0.32 |
| CD69 | 0.40 | 0.45 |
| CD70 | 0.17 | 0.33 |
| CD72 | 0.17 | 0.38 |
| CD74 | 0.00 | 0.66 |
| CD84 | 0.42 | 0.39 |
| CD85 | 0.02 | 0.45 |
| CD86 | 0.23 | 0.21 |
| CD87 | 0.66 | 0.46 |
| CD88 | 0.30 | 0.19 |
| CD89 | 0.56 | 0.32 |
| CDw93 | 0.42 | 0.40 |
| CD94 | 0.38 | 0.23 |
| CD97 | 0.67 | 0.27 |
| CD100 | 0.28 | 0.24 |
| CD103 | 0.24 | 0.19 |
| CD104 | 0.22 | 0.19 |
| CD106 | 4.82 | 1.68 |
| CD114 | 0.12 | 0.18 |
| CD117 | 0.38 | 0.23 |
| CD118 | 0.25 | 0.17 |
| CD120b | 0.04 | 0.05 |
| CD121b | 0.13 | 0.19 |
| CD122 | 0.20 | 0.27 |
| CD123 | 0.21 | 0.18 |
| CD124 | 0.21 | 0.25 |
| CD126 | 0.12 | 0.20 |

Table 16C Surface antigens found negative in intermediate cells

| Surface antigens | Intermediates Mean | SE |
|---|---|---|
| CD127 | 0.23 | 0.26 |
| CD128b | 0.09 | 0.19 |
| CD132 | 0.19 | 0.05 |

(continued)

| Surface antigens | Intermediates Mean | SE |
|---|---|---|
| CD134 | 0.26 | 0.33 |
| CD135 | 0.38 | 0.23 |
| CD137 | 0.22 | 0.26 |
| CD137Ligand | 0.34 | 0.25 |
| CD138 | 0.54 | 0.38 |
| CD141 | 0.81 | 0.37 |
| CD144 | 0.29 | 0.42 |
| CD150 | 0.18 | 0.18 |
| CD154 | 0.26 | 0.26 |
| CD158a | 0.00 | 0.09 |
| CD158b | 0.02 | 0.32 |
| CD161 | 0.02 | 0.20 |
| CD162 | 0.20 | 0.32 |
| CD163 | 0.00 | 0.19 |
| CD172b | 0.21 | 0.25 |
| CD177 | 0.03 | 0.21 |
| CD178 | 0.06 | 0.23 |
| CD180 | 0.16 | 0.27 |
| CD183 | 1.24 | 0.51 |
| CD184 | 0.00 | 0.27 |
| CD195 | 0.00 | 0.27 |
| CD197 | 0.36 | 0.17 |
| CD205 | 0.76 | 0.53 |
| CD206 | 0.00 | 0.17 |
| CD210 | 0.18 | 0.03 |
| CD212 | 0.31 | 0.08 |
| CD220 | 0.03 | 0.19 |
| CD226 | 0.00 | 0.16 |
| CD229 | 0.12 | 0.20 |
| CD231 | 0.00 | 0.20 |
| CD235a | 0.77 | 0.50 |
| CD244 | 0.00 | 0.43 |
| CD255 | 0.16 | 0.18 |

Table 16D Surface antigens found negative in intermediate cells

| Surface antigens | Intermediates Mean | SE |
|---|---|---|
| CD267 | 0.20 | 0.05 |

(continued)

| Surface antigens | Intermediates Mean | SE |
|---|---|---|
| CD268 | 0.19 | 0.18 |
| CD271 | 3.62 | 0.74 |
| CD278 | 0.00 | 0.10 |
| CD279 | 0.17 | 0.17 |
| CD282 | 0.19 | 0.15 |
| CD294 | 0.06 | 0.05 |
| CD305 | 0.33 | 0.21 |
| CD309 | 0.39 | 0.26 |
| CD314 | 0.35 | 0.21 |
| CD321 | 0.40 | 0.25 |
| CD326 | 2.37 | 0.41 |
| CDw327 | 0.34 | 0.22 |
| CDw328 | 0.42 | 0.22 |
| CD329 | 0.37 | 0.18 |
| CD335 | 0.31 | 0.18 |
| CD336 | 0.20 | 0.18 |
| CD337 | 0.84 | 0.42 |
| $\alpha\beta$TCR | 1.44 | 0.27 |
| BLTR-1 | 0.33 | 0.22 |
| CLIP | 0.25 | 0.23 |
| CMRF-44 | 0.48 | 0.23 |
| CMRF-56 | 0.26 | 0.23 |
| fMLP-R | 0.44 | 0.20 |
| $\gamma\lambda$TCR | 0.48 | 0.19 |
| Invariant NKT | 0.08 | 0.15 |
| NKB1 | 0.22 | 0.12 |
| SSEA-1 | 0.00 | 0.15 |
| TRA-1-60 | 0.00 | 0.12 |
| TRA-1-81 | 0.00 | 0.10 |
| V$\beta$23 | 0.21 | 0.13 |
| V$\beta$8 | 0.44 | 0.15 |
| SSEA-3 | 0.11 | 0.05 |
| CLA | 0.07 | 0.05 |
| Integrin $\beta$7 | 0.06 | 0.04 |

Table 17A Surface antigens found negative in cells contained in preparation

| Surface antigens | Preparations Mean | SE |
|---|---|---|
| CD1a | 0.00 | 0.85 |
| CD1b | 0.00 | 0.80 |
| CD1d | 0.00 | 0.83 |
| CD2 | 0.00 | 0.73 |
| CD3 | 0.00 | 0.22 |
| CD4 | 0.00 | 0.76 |
| CD4v4 | 0.52 | 0.84 |
| CD5 | 0.00 | 0.24 |
| CD6 | 0.00 | 0.78 |
| CD7 | 0.00 | 0.77 |
| CD8a | 0.00 | 0.85 |
| CD8b | 0.00 | 0.17 |
| CD11b | 0.00 | 0.23 |
| CD11c | 0.00 | 0.80 |
| CD14 | 0.71 | 0.36 |
| CD15 | 0.05 | 0.21 |
| CD15s | 0.00 | 0.21 |
| CD16 | 0.00 | 0.84 |
| CD18 | 0.00 | 0.82 |
| CD19 | 0.00 | 0.75 |
| CD20 | 0.00 | 0.22 |
| CD21 | 0.00 | 0.75 |
| CD22 | 0.00 | 0.76 |
| CD23 | 0.00 | 0.80 |
| CD24 | 0.00 | 0.24 |
| CD25 | 0.00 | 0.78 |
| CD26 | 9.03 | 1.86 |
| CD27 | 0.00 | 0.78 |
| CD28 | 0.00 | 0.81 |
| CD30 | 0.00 | 0.74 |
| CD31 | 0.00 | 0.69 |
| CD32 | 0.00 | 0.21 |
| CD33 | 0.00 | 0.71 |

Table 17B Surface antigens found negative in cells contained in preparation

| Surface antigens | Preparations Mean | SE |
|---|---|---|
| CD34 | 0.00 | 0.58 |
| CD35 | 0.00 | 0.75 |

(continued)

| Surface antigens | Preparations Mean | SE |
|---|---|---|
| CD36 | 0.71 | 0.28 |
| CD37 | 0.00 | 0.75 |
| CD38 | 0.00 | 0.74 |
| CD41a | 0.00 | 0.75 |
| CD41 b | 0.00 | 0.29 |
| CD42a | 0.00 | 0.69 |
| CD42b | 0.00 | 0.83 |
| CD43 | 0.00 | 0.78 |
| CD45 | 0.00 | 0.83 |
| CD45RA | 1.10 | 0.44 |
| CD45RB | 0.00 | 0.84 |
| CD45RO | 0.74 | 0.43 |
| CD48 | 0.19 | 0.29 |
| CD50 | 0.29 | 0.38 |
| CD53 | 0.00 | 0.73 |
| CD56 | 2.67 | 1.53 |
| CD57 | 0.13 | 0.29 |
| CD62E | 0.00 | 0.75 |
| CD62L | 0.00 | 0.74 |
| CD62P | 0.00 | 0.79 |
| CD64 | 0.00 | 0.85 |
| CD66 (a,c,d,e) | 0.14 | 0.37 |
| CD66b | 0.43 | 0.33 |
| CD66f | 0.00 | 0.84 |
| CD69 | 0.00 | 0.84 |
| CD70 | 0.00 | 0.31 |
| CD72 | 0.00 | 0.29 |
| CD75 | 0.01 | 0.33 |
| CD79b | 0.91 | 0.93 |
| CD83 | 0.51 | 0.86 |
| CD84 | 0.00 | 0.73 |

Table 17C Surface antigens found negative in cells contained in preparation

| | | |
|---|---|---|
| CD85 | 0.00 | 0.35 |
| CD86 | 0.00 | 0.77 |
| CD87 | 0.00 | 0.79 |
| CD88 | 0.00 | 0.82 |

(continued)

| | | |
|---|---|---|
| CD89 | 0.00 | 0.78 |
| CDw93 | 0.00 | 0.24 |
| CD94 | 0.00 | 0.76 |
| CD97 | 0.00 | 0.90 |
| CD99R | 0.00 | 0.30 |
| CD100 | 0.00 | 0.77 |
| CD102 | 0.00 | 0.20 |
| CD103 | 0.00 | 0.80 |
| CD106 | 4.43 | 0.79 |
| CD114 | 0.00 | 0.77 |
| CD117 | 0.00 | 0.72 |
| CD118 | 0.00 | 0.82 |
| CD120b | 0.00 | 0.07 |
| CD121b | 0.00 | 0.78 |
| CD122 | 0.00 | 0.79 |
| CD123 | 0.00 | 0.78 |
| CD124 | 0.00 | 0.77 |
| CD126 | 0.00 | 0.77 |
| CD127 | 0.00 | 0.78 |
| CD128b | 0.00 | 0.81 |
| CD132 | 0.26 | 0.06 |
| CD134 | 0.00 | 0.80 |
| CD135 | 0.00 | 0.76 |
| CD137 | 0.00 | 0.76 |
| CD137Ligand | 0.00 | 0.79 |
| CD138 | 0.00 | 0.76 |
| CD144 | 0.00 | 0.79 |
| CD146 | 9.80 | 3.52 |
| CD150 | 0.00 | 0.80 |

Table 17D Surface antigens found negative in cells contained in preparation

| Surface antigens | Preparations Mean | SE |
|---|---|---|
| CD152 | 0.35 | 0.50 |
| CD153 | 0.00 | 0.74 |
| CD154 | 0.00 | 0.73 |
| CD158a | 0.00 | 0.14 |
| CD158b | 0.00 | 0.23 |
| CD161 | 0.00 | 0.77 |

(continued)

| Surface antigens | Preparations Mean | SE |
|---|---|---|
| CD162 | 0.00 | 0.74 |
| CD163 | 0.00 | 0.78 |
| CD172b | 0.00 | 0.63 |
| CD177 | 0.00 | 0.77 |
| CD178 | 0.00 | 0.77 |
| CD180 | 0.00 | 0.79 |
| CD183 | 0.00 | 0.79 |
| CD184 | 0.00 | 0.14 |
| CD193 | 0.12 | 0.33 |
| CD195 | 0.00 | 0.16 |
| CD196 | 0.00 | 0.71 |
| CD197 | 0.00 | 0.18 |
| CD205 | 0.00 | 0.22 |
| CD206 | 0.00 | 0.79 |
| CD209 | 1.62 | 0.47 |
| CD210 | 0.00 | 2.02 |
| CD212 | 0.00 | 2.09 |
| CD220 | 0.00 | 0.80 |
| CD226 | 0.00 | 0.78 |
| CD229 | 0.00 | 0.79 |
| CD231 | 0.00 | 0.78 |
| CD235a | 0.00 | 0.22 |
| CD244 | 0.00 | 0.11 |
| CD255 | 0.00 | 0.19 |
| CD267 | 0.00 | 2.11 |
| CD268 | 0.00 | 0.67 |
| CD271 | 4.62 | 0.78 |

Table 17E Surface antigens found negative in cells contained in preparation

| CD275 | 0.74 | 0.44 |
|---|---|---|
| CD278 | 0.00 | 0.76 |
| CD279 | 0.00 | 0.73 |
| CD282 | 0.00 | 0.82 |
| CD294 | 0.00 | 2.05 |
| CD305 | 0.00 | 0.78 |
| CD309 | 0.00 | 0.83 |
| CD314 | 0.00 | 0.81 |

(continued)

| | | |
|---|---|---|
| CD321 | 0.00 | 0.75 |
| CD326 | 0.99 | 0.74 |
| CDw327 | 0.00 | 0.77 |
| CDw328 | 0.00 | 0.82 |
| CD329 | 0.00 | 0.83 |
| CD335 | 0.00 | 0.81 |
| CD336 | 0.00 | 0.81 |
| CD337 | 0.00 | 0.74 |
| $\alpha\beta$TCR | 0.95 | 0.27 |
| BLTR-1 | 0.00 | 0.79 |
| CLIP | 0.00 | 0.74 |
| CMRF-44 | 0.27 | 0.18 |
| CMRF-56 | 0.00 | 0.85 |
| fMLP-R | 0.00 | 0.82 |
| $\gamma\delta$TCR | 0.00 | 0.80 |
| Invariant NKT | 0.00 | 0.79 |
| SSEA-1 | 0.00 | 0.12 |
| TRA-1-60 | 0.00 | 0.13 |
| SSEA-3 | 0.96 | 0.21 |
| CLA | 0.14 | 0.13 |
| Integrin $\beta$7 | 0.00 | 2.03 |
| MIC A/B | 0.45 | 0.41 |

Fig. 7 shows those surface antigens for which not less than 10% difference in the positive ratio was found between the intermediate cells and the cells contained in the dental pulp-derived cell preparation. As compared with the intermediate cells, the cell contained in the dental pulp-derived cell preparation, which was prepared through further culture of the intermediate cells by the methods described in Examples 9-15, exhibited a 20% or more increase in the positive ratio for CD39, CD49a, CD61, CD107a, CD107b, and CD143, while exhibiting a 60% decrease in the positive ratio for CD146. These results indicate that the cells contained in the dental pulp-derived cell preparation have a different property from the intermediate cells. Thus, it is shown that performing culture by the methods described in Examples 9-15 gives cells contained in the dental pulp-derive cell preparation as a new kind of cells which differ in their property from the intermediate cells.

[Example 23: Properties of dental pulp-derived cells (Test for ability to secrete vascular endothelial cell growth factor (VEGF)]

The intermediate cells frozen prepared in Example 7 and the dental pulp-derived cell preparation prepared in Example 16 were thawed and centrifuged (1500 rpm, 5 minutes) to spun down the cells. The cells then were suspended in DMEM (10% FBS) medium and seeded onto a cell culture plate at a density of 5000-15000 cells/cm$^2$, and cultured until the cells came to be 90-100% confluent. The cells were washed with PBS, and after addition of the trypsin-EDTA, left undisturbed for 5-10 minutes at 37°C to detach the cells. The DMEM (10% FBS) medium was added to suspend the cells, and the number of viable cells was counted. After suspending the cells in DMEM (10% FBS) medium at a density of $0.7 \times 10^5$ cells/mL, 10 mL of the suspension was seeded to a T25 cell culture plate and cultured for 5 days. Following this culture, the entire volume of the culture supernatant was recovered, and the weight of the recovered culture supernatant was measured using an electronic analytical scale (Shimadzu Corporation), and stored at -80°C until measurement was conducted. After this recovery/removal of the supernatant, the cells were washed with PBS, and following addition of trypsin-EDTA, left undisturbed for 5-10 minutes at 37°C to detach the cells. The cells were suspended by addition of DMEM (10% FBS) medium, and the number of viable cells was counted.

The culture supernatant cryopreserved above was thawed, and VEGF contained in the culture supernatant was labelled using Quantikine ELISA Human VEGF kit (R&D Systems), and read on a microplate reader (Molecular Devices). The concentration of VEGF contained in the culture supernatant was determined by interpolating the read value into a standard curve produced with known concentrations of VEGF. The amount of secreted VEGF was calculated as the amount secreted per a certain number of cells according to the following formula:

[Amount of secreted VEGF = VEGF concentration determined × volume of culture supernatant /number of viable cells ($1 \times 10^5$ cells)]

Fig. 8 shows an example of the results of measurement. The result indicates that while both the intermediate cells and the cells contained in the dental pulp-derived cell preparation have an ability to secrete VEGF, the ability is potentiated in the process of further culture starting with intermediate cells and ending in a dental pulp-derived cell preparation. Since VEGF is a compound having an angiogenetic activity, the dental pulp-derived cell preparation administered to a human can promote vascular regeneration via VEGF in the body. As the ability to secrete VEGF has been potentiated in the cells contained in the dental pulp-derived cell preparation produced via intermediate cells, the dental pulp-derived cell preparation produced by such a production method is considered to be more effective as a medicine for the treatment of diseases in which angiogenetic activity should be exerted.

[Example 24: Properties of dental pulp-derived cells (Test for ability to secrete prostaglandin $E_2$ ($PGE_2$)]

The intermediate cells frozen prepared in Example 7 and the dental pulp-derived cell preparation prepared in Example 16 were thawed and centrifuged (1500 rpm, 5 minutes) to spun down the cells. The cells then were suspended in DMEM (10% FBS) medium and seeded onto a cell culture plate at a density of 5000-15000 cells/cm$^2$, and cultured until the cells came to be 90-100% confluent. The cells were washed with PBS, and after addition of the trypsin-EDTA, left undisturbed for 5-10 minutes at 37°C to detach the cells. The DMEM (10% FBS) medium was added to suspend the cells, and the number of viable cells was counted. After suspending the cells in DMEM (10% FBS) medium at a density of $1 \times 10^5$ cells/mL, 1 mL of this diluted cell suspension was respectively seeded to 6 wells of a 12-well cell culture plate. Further, 1 mL each of DMEM (10% FBS) medium containing 40 mg/mL TNFα (R&D Systems) was added to 3 wells, and 1 mL each of DMEM (10% FBS) medium to the remaining 3 wells, and culture was carried out for 24 hours, wherein the former was designated a TNFα-stimulated group, and the latter a non-stimulated group (control group). Following this culture, the entire volume of the culture supernatant was recovered, the weight of the recovered culture supernatant was measured using an electronic analytical scale (Shimadzu Corporation), and cryopreserved at -80°C until measurement was conducted. After this recovery/removal of the supernatant, the cells were washed with PBS, and following addition of trypsin-EDTA, left undisturbed for 5-10 minutes at 37°C to detach the cells. The cells were suspended by addition of DMEM (10% FBS) medium, and the number of viable cells was counted.

The culture supernatant cryopreserved above was thawed, and prostaglandin $E_2$ ($PGE_2$) contained in the culture supernatant was labelled using Prostaglandin $E_2$ Express EIA Kit (Cayman Chemical), and read on a microplate reader (Molecular Devices). The concentration of $PGE_2$ contained in the culture supernatant was determined by interpolating the read value into a standard curve produced with known concentrations of $PGE_2$. The amount of secreted $PGE_2$ was calculated as a secreted amount per $1 \times 10^5$ cells.

[Amount of secreted $PGE_2$ = $PGE_2$ concentration determined × volume of culture supernatant /number of viable cells ($1 \times 10^5$ cells).

Fig. 9 shows the result of measurement. The result indicates that while both the intermediate cells and the cells contained in the dental pulp-derived cell preparation have an ability to secrete $PGE_2$ in response to TNFα, this ability is potentiated in the process of culture starting with the intermediate cells and ending in the dental pulp-derived cell preparation. Since $PGE_2$ has a potent antiinflammatory activity, the dental pulp-derived cell preparation administered to a human can act on a site of inflammation via $PGE_2$ in the body to suppress tissues destruction accompanying inflammation. As the ability to secrete $PGE_2$ has been potentiated in the cells contained in the dental pulp-derived cell preparation produced via intermediate cells, the dental pulp-derived cell preparation produced by such a production method is considered to be more effective as a medicine for the treatment of diseases in which antiinflammatory activity should be exerted.

[Example 25: Test for kynurenine secretion]

(Test method)

The intermediate cells frozen prepared in Example 7 and the dental pulp-derived cell preparation prepared in Example 16 were thawed and centrifuged to spun down the cells, and the cells were suspended in DMEM (10% FBS) medium and seeded in a cell culture flask at a density of 5000-15000 cells/cm$^2$, and cultured for 4 days until the cells came to be 90-100% confluent. The cells were washed with PBS, and after addition of the trypsin-EDTA (Thermo Fisher Scientific), left undisturbed for 5-10 minutes at 37°C to detach the cells. The DMEM (10% FBS) medium was added to suspend the cells, and the number of viable cells was counted. The cells were seeded in two cell culture flaks at a density of 15000-25000 cells/cm$^2$. To one of these cell culture flasks was added DMEM (10% FBS) medium containing 100 U/mL IFNγ (Shionogi) and to the other flask was added DMEM (10% FBS) medium, and culture was carried out for 3 days, wherein the former was designated the "IFNy-stimulated group" and the latter the "non-stimulated group" (control group). After the culture, the entire volume of cell culture supernatant was recovered. The weight of the recovered culture supernatant was measured using an electronic analytical scale (Shimadzu Corporation), and cryopreserved at -80°C until measurement was conducted. After this recovery/removal of the supernatant, the cells were washed with PBS, and following addition of trypsin-EDTA, left undisturbed for 5-10 minutes at 37°C to detach the cells. The cells were suspended by addition of DMEM (10% FBS) medium, and the number of viable cells was counted of the IFNy-stimulated group and non-stimulated group, respectively. The culture supernatant cryopreserved above was thawed, and following addition of 30% trichloroacetic acid (Nacalai Tesque) and centrifugation (10000 × g, 10 minutes), the concentration of kynurenine contained in the culture supernatant was measured by HPLC (Shimadzu Corporation). The concentration (mol concentration) of kynurenine contained in the culture supernatant was determined by interpolating the measured value into a standard curve produced with known concentrations of kynurenine. The amount of secreted kynurenine was calculated as a secreted amount per a certain number of cells according to the following formula.

$$\text{[Amount of secreted kynurenine = kynurenine concentration determined} \times 208.21 \times \text{volume of culture supernatant /number of viable cells (1} \times 10^5 \text{ cells)]}$$

(Results)

Fig. 10 shows the result. Both the intermediate cells and the cells contained in the dental pulp-derived cell preparation exhibited a marked increase in the amount of secreted kynurenine when cultured in the presence of IFNγ. Kynurenine can suppress proliferation of T cells and induce differentiation of monocytes into antiinflammatory M2 macrophages. Therefore, the multipotent stem cell-enriched dental pulp-derived cells administered to a human is considered to be able to exert an antiinflammatory activity via kynurenine.

[Example 26: Properties of dental pulp-derived cells (test for low antigenicity)]

The intermediate cells frozen prepared in Example 7 and the dental pulp-derived cell preparation prepared in Example 16 were thawed and centrifuged (1500 rpm, 5 minutes) to spun down the cells. The cells then were suspended in DMEM (10% FBS) medium and seeded onto a cell culture plate at a density of 5000-15000 cells/cm$^2$, and cultured until the cells came to be 90-100% confluent. The cells were washed with PBS, and after addition of the trypsin-EDTA, left undisturbed for 5-10 minutes at 37°C to detach the cells. The DMEM (10% FBS) medium was added to suspend the cells, and the number of viable cells was counted. The cells were seeded onto two cell culture plates at a density of 15000-25000 cells/cm$^2$. To one of these cell culture plates was added DMEM (10% FBS) medium containing 100 U/mL IFNγ (Shionogi) and to the other flask was added DMEM (10% FBS) medium, and culture was carried out for 3 days, wherein the former was designated the "IFNy-stimulated group" and the latter the "non-stimulated group" (control group). After the culture, the cells were washed with PBS, and following addition of trypsin-EDTA, left undisturbed for 5-10 minutes at 37°C to detach the cells. The cells were suspended by addition of DMEM (10% FBS) medium, and the number of viable cells was counted of the IFNγ-stimulated group and non-stimulated group, respectively. The cell suspension of each group then was centrifuged (1500 rpm, 5 minutes) to remove the supernatant, and following addition of the blocking solution (described in Example 21) to adjust the cell density to $1 \times 10^7$ cells/mL, the cells were left undisturbed on ice for 1 hour.

As antibodies to MHC-class I antigen, MHC-class II antigen, CD40, CD80, and CD86, FITC-labelled anti-human MHC-class I antigen antibody (Ancell), FITC- labelled anti-human MHC-class II antigen antibody (Ancell), FITC-labelled anti-human CD40 antibody (BD Biosciences), FITC- labelled anti-human CD80 antibody (BD Biosciences), FITC- labelled anti-human CD86 antigen antibody (BD Biosciences), respectively, were employed. As a control antibody, FITC-labelled mouse IgG1 isotype control (Beckman Coulter), and FITC-labelled mouse IgG2a isotype control (BD Biosciences) were employed.

The antibody solutions were added to 5-mL reaction tubes (Nos. (1)-(16)) as shown in Table 18. To each of the 5-mL reaction tubes (1)-(8) then was added 100 μL cell suspension of the IFNy-stimulated-group, and to each of the 5-mL reaction tubes (9)-(16) was added 100 μL cell suspension of the IFNy-non-stimulated group, and following light shaking, the suspensions were left undisturbed for 20 minutes on ice to let the antibodies contained in the antibody solutions bind to surface antigen markers expressed on cell surfaces. PBS-B then was added 3 mL each, mixed, and the cells were centrifuged (1500 rpm, 5 minutes) to spun down the cells, and antibodies unbound to the cells were removed by removing the supernatant. This procedure for removing antibodies was repeated 3 times. To the tubes then were added 400 μL each of PBS-B to suspend the cells.

Table 18. Kind and amount of antibody solutions added to reaction tubes

| Tube numbers | Antibody solutions | Volume added |
|---|---|---|
| (1), (9) | - | - |
| (2), (10) | IgG1-FITC | 20 |
| (3), (11) | IgG2a-FITC | 20 |
| (4), (12) | anti-MHC-Class I | 2 |
| (5), (13) | anti-MHC-Class II | 2 |
| (6), (14) | anti-CD40-FITC | 20 |
| (7), (15) | a nti-CD80-FITC | 20 |
| (8), (16) | a nti-CD86-FITC | 20 |

The cells in each of the tubes Nos. (1)-(16) were measured for the amount of the fluorochrome FITC using BD FACSVerse (BD), and in comparison with a negative control, the amount of the fluorochrome was determined, which bound to the cell surfaces via antibodies specifically bound to the surface antigens. Besides, tube (2) corresponds to the negative control for tubes (4) and (6)-(8), tube (3) for tube (5), tube (10) for tubes (12) and (14)-(16), and tube (11) for tube (13), respectively. Tubes (1) and (9) correspond to non-stained cells.

(Results)

The results are shown in Fig. 11A and Fig. 11b. As to the results of measurement of MHC-class I antigen, nearly all the cells both of the intermediate cells and the dental pulp-derived cell preparation were found positive for the antigen regardless of IFNγ stimulation. As to the results of measurement of MHC-class II antigen, although substantially all the cells of both the intermediate cells and the dental pulp-derived cell preparation were negative for the antigen when not stimulated with IFNγ, most of them turned positive after stimulation with IFNγ. As to the results of measurement of CD40, CD80, and CD86, nearly all the cells of both the intermediate cells and the dental pulp-derived cell preparation were negative for the antigen regardless of IFNγ stimulation.

MHC-class I antigen and MHC-class II antigen are the cell surface antigens which function to display antigens to immune cells. MHC-class I antigen is known to be expressed on almost all the cells, and expression of MHC-class II antigen is known to be induced by IFNγ stimulation in many of cells. On the other hand, CD40, CD80, and CD86 are surface antigens which take part in the activation of cells of the immune system. Thus, as neither the intermediate cells frozen nor the dental pulp-derived cell preparation express CD40, CD80, or CD86, they are not considered to activate immune cells by themselves. Consequently, it is indicated by these results that either the intermediate cells frozen or the cells contained in the dental pulp-derived cell preparation are free of immunogenicity, and will not turn immunogenic even if stimulated with IFNγ.

[Example 27: Measurement of abilities of the intermediate cells and the cells contained in the dental pulp-derived cell preparation to secrete various factors including cytokines]

(Preculture)

The intermediate cells frozen prepared in Example 7 and the dental pulp-derived cell preparation prepared in Example 16 were thawed, and the entire volume of the cell suspension thus melt was added to DMEM medium. After centrifugation (300 × g, 5 minutes, room temperature) to remove the supernatant, the cells were suspended by addition of DMEM medium, and the number of viable cells and the number of the total cells in the cell suspension were counted using Muse Cell Analyzer (Millipore Sigma) to calculate the viable cell ratio. T225 flasks were seeded with cells so that the number of viable cells would be 7000 cells/cm$^2$ in the case of the cells contained in the intermediate cells frozen and 12000 cells/cm$^2$ in the case of cells contained in dental pulp-derived cell preparation, and cultured for 4 days (37°C, 5% CO$_2$) in a CO$_2$ incubator. After the culture, the medium was removed from the T225 flasks, and the cells were washed with D-PBS, and following addition of 0.25% Trypsin-EDTA, left undisturbed for 5 minutes in a CO$_2$ incubator. After having confirmed that the cells had been detached, DMEM medium was added to suspend the cells, and the whole volume was recovered. The cells were spun down by centrifugation (300 × g, 5 minutes, room temperature). The supernatant was removed, and after suspending the cells by addition of DMEM medium, the number of viable cells and the number of the total cells in the cell suspension were counted using Muse Cell Analyzer (Millipore Sigma) to calculate the viable cell ratio.

(Recovery of culture supernatant)

The cells recovered in the preculture were seeded in T25 flasks so that the number of viable cells would be 28000 cells/flask, and after allotting them to one of non-stimulated group, TNFα-stimulated group, and IFNy-stimulated group, culture was started. The volume of the medium for each group was 10 mL/flask, and the final concentration of TNFα was adjusted to 10 ng/mL (stock solution: 2 μg/mL) and the final concentration of IFNγ was adjusted to 100 U/mL (stock solution: 1×10$^6$ U/mL). On day 3 from the start of culture, the culture supernatant was recovered, 0.5 mL each, into cryotubes, and cryopreserved. After recovery of the culture supernatant, cells were washed with D-PBS, and following addition of 0.25% Trypsin-EDTA, left undisturbed for 5 minutes in a CO$_2$ incubator. After confirming that the cells had been detached, DMEM medium was added, and the whole volume was recovered. After centrifugation (300 × g, 5 minutes, room temperature) and removal of the supernatant, the cells were suspended by addition of DMEM medium, and the number of viable cells and the number of the total cells in the cell suspension were counted using Muse Cell Analyzer (Millipore Sigma) to calculate the viable cell ratio.

(Measurement of cytokines)

The concentration of various factors including cytokines contained in the recovered supernatant was measured using LEGENDplex (BioLegend). The principle of measurement using LEGENDplex is outlined here: Beads-bound primary antibodies differing antigen to antigen (kinds of cytokines) are added to samples containing compounds to be measured, and the primary antibodies are allowed to bind to the compounds. Biotinylated secondary antibodies then are added to let them bind to the compounds to which the primary antibodies had bound, to which phycoerythrin (PE)-labelled streptavidin is allowed to bind further. Since avidin specifically binds to biotin, measuring it using flowcytometry enables determination of the amount of phycoerythrin (PE)-labelled streptavidin bound to the beads as being corresponding to the fluorescence intensity. As the amount thus determined is in proportion to the amount of the compound to be measured in the sample, it allows quantification of the compound.

(Results: Amount of factors expressed in non-stimulated group)

The concentration of factors contained in the culture supernatant of the non-stimulated group is shown in Fig. 12. Both the intermediate cells and the cells contained in the dental pulp-derived cell preparation express MMP-2, IGFBP-4, and cystatin C at high levels (Fig. 12).

Further, both the intermediate cells and the cells contained in the dental pulp-derived cell preparation express IL-6, IL-11, MCP-1,I L-8, GROα, HGF, VEGF, VCAM-1, TIMP-3, TIMP-2, and TIMP-1 (Fig.13).

Further, although in a minute amount, both the intermediate cells and the cells contained in the dental pulp-derived cell preparation express IL-23, TNF-α, IL-18, IL-33, IL-27, TARC, ENA-78, MIP-3α, MIP-1β, IP-10 (CXCL10), SCF, and ICAM-1 (Fig.14). Furthermore, either the intermediate cells or the cells contained in the dental pulp-derived cell preparation do not express IL-21, Exotaxin, MIP-1α, MIG, I-TAC, or GM-CSF, or they hardly express these. While IFN-α expression is not detected in the intermediate cells, it is expressed in a minute amount in the cells contained in the dental pulp-derived cell preparation. Furthermore, though not shown in the tables, either the intermediate cells or the cells contained in the dental pulp-derived cell preparation do not express inflammatory cytokines IL-2, IL-4, IL-5, IL-9, or IL-13, or they hardly express these.

(Results: Differences in the amount of factors expressed between intermediate cells and cells contained in the dental pulp-derived cell preparation, in non-stimulated groups)

Fig. 15 shows the concentration ratios of those factors contained in the culture supernatant whose expression amount relatively differs between the intermediate cells and the cells contained in the dental pulp-derived cell preparation. Expression amount of those factors is greater in the cells contained in the dental pulp-derived cell preparation than in the intermediate cells. In particular, the expression amount of IL-6, IL-11, HGF, IGFBP-4, TIMP-3, and TIMP-1 is 1.5 times or more greater in the cells contained in the dental pulp-derived cell preparation than in the intermediate cells.

(Results: Change in expression amount of IL-6 by stimulation with TNF-$\alpha$ and IFN-$\gamma$)

Fig. 16 shows the IL-6 concentration in the culture supernatant when stimulated with TNF-$\alpha$ and IFN-$\gamma$. In the intermediate cells, expression amount of IL-6 increases when stimulated either with TNF-$\alpha$ or IFN-$\gamma$ as compared with a non-stimulated condition. The same is also observed in the cells contained in the dental pulp-derived cell preparation.

(Results: Change in expression amount of IL-11 by stimulation with TNF-$\alpha$ and IFN-$\gamma$)

The concentration of IL-11 contained in the culture supernatant when stimulated with TNF-$\alpha$ and IFN-$\gamma$ is shown in Fig. 17. The expression amount of IL-11 increases under stimulation either with TNF-$\alpha$ or IFN-$\gamma$ in the intermediate cells as compared with a non-stimulated condition. The same is also observed in the cells contained in the dental pulp-derived cell preparation.

(Results: Change in expression amount of IP-10 (CXCL 10) by stimulation with TNF-$\alpha$ and IFN-$\gamma$)

The concentration of IP-10 (CXCL 10) contained in the culture supernatant when stimulated with TNF-$\alpha$ and IFN-$\gamma$ is shown in Fig. 18. Though the concentration of IP-10 does not appear in the figure with the intermediate cells, that is due to low concentrations of IP-10, and it is expressed even under a non-stimulated condition as shown in Fig. 14. The same is also observed in the cells contained in the dental pulp-derived cell preparation.

(Results: Change in expression amount of MCP-1 by stimulation with TNF-$\alpha$ and IFN-$\gamma$)

The concentration of MCP-1 contained in the culture supernatant when stimulated with TNF-$\alpha$ and IFN-$\gamma$ is shown in Fig. 19. The expression amount of MCP-1 increases when stimulated either with TNF-$\alpha$ or IFN-$\gamma$ in the intermediate cells as compared with a non-stimulated condition. The same is also observed in the cells contained in the dental pulp-derived cell preparation.

(Results: Change in expression amount of GM-CSF by stimulation with TNF-$\alpha$ and IFN-$\gamma$)

The concentration of GM-CSF contained in the culture supernatant when stimulated with TNF-$\alpha$ and IFN-$\gamma$ is shown in Fig. 20. Under no stimulation, expression of GM-CSF is hardly observed either in the intermediate cells or in the cells contained in the dental pulp-derived cell preparation. While expression of GM-CSF is induced by TNF-$\alpha$ stimulation in the intermediate cells, no expression is induced by IFN-$\gamma$ stimulation. The same is also observed in the cells contained in the dental pulp-derived cell preparation.

(Results: Change in expression amount of HGF by stimulation with TNF-$\alpha$ and IFN-$\gamma$)

The concentration HGF contained in the culture supernatant when stimulated with TNF-$\alpha$ and IFN-$\gamma$ is shown in Fig. 21. While the expression amount of HGF decreases in the intermediate cells by TNF-$\alpha$ stimulation as compared with a non-stimulated condition, it increases by IFN-$\gamma$ stimulation. The same is also observed in the cells contained in the dental pulp-derived cell preparation.

(Results: Change in expression amount of IL-8 by stimulation with TNF-$\alpha$ and IFN-$\gamma$)

The concentration IL-8 contained in the culture supernatant when stimulated with TNF-$\alpha$ and IFN-$\gamma$ is shown in Fig. 22. While the expression amount of IL-8 increases in the intermediate cells by TNF-$\alpha$ stimulation as compared with a non-stimulated condition, no change is observed by IFN-$\gamma$ stimulation. The same is also observed in the cells contained in the dental pulp-derived cell preparation.

[Example 28: Properties of dental pulp-derived cells (ability to undergo cell divisions)]

The intermediate cells frozen prepared in Example 7 and the dental pulp-derived cell preparation prepared in Example 16 were thawed and centrifuged (1500 rpm, 5 minutes) to spun down the cells. The cells then were suspended in DMEM (10% FBS) medium and seeded to a cell culture plate at a density of 5000-15000 cells/cm$^2$, and cultured until the cells came to be 90-100% confluent. The cells were washed with PBS, and after addition of the trypsin-EDTA, left undisturbed for 5-10 minutes at 37°C to detach the cells. The DMEM (10% FBS) medium was added to suspend the cells, and the number of viable cells was counted. The cells were seeded again to a cell culture plate at a density of 5000-15000 cells /cm$^2$, and cultured until the cells came to be 90-100% confluent. Passage culture of the cells were repeated, and the number of viable cells was counted at the end of every passage culture to calculate the number of cell divisions. The number of cell divisions were calculated based on the number of viable cells at the start of each passage culture and the number of viable cells at the end of it, using the following formula.

Number of cell divisions = log$_2$ (number of viable cells at the end of passage culture/ number of viable cells at the at the start of passage culture)

(Results)

The cells contained in the intermediate cells frozen had, after they are thawed, an ability to divide not less than 14 times, with their doubling time being not more than 3 days (not more than 72 hours). Further, the cells contained in the dental pulp-derived cell preparation had, after they are thawed, an ability to divide not less than 4 times, with their doubling time being not more than 3 days (not more than 72 hours).

[Example 29: Test of intermediate cells and the cells contained in dental pulp-derived cell preparation for ability to secrete granulocyte colony-stimulating factor (G-CSF)]

(Preculture)

The intermediate cells frozen prepared in Example 7 and the dental pulp-derived cell preparation prepared in Example 16 were thawed, and the entire volume of the cell suspension thus melt was added to DMEM medium. After centrifugation (300 × g, 5 minutes, room temperature) and removal of the supernatant, the cells were suspended by addition of DMEM medium, and the number of viable cells and the number of the total cells in the cell suspension were counted using Muse Cell Analyzer (Millipore Sigma) to calculate the viable cell ratio. T225 flasks were seeded with cells so that the number of viable cells would be 7000 cells/cm$^2$ in the case of the cells contained in the intermediate cells frozen and 12000 cells/cm$^2$ in the case of dental pulp-derived cell preparation, and cultured for 4 days (37°C, 5% CO$_2$) in a CO$_2$ incubator. After the culture, the medium was removed from the T225 flasks, and the cells were washed with D-PBS, and following addition of 0.25% Trypsin-EDTA, left undisturbed for 5 minutes in a CO$_2$ incubator. After having confirmed that the cells had been detached, DMEM medium was added to suspend the cells, and the whole volume was recovered. The cells were spun down by centrifugation (300 × g, 5 minutes, room temperature). The supernatant was removed, and after suspending the cells by addition of DMEM medium, the number of viable cells and the number of the total cells in the cell suspension were counted using Muse Cell Analyzer (Millipore Sigma) to calculate the viable cell ratio.

(Recovery of culture supernatant)

The cells recovered in the preculture were seeded in T25 flasks so that the number of viable cells would be 28000 cells/flask, and after allotting them to one of non-stimulated group, TNFα-stimulated group, and IFNy-stimulated group, culture was started. The volume of the medium for each group was 10 mL/flask, and the final concentration of TNFα was adjusted to 10 ng/mL (stock solution: 2 μg/mL) and the final concentration of IFNγ was adjusted to 100 U/mL (stock solution: 1×10$^6$ U/mL). On day 3 from the start of culture, the culture supernatant was recovered, 0.5 mL each, into cryotubes, and cryopreserved. After recovery of the culture supernatant, cells were washed with D-PBS, and following addition of 0.25% Trypsin-EDTA, left undisturbed for 5 minutes in a CO$_2$ incubator. After confirming that the cells had been detached, DMEM medium was added, and the whole volume was recovered. After centrifugation (300 × g, 5 minutes, room temperature) and removal of the supernatant, the cells were suspended by addition of DMEM medium, and the number of viable cells and the number of the total cells in the cell suspension were counted using Muse Cell Analyzer (Millipore Sigma) to calculate the viable cell ratio.

(Measurement of granulocyte colony-stimulating factor (G-CSF))

The concentration of granulocyte colony-stimulating factor (G-CSF) contained in the recovered supernatant was measured using LEGENDplex (BioLegend).

(Results: Change in expression amount of G-CSF by stimulation with TNF-$\alpha$ and IFN-$\gamma$)

The concentration G-CSF contained in the culture supernatant when stimulated with TNF-$\alpha$ and IFN-$\gamma$ is shown in Fig. 23. Under no stimulation, almost no expression of G-CSF is observed either in the intermediate cells or the cells contained in the dental pulp-derived cell preparation. In the intermediate cells, expression of G-CSF is induced by TNF-$\alpha$ stimulation, but not by IFN-$\gamma$ stimulation. The same is also observed in the cells contained in the dental pulp-derived cell preparation. G-CSF receptor is expressed on the neurons in the brain and the spinal cord, and G-CSF is reported to have an activity to protect nerves, and its clinical studies was conducted on cerebral infarction (Shyu WC.CMAJ.174.927-33(2006)). G-CSF is also suggested to have a therapeutic effect on spinal cord injury (Nishio Y.J Neuropathol Exp Neurol.66.724-31). Further, it has also been reported that G-CSF suppresses expression of inflammatory cytokines, TNF-$\alpha$, IL-1$\beta$ (Masao Koda, Orthopedic Surgery, 58.1464(2007)). It therefore is foreseeable that the intermediate cells or the cells contained in the dental pulp-derived cell preparation would exhibit a therapeutic effect on neuronal injuries, e.g., in the brain, the spinal cord, and the like, via G-CSF.

[Comparative Example 1: Culture of human bone marrow-derived mesenchymal stem cells (hMSC-BM)

Mesenchymal stem cells isolated from normal human bone marrow were employed (Takara Bio, mfd. by PromoCell, Lot: 413Z021.4 or 429Z022)). The cells were plate cultured in petri dish with DMEM (Thermo Fisher Sicentific, Cat. No.: 10567) supplemented with 10% fetal bovine serum (FBS, Hyclone Laboratories, Cat. No.: SH30396) or with a manufacturer's recommended medium (PromoCell, Cat. No.:C-28009) up to 3-6$\times10^7$ cells, recovered, suspended in Stem-CellBanker (Takara Bio, Cat. No.:119229), and cryopreserved.

[Example 30: Properties of dental pulp-derived cells (test for ability to secrete angiopoietin-1 (Ang-1))]

The dental pulp-derived cell preparation prepared in Example 16 were thawed, seeded to a 12-well plate at $1\times10^5$ cells/well, and cultured for one day with DMEM (10% FBS) medium. The medium then was replaced with 500 $\mu$L of FBS-free DMEM, and following further culture for 24 hours, the culture supernatant was recovered, and the cells were detached from the plate to count their number. The concentration of human Ang-1 in the culture supernatant was measured by ELISA (R&D systems, cat. No.: DANG10). The amount of Ang-1 secreted was calculated by multiplying the Ang-1 concentration in the culture supernatant by the amount of the culture supernatant (500 $\mu$L) and then dividing the result by the number of the cells. The test was conducted with 3 samples and the mean value and the standard error were calculated. Further, the amount of Ang-1 secreted were determined as the amount secreted by $1\times10^4$ cells. The amount of Ang-1 secreted was 132 $\pm$ 23 (pg/10$^4$ cells, mean $\pm$ standard error).

[Comparative Example 2: test of human bone marrow-derived mesenchymal stem cells (hMSC-BM) for ability to secrete angiopoietin-1 (Ang-1)]

With the human bone marrow-derived mesenchymal stem cells (hMSC-BM) prepared in Comparative Example 2, the amount of angiopoietin-1 secreted was measured in the same manner as in Example 30. The amount of Ang-1 secreted was 16 $\pm$ 2 (pg/10$^4$ cells, mean $\pm$ standard error).

(Results)

Fig. 24 summarizes the result of measurement of amount of angiopoietin-1 (Ang-1) secreted by the dental pulp-derived cells and the hMSC-BM. The results indicate that the dental pulp-derived cells secrete approximately 8.25 times greater amount of angiopoietin-1 than hMSC-BM (Fig. 24).

[Example 31: Comparison of actions on vascular permeability using human umbilical vein endothelial cells (HUVEC)]

(Preparation of culture supernatant)

The dental pulp-derived cell preparation prepared in Example 16 were thawed and mixed with HUVEC (PromoCell, Cat. No.:C12200) at various ratios to prepare cell mixture-containing solutions. A mixing ratio was the number calculated as [mixing ratio = number of dental pulp-derived cells/number of all the cells × 100%]. Those cell mixture-containing solutions were seeded to a 24-well plate at 0.5 × $10^5$ cells/well on total cell basis, and cultured for 3 days in DMEM medium (10% FBS). The medium then was replaced with 0.5 mL of endothelial cell basal medium (ScienCell, Cat. No.: 1001), and after a 24-hour culture, the culture supernatant was recovered. The supernatant having undergone centrifugation was employed to examine its action on vascular permeability.

(Examination of actions on vascular permeability)

Collagen-coated 24-well plate insert were seeded with HUVEC suspended in the endothelial cell basal medium, at 1 × $10^4$ cells/well, and placed in a 24-well plate where the endothelial cell basal medium had been added, and static culture was conducted for 3-4 days to let a HUVEC monolayer form on the membrane in the insert. After the culture, all the medium in the insert and the plate was removed. To each insert then was added the above culture supernatant or the endothelial cell basal medium supplemented with human recombinant angiopoietin-1 solution (Merck, Cat. No.: GF164) at a concentration of 100 ng/mL, placed in a 24-well plate containing the endothelial cell basal medium, and left undisturbed for 1 hour. The whole medium in the insert and the plate was removed again. To each insert then was added the endothelial cell basal medium containing 25 μg/ml FITC-dextran (Sigma-Aldrich, Cat. No.: FD70S-100MG), and placed undisturbed in a 24-well plate containing the endothelial cell basal medium for 30 minutes.

The medium in each plate well then was recovered, and fluorescence (Ex 485 nm/Em 535 nm) emitted from the FITC-dextran that had passed from the insert to the well plate side through the HUVEC monolayer was measured with a fluorophotometer. The fluorescence intensity of each culture supernatant was calculated as a relative permeability (%) to the fluorescence intensity of the culture supernatant obtained by culture of the cells whose mixing ratio was 0% (i.e., HUVEC only), which was set as 100%. Having repeated the same experiment 3-4 times, the mean and the standard error was calculated for each group.

[Comparative Example 3] A relative permeability (%) was determined by the same method as in Example 31 using the human bone marrow-derived mesenchymal stem cells (hMSC-BM) obtained in Comparative Example 1. Besides, the mixing ratio in this case was a value determined as [mixing ratio = number of human bone marrow-derived mesenchymal stem cells (hMSC-BM)/number of all the cells × 100%].

(Results)

The results are shown in Fig. 25. The relative permeability measured by applying the culture supernatant which was obtained by culturing a mixture of dental pulp-derived cells with HUVEC at a mixing ratio of 10, 50, or 100%, to a HUVEC monolayer was 79.0 ± 10.4, 65.9 ± 9.7, and 63.4 ± 6.5 (%, mean ± standard error), respectively, where a decrease in the relative permeability was observed by application of the culture supernatant obtained at a mixing ratio of 10%, and a statistically significant decrease in the relative permeability was observed by application of the culture supernatant obtained at a mixing ratio of 50%. On the other hand, the relative permeability measured by applying the culture supernatant obtained by culturing a mixture of hMSC-BM with HUVEC at a mixing ratio of 10, 50, or 100%, to a HUVEC monolayer was 99.6 ± 5.6, 81.4 ± 4.0, and 62.8 ± 14.4 (%), respectively. Further, the relative permeability measured by application of a medium supplemented with rhAng-1, instead of a culture supernatant, was 67.7 ± 6.7 (%), with a statistically significant suppression observed. These results indicates that the dental pulp-derived cells have a more potent suppressive activity on permeability of vascular endothelium than hMSC-BM. Furthermore, in combination with the results obtained in Example 30, it is suggested that the activity of administered dental pulp-derived stem cells to suppress the permeability of vascular endothelium is exerted via angiopoieytin-1 (Ang-1) secreted by the dental pulp-derived stem cells.

[Example 32: Comparison between dental pulp-derived stem cells and human bone marrow-derived mesenchymal stem cells (hMSC-BM) in the effect on neutrophil infiltration in lipopolysaccharide (LPS)-induced acute lung injury mouse model]

(Administration)

The dental pulp-derived cell preparation prepared in Example 16 were thawed, and the dental pulp-derived cells and hMSC-BM were compared in their action on neutrocyte infiltration into sites of damaged tissues using an LPS-induced acute lung injury mouse model, in which an acute injury takes place in the lung, where microvessels are abundant. The dental pulp-derived cells prepared in Example 16 were thawed, and administered to male, 10-week old C57BU6J mice (Charles River Laboratories Japan), at a number of 1.5 × $10^3$, or 1.5 × $10^4$ cells/body via tail vein. To negative control animals was administered a vehicle (cryopreservation solution for cells for a preparation).

(Induction of pathology)

The mice administered with the dental pulp-derived cells or the vehicle were, 24 hours thereafter, kept for 1 hour in a chamber filled with atomized 0.5 mg/mL LPS solution (Sigma-Aldrich, Cat. No.: L4130) to let them aspirate LPS and for acute lung injury to take place.

(Count of cells in pulmonary alveoli wash solution)

Twenty-four hours after termination of LPS aspiration, the mice were euthanized by exsanguination. A cannula was inserted into a bronchus and 1 mL of phosphate buffered physiological saline (PBS, Sigma-Aldrich, Cat. No.: D8537-1L) was infused to wash the pulmonary alveoli. The pulmonary alveoli wash solution was recovered, and the total number of cells contained in the solution was counted using a cell counter under a microscope.

[Comparative Example 4]

Experiment was conducted in same matter as in Example 32 using the human bone marrow-derived mesenchymal stem cells (hMSC-BM) prepared in Comparative Example 1.

(Results)

The results are shown in Fig. 26. The total number of cells in the pulmonary alveoli wash solution of the negative control group was markedly greater than the non-pathological group, revealing cell infiltration into the pulmonary alveoli due to the induction of pathology. In the group administered with dental pulp-derived cells, the total number of cells in the pulmonary alveoli wash solution was reduced depending on the number of the cells administered, with a statistically significant reduction in the total number of cells at a dose of $1.5 \times 10^3$ cells/body or more. On the other hand, in the group administered with hMSC-BM, although a tendency of reduction was seen at a dose of $1.5 \times 10^4$ cells/body, no statistically significant reduction was observed with either of the doses tested. Meanwhile, it is known that neutrophils infiltrate into the pulmonary alveoli in the mouse model employed above. The above results thus indicate that the dental pulp-derived cells have a more potent activity to suppress neutrophil infiltration into the sites of damaged tissues than hMSC-BM.

[Example 33: Comparison between dental pulp-derived stem cells and human bone marrow-derived mesenchymal stem cells (hMSC-BM) in the effect on VCAM (vascular cell adhesion molecule)-1 expression in lipopolysaccharide (LPS)-induced acute lung injury mouse model] (Induction of pathology)

Preparation of LPS-induced acute lung injury mouse model, administration of dental pulp-derived cells, and collection of pulmonary alveoli wash solution were carried out by the method described in Example 32.

(Western blotting)

Having collected the pulmonary alveoli wash solution, the lungs were excised, part of which was homogenized with RIPA buffer (composition: 100 mM Tris-HC l(pH7.4), 150mM NaCl, 1% NonidetP40, 0.5% Sodium Deoxycholate, 0.1% SDS), which was a tissue lysis buffer, to prepare a tissue extraction solution. Samples were applied to respective lanes at total protein mass of 10 $\mu$g, and following SDS-PAGE electrophoresis, transferred to a membrane. After immunostaining with anti-VCAM-1 antibody (R&D Systems, Cat. No.: AF643-SP) and detection of the band originating from the protein of interest by an augmented chemiluminescence method, the signal intensities were quantified using an image analysis software. The expression amount of VCAM-1 protein was calculated as a relative value to the mean intensity of the signal originating from VCAM-1 in the non-pathological group, whose mean signal intensity was set as 1.

[Comparative Example 5]

The experiment was carried out in the same method as in Example 33 using the human bone marrow-derived mesenchymal stem cells (hMSC-BM) prepared in Comparative Example 1.

(Results)

The results are shown in Fig. 27. The amount of VCAM-1 protein expressed in the negative control group was higher than that in the non-pathological group with a statistical significance. In the group which was administered with the dental pulp-derived cells at a dose of $1.5 \times 10^4$ cell/body, the expressed amount of VCAM-1 protein was lower than that in the negative control group with a statistical significance, and suppressed to a level comparable to that in the non-pathological group. On the other hand, in the group which was administered with hMSC-BM at a dose of $1.5 \times 10^4$ cell/body, no notable difference from the vehicle group was observed in the expressed amount of VCAM-1 protein. VCAM-1 is an adhesion factor expressed on the surface of vascular endothelial cells, and neutrophils can adhere to vascular endothelial cells via VCAM-1. This adhesion of neutrophils to vascular endothelial cells is an important step for the neutrophils to infiltrate into tissues. Combined with the results in Example 32, it is suggested that the suppression of the amount of VCAM-1 protein expression, which is caused by administered dental pulp-derived cells at site of inflammation, takes part in the suppression of the neutrophil infiltration into tissues by the administered dental pulp-derived cells. In addition to neutrophils, lymphocytes and monocytes also have an ability to adhere to vascular endothelial cells via VCAM-1. Therefore, the dental pulp-derived cells administered is considered to suppress infiltration of not only neutrophils, but lymphocytes and monocytes, into sites of injury.

[Example 34: Effect by administration of dental pulp-derived cell preparation on recovery from neurological symptoms (cerebral infarction rat model)]

(Preparation of cerebral infarction model rats (SD rats))

Male, 8-week-old Sprague-Dawley rats (Charles River Laboratories Japan), under inhalation anesthesia with 2% isoflurane (Lot:165AMM, Mylan Seiyaku) (nitrous oxide : oxygen=7 : 3), were immobilized in dorsal position. The right common carotid artery, the right external carotid artery, and the right internal carotid artery of the rats were exposed, and the right common carotid artery and the right external carotid artery were ligated with a suture thread. A nylon thread coated with silicone (Heraeus Kulzer Japan) (embolus), cut 19 mm in length, was inserted from the bifurcation point between the right external carotid artery and the right internal carotid artery to clog the right middle cerebral artery (MCA). Following the occlusion of the right MCA and suturing of the neck skin, the animals were allowed to recover from anesthesia. Thirty minutes after MCA occlusion, it was confirmed that the forelimb on the side opposite to the occluded side (left forelimb) was crooked. Under inhalation anesthesia with 2% isoflurane, the right common carotid artery and the right internal carotid artery were exposed again, and the embolus was taken out 2 hours after MCA occlusion to restore the blood flow through the right MCA, thereby causing cerebral infarction. Following restoration of the blood flow through the right MCA, the right internal carotid artery was ligated, the head skin was sutured, and the rats were allowed to recover from anesthesia. These animals were used in the following experiment as cerebral infarction model rats. Besides, prior to the MCA occlusion operation, benzylpenicillin potassium (Lot: PGSD804, Meiji Seika Pharmacia) was intramuscularly administered at a dose of 20000 units/kg.

(Administration of dental pulp-derived cells)

The dental pulp-derived cell preparation prepared in Example 16 was thawed, and the dental pulp-derived cells were administered to the cerebral infarction model animals 24 hours after the MCA occlusion, at $1.0 \times 10^6$ cells/body via the tail vein. The negative control group was administered with the vehicle.

(Rota-rod test)

Before the creation of cerebral infarction, the animals were trained using a Rata-rod (KN-75, Natsume Seisakusho), 9 cm in diameter and 8 cm in width, whose rotation rate was set at 8 rev/minute. The period of training was for 2 days at longest, and the training was given so that they would get able to stay on the rod for 120 seconds, which value was used as the measurement before the operation (pre-value). On days 3, 7, 14, and 28 after creation of cerebral infarction, staying time (seconds) on the rod was measured using the Rota-rod, with the rotation rate set at 8 rev/minute. The maximum length of time for measurement was 120 seconds.

(Results)

The results are shown in Fig. 28. In the Rota-rod test, the staying time on the rod was constantly longer in the dental pulp-derived cells-administered group than that in the vehicle-administered group, and in particular, a significant difference was observed on day 14 and thereafter between the two groups. The results indicates that dental pulp-derived cells have an ability to improve neurological symptoms in a rat having cerebral infarction.

[Example 35: Neutrocyte infiltration into sites of infarction in rat cerebral infarction model]

The dental pulp-derived cells were administered, at a dose of $1.0 \times 10^6$ cells/body via tail vein to the cerebral infraction model rats prepared by the method described in Example 34. Twenty-four hours or 48 hours following the administration, the animals were euthanized by exsanguination, and cerebral tissues were collected, on which immunohistochemical analysis was conducted using formalin-fixed, paraffin-embedded sections. Immunostaining was carried out using anti-angiopoietin-1 antibody (Abcam, Cat.# Ab133425) or anti-myeloperoxidase (MPO) antibody (Abcam, Cat.# Ab208670). In the above, myeloperoxidase is an enzyme highly expressed in neutrophils, and employed as a neutrophil marker.

(Results)

The results of immunohistochemical analysis using the anti-angiopoietin-1 antibody are shown in Fig. 29. In the vehicle-administered group, no angiopoietin-1 (Ang-1) positive cell was observed on the non-infarction side (Fig. 29a), and weakly positive cells were observed around blood vessels and in the brain parenchyma on the infarction side (Fig. 29b). On the other hand, in the dental pulp-derived cell-administered group, staining with the anti-Ang-1 antibody was observed across the whole brain tissue including that on the non-infarction side (Fig. 29c), and a number of Ang-1 highly positive cells were observed especially around blood vessels (Fig. 29d). These results indicate that administration of dental pulp-derived cells causes an increase in the concentration of angiopoietin-1 in the whole brain, and in particular, at the site of cerebral infarction, which then suggests that the effect of administered dental pulp-derived cells to bring about recovery from neurological symptoms is exerted via angiopoetin-1.

The results of immunohistochemical analysis employing the anti-myeloperoxidase antibody are shown in Fig. 30 and Fig. 31. In the vehicle-administered group, while no MPO positive cells were observed on the non-infarction side, a number of MPO positive cells were observed in the area surrounding the sites of infarction (Fig. 30a). In the dental pulp-derived cell-administered group, positive cells were observed in the area surrounding the sites of infarction (Fig. 30b). However, while the number of positive cells per field of view was $157.9 \pm 11.5$ (cells, mean $\pm$ standard error) in the vehicle-administered group, that was $66.0 \pm 18.8$ (cells, mean $\pm$ standard error) in the dental pulp-derived cell-administered group, which was lower with a statistical significance (Fig. 31). These results indicates that administration of dental pulp-derived cells suppresses neutrophil infiltration into the site of infarction. Combined with the result in Example 33, it is considered that the effect of administered dental pulp-derived cells to bring about recovery from neurological symptoms is due to amelioration of inflammation at the site of infarction as a result of suppression of neutrophil infiltration into the site of infarction, by the dental pulp-derived cells.

INDUSTRIAL APPLICABILITY

The present invention is useful in providing a pharmaceutical product comprising as an active ingredient the dental pulp-derived cells that can be administered to a human and aimed, for example, at suppression of excessively increased permeability of vascular endothelium or suppression of infiltration of immune cells such as neutrocytes.

REFERENCE SIGNS LIST

1. Angiopoietin-1 highly positive cells
2. Blood vessels
3. Brain parenchyma
4. Myeloperoxidase positive cells

**Claims**

1. A pharmaceutical composition comprising dental pulp-derived stem cells for suppressing infiltration of at least one of neutrophils, monocytes, or lymphocytes into a tissue.

2. The pharmaceutical composition according to claim 1 that suppresses expression of an adhesion factor.

3. The pharmaceutical composition according to claim 2, wherein the adhesion factor is expressed on the surfaces of vascular endothelial cells of the tissue.

4. The pharmaceutical composition according to claim 2 or 3, wherein the adhesion factor is VCAM-1.

5. The pharmaceutical composition according to one of claims 2 to 4, wherein the dental pulp-derived stem cells increase the tissue concentration of a factor that suppresses expression of the adhesion factor.

6. The pharmaceutical composition according to claim 5, wherein the factor includes angiopoietin-1.

7. The pharmaceutical composition according to one of claims 1 to 6, wherein the composition suppresses excessive increase of vascular permeability in the tissue.

8. The pharmaceutical composition according to one of claims 1 to 7 for treatment and/or prophylaxis of a disorder accompanied by inflammation.

9. The pharmaceutical composition according to claim 8, wherein the disorder accompanied by inflammation is selected from the group consisting of cerebral infarction, cerebral bleeding, retinopathy accompanied by macular degeneration, acute lung injury, chronic inflammatory pulmonary disease, asthma, acute pancreatitis, myocardial infarction, heart failure, dermatitis, and scepsis.

10. The pharmaceutical composition according to claim 9, wherein the disorder accompanied by inflammation is acute lung injury, wherein the acute lung injury is selected from the group consisting of aspiration pneumonitis, bacterial pneumonitis, viral pneumonitis, acute respiratory distress syndrome (also written as ARDS), and interstitial pneumonia.

11. The pharmaceutical composition according to claim 10, wherein the acute lung injury is the viral pneumonitis, wherein the viral pneumonitis is caused by infection with corona virus, influenza virus, or RS virus.

12. The pharmaceutical composition according to claim 11, wherein the viral pneumonitis is caused by infection with corona virus, wherein the corona virus is SARS-CoV, MARS-CoV, or SARS-CoV-2.

13. The medicinal composition according to claim 9, wherein the disorder accompanied by inflammation is chronic inflammatory pulmonary disease, wherein the chronic inflammatory pulmonary disease is selected from the group consisting of cystic fibrosis (also written as CF), chronic obstructive pulmonary disease (also written as COPD), lung dysplasia, chronic pulmonary disease, and idiopathic pulmonary fibrosis (also written as IPF).

14. The pharmaceutical composition according to one of claims 1 to 7 comprising dental pulp-derived stem cells as an active ingredient for treatment of infection with SARS-CoV, MARS-CoV, or SARS-CoV-2.

15. The pharmaceutical composition according to one of claims 1 to 14 for administration to a human.

16. The pharmaceutical composition according to one of claims 1 to 15, wherein the dental pulp-derived stem cells are of human origin

17. The pharmaceutical composition according to one of claims 1 to 16, wherein the pharmaceutical composition is for administration via a route of administration selected from the group consisting of intravenous administration, intra-arterial administration, intraportal administration, intracutaneous administration, subcutaneous administration, intramuscular administration, intracerebroventricular administration, intra-endocardium administration, intra-respiratory tract administration, intraocular administration, intra-aural administration, intranasal application, and intra-articular administration.

18. The pharmaceutical composition according to one of claims 1 to 17, wherein the pharmaceutical composition is administered either only once or repeatedly.

19. The pharmaceutical composition according to one of claims 1 to 18, wherein the dental pulp-derived stem cells have at least one of the characteristics defined in (1) to (4) below:

   (1) positive for CD73, CD90, CD105, and CD166; negative for CD34, CD40, CD45, CD80, CD86, and MHC-class II antigen; turning positive for MHC-class II antigen when stimulated with interferon $\gamma$; expressing a prostaglandin $E_2$ and/or vascular endothelial growth factor, wherein the levels of prostaglandin $E_2$ expression in-

creasing when stimulated with TNF-$\alpha$.

(2) positive for at least one of CD73, CD90, D105, and D166; and negative for CD34 and CD45,

(3) positive for at least one of CD47, CD81, and CD147; and negative for at least one of CD19, CD34, and CD206,

(4) positive for at least one of CD47, CD81, and CD147; and negative for at least one of CD19, CD31, CD33, CD34, CD38, CD45, CD206, CD235a, and SSEA-1.

20. The pharmaceutical composition according to one of claims 1 to 19, wherein the dental pulp-derived stem cells have an ability to differentiate into osteocytes and chondrocytes.

21. The pharmaceutical composition according to one of claims 1 to 20, wherein the mean diameter of the dental pulp-derived stem cells is 16-20 $\mu$m when suspended in a culture medium.

22. The pharmaceutical composition according to one of claims 1 to 21, wherein the dental pulp-derived stem cells have an ability to undergo cell divisions not less than three times in an in vitro environment, and their mean doubling time is not more than 96 hours.

23. The pharmaceutical composition according to one of claims 1 to 21, wherein the dental pulp-derived stem cells are characterized as follows:

having undergone cell divisions in an in vitro environment not less than 10 times, not less than 15 times, not less than 16 times or not less than 17 times, with their mean time required per cell division being not more than 48 hours; and
having an ability to undergo cell divisions in an in vitro environment not less than 10 times, not less than 14 times of not less than 15 times, with their mean time required per cell division being not more than 96 hours, not more than 84 hours, not more than 72 hours, not more than 48 hours, or not more than 36 hours.

24. The pharmaceutical composition according to one of claims 1 to 21, wherein the dental pulp-derived stem cells are characterized as follows:

having undergone cell divisions under an in vitro condition not less than 16 times, not less than 21 times, not less than 22 times, or not less than 23 times; and
having an ability to undergo cell divisions in an in vitro environment not less than 3 times, not less than 4 times, or not less than 5 times, and their mean time required per cell division being not more than 96 hours, not more than 84 hours, not more than 72 hours, not more than 48 hours, or not more than 36 hours.

25. The pharmaceutical composition according to one of claims 1 to 24, wherein the dental pulp-derived stem cells are negative for at least one of CD19, CD26, CD106, CD117, and CD271.

26. The pharmaceutical composition according to one of claims 1 to 25, wherein the dental pulp-derived stem cells are positive for at least one of CD140b and HLA-A, B, C.

27. The pharmaceutical composition according to one of claims 1 to 26, wherein the dental pulp-derived stem cells are negative for at least one of CD56 and CD146.

28. The pharmaceutical composition according to one of claims 1 to 27, wherein the dental pulp-derived stem cells are positive for at least one of CD49e and CD95.

29. The pharmaceutical composition according to one of claims 1 to 28, wherein the dental pulp-derived stem cells are positive for at least one of CD10, CD46, CD47, CD55, CD58, and CD59.

30. The pharmaceutical composition according to one of claims 1 to 29, wherein the dental pulp-derived stem cells express at least one of MMP-2, IGFBP-4, cystatin C, IL-6, IL-11, MCP-1, IL-8, HGF, VEGF, TIMP-1, TIMP-2, and TIMP-3.

31. The pharmaceutical composition according to one of claims 1 to 30, wherein the dental pulp-derived stem cells express at least one of GRO$\alpha$, VCAM-I, and IP-10.

32. The pharmaceutical composition according to one of claims 1 to 31, wherein the dental pulp-derived stem cells express IL-6, wherein the levels of expression thereof increase when stimulated with TNF-$\alpha$, and when stimulated with interferon $\gamma$.

33. The pharmaceutical composition according to one of claims 1 to 32, wherein the dental pulp-derived stem cells express IL-11, wherein the levels of expression thereof increase when stimulated with TNF-$\alpha$, and when stimulated with interferon $\gamma$.

34. The pharmaceutical composition according to one of claims 1 to 33, wherein the dental pulp-derived stem cells express IP-10, wherein the levels of expression thereof increase when stimulated TNF-$\alpha$, and when stimulated with interferon $\gamma$.

35. The pharmaceutical composition according to one of claims 1 to 34, wherein the dental pulp-derived stem cells express MCP-1, wherein the levels of expression thereof increase when stimulated with TNF-$\alpha$, and when stimulated with interferon $\gamma$.

36. The pharmaceutical composition according to one of claims 1 to 35, wherein the dental pulp-derived stem cells are induced to express GM-CSF when stimulated with TNF-$\alpha$.

37. The pharmaceutical composition according to one of claims 1 to 36, wherein the dental pulp-derived stem cells express HGF, wherein the level of expression thereof decreases when stimulated with TNF-$\alpha$, and increase when stimulated with interferon $\gamma$.

38. The pharmaceutical composition according to one of claims 1 to 37, wherein the dental pulp-derived stem cells express IL-8, wherein the level of expression thereof increases when stimulated with TNF-$\alpha$.

39. The pharmaceutical composition according to one of claims 1 to 38, wherein the dental pulp-derived stem cells express G-CSF, wherein the level of expression thereof increases when stimulated with TNF-$\alpha$.

40. The pharmaceutical composition according to one of claims 1 to 39, wherein the amount of angiopoietin-1 produced by the dental pulp-derived stem cells is not less than three times the amount thereof produced by human bone marrow-derived stem cells, when the dental pulp-derived stem cells and the human bone marrow-derived stem cells are respectively cultured under the same condition.

41. The pharmaceutical composition according to one of claims 1 to 40, wherein the dental pulp-derived stem cells are induced to express G-CSF when stimulated with TNF-$\alpha$.

42. The pharmaceutical composition according to one of claims 1 to 41, wherein the dental pulp-derived stem cells are derived from dental pulp obtained from a human permanent tooth or deciduous tooth.

43. The pharmaceutical composition according to one of claims 1 to 42, wherein the dental pulp-derived stem cells are contained in a suspended form in a solution that comprises sodium ion, potassium ion, calcium ion, magnesium ion, bicarbonate ion, citrate ion, human serum albumin, and dimethyl sulfoxide.

44. The pharmaceutical composition according to one of claims 1 to 42, wherein the dental pulp-derived stem cells are contained in a suspended form in bicarbonate Ringer's solution that contains human serum albumin and dimethyl sulfoxide.

45. The pharmaceutical composition according to claim 43 containing 91-113 mM sodium ion, 2.52-3.08 mM potassium ion, 0.95-1.16 mM calcium ion, 0.315-0.385 mM magnesium ion, 15.6-19.2 mM bicarbonate ion, 1.04-1.28 mM citrate ion, 46-56 g/L human serum albumin, and 9-11 % (v/v) dimethyl sulfoxide.

46. The pharmaceutical composition according to one of claims 43 to 45, further comprising acetyltryptophan or a salt thereof and caprylic acid or a salt thereof.

47. The pharmaceutical composition according to one of claims 43 to 46, wherein the dental pulp-derived stem cells are contained at a density of $5\times10^6$ to $8\times10^7$ cells/mL.

48. The pharmaceutical composition according to one of claims 43 to 47, sealed in a container in an amount of 1-20 mL.

**49.** The pharmaceutical composition according to claim 48, wherein the container is made of a glass or plastic.

**50.** The pharmaceutical composition according to one of claims 43 to 49 in a frozen state.

**51.** A pharmaceutical composition for elevating angiopoietin-1 concentration in a tissue, wherein the composition comprises dental pulp-derived stem cells as an active ingredient.

**52.** A pharmaceutical composition for suppressing expression of an adhesion factor, wherein the composition comprises dental pulp-derived stem cells as an active ingredient.

**53.** A pharmaceutical composition for treatment or prophylaxis of a disorder accompanied by inflammation, wherein the composition comprises dental pulp-derived stem cells as an active ingredient.

Fig. 1

Fig. 2

Fig. 3

Fig. 4

Fig. 5

Fig. 6

Fig. 7

Fig. 8

Fig. 9

Fig. 10

Fig. 11A

Fig. 11B

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Days from creation of pathological conditions (days)

Fig. 28

EP 4 098 267 A1

Whole image of cerebral tissues     Enlarged image around site of infarction

(a)

Vehicle

Non-infarction side          Infarction side

(b)

3

2

(c)

Dental pulp-derived
cells

Non-infarction side          Infarction side

(d)

1          3

2

Fig. 29

99

Enlarged image around site of infarction

(a)

4

Vehicle

(b)

Dental pulp-derived
cells

Fig. 30

Fig. 31

## INTERNATIONAL SEARCH REPORT

| International application No. |
| --- |
| PCT/JP2021/003201 |

### A. CLASSIFICATION OF SUBJECT MATTER

A61K 35/28(2015.01)i; A61K 9/10(2006.01)i; A61K 47/02(2006.01)i; A61K 47/12(2006.01)i; A61K 47/20(2006.01)i; A61K 47/22(2006.01)i; A61K 47/42(2017.01)i; A61P 1/18(2006.01)i; A61P 7/04(2006.01)i; A61P 9/04(2006.01)i; A61P 9/10(2006.01)i; A61P 9/14(2006.01)i; A61P 11/00(2006.01)i; A61P 11/06(2006.01)i; A61P 17/00(2006.01)i; A61P 27/02(2006.01)i; A61P 29/00(2006.01)i; A61P 31/04(2006.01)i; A61P 31/12(2006.01)i; A61P 31/14(2006.01)i; A61P 31/16(2006.01)i; A61P 43/00(2006.01)i

FI:     A61K35/28; A61K9/10; A61K47/02; A61K47/12; A61K47/20; A61K47/22; A61K47/42; A61P1/18; A61P7/04; A61P9/04; A61P9/10; A61P9/14; A61P11/00; A61P11/06; A61P17/00; A61P27/02; A61P29/00; A61P31/04; A61P31/12; A61P31/14; A61P31/16; A61P43/00 105

According to International Patent Classification (IPC) or to both national classification and IPC

### B. FIELDS SEARCHED

Minimum documentation searched (classification system followed by classification symbols)
A61K35/28; A61K9/10; A61K47/02; A61K47/12; A61K47/20; A61K47/22; A61K47/42; A61P1/18; A61P7/04; A61P9/04; A61P9/10; A61P9/14; A61P11/00; A61P11/06; A61P17/00; A61P27/02; A61P29/00; A61P31/04; A61P31/12; A61P31/14; A61P31/16; A61P43/00

Documentation searched other than minimum documentation to the extent that such documents are included in the fields searched
    Published examined utility model applications of Japan    1922–1996
    Published unexamined utility model applications of Japan    1971–2021
    Registered utility model specifications of Japan    1996–2021
    Published registered utility model applications of Japan    1994–2021

Electronic data base consulted during the international search (name of data base and, where practicable, search terms used)
    JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| X | WO 2016/076434 A1 (JCR PHARMACEUTICALS CO., LTD.) 19 May 2016 (2016-05-19) claims 1-5, paragraphs [0022]-[0040], examples 1, 3, 4, 6 | 1-9, 15-45, 47, 50-53 |
| Y | | 9-50 |

☒    Further documents are listed in the continuation of Box C.    ☒    See patent family annex.

\*    Special categories of cited documents:
"A"    document defining the general state of the art which is not considered to be of particular relevance
"E"    earlier application or patent but published on or after the international filing date
"L"    document which may throw doubts on priority claim(s) or which is cited to establish the publication date of another citation or other special reason (as specified)
"O"    document referring to an oral disclosure, use, exhibition or other means
"P"    document published prior to the international filing date but later than the priority date claimed

"T"    later document published after the international filing date or priority date and not in conflict with the application but cited to understand the principle or theory underlying the invention
"X"    document of particular relevance; the claimed invention cannot be considered novel or cannot be considered to involve an inventive step when the document is taken alone
"Y"    document of particular relevance; the claimed invention cannot be considered to involve an inventive step when the document is combined with one or more other such documents, such combination being obvious to a person skilled in the art
"&"    document member of the same patent family

| Date of the actual completion of the international search | Date of mailing of the international search report |
| --- | --- |
| 31 March 2021 (31.03.2021) | 13 April 2021 (13.04.2021) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
| --- |
| PCT/JP2021/003201 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | JP 2016-008198 A (NAGOYA UNIVERSITY) 18 January 2016 (2016-01-18) claim 1, paragraphs [0030]- | 1-8, 15, 16, 18-42, 51-53 |
| Y | [0036], examples | 9-50 |
| X | HILKENS, P. et al., "Pro-angiogenic impact of | 51 |
| Y | dental stem cells in vitro and in vivo.", Stem Cell Research, 2014, vol. 12, pp. 778-790, abstract part, table 2, page 788, right column, column "result" | 1-53 |
| Y | KIM, I. et al., "Angiopoietin-1 Reduces VEGF-Stimulated Leukocyte Adhesion to Endothelial Cells by Reducing ICAM-1, VCAM-1, and E-Selectin Expression", Circulation Research, 2001, vol. 89, pp. 477-479, abstract part | 1-53 |

Form PCT/ISA/210 (continuation of second sheet) (January 2015)

| International application No. |
|---|
| PCT/JP2021/003201 |

| Patent Documents referred in the Report | Publication Date | Patent Family | Publication Date |
|---|---|---|---|
| WO 2016/076434 A1 | 19 May 2016 | US 2017/0327791 A1 claims 1-5, paragraphs [0062]- [0080], examples 1, 3, 4, 6 EP 3219322 A1 | |
| JP 2016-008198 A | 18 Jan. 2016 | (Family: none) | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5486359 A
- US 5827740 A
- US 6835377 B
- US 6387369 B
- JP 2004129549 A
- JP 2004210713 A
- JP 2010252778 A
- WO 2002007679 A
- JP 2008507962 A
- WO 2009072527 A
- JP 2004201612 A
- JP 2009527223 A
- JP 2005516616 A
- JP 2006238875 A
- JP 2008295420 A
- JP 2010268715 A
- JP 2011219432 A
- WO 2017099105 A

### Non-patent literature cited in the description

- **KATRITSIS DG.** *Catheter Cardiovasc Interv,* 2005, vol. 65 (3), 321-9
- **GRONTHOS S.** *J Dent Res.,* 2002, vol. 81 (8), 531-5
- **TIRINO V.** *Stem Cell Rev,* 2011, vol. 7 (3), 608-15
- **VISHWANATH VR.** *J Conserv Dent.,* 2013, vol. 16 (5), 423-8
- **HUANG ZW.** *Yonsei Med J.,* January 2017, vol. 58 (1), 206-216
- **HALIM NSS.** *Stem Cell Rev Rep.,* February 2019, vol. 15 (1), 112-125
- **XU J.** *J Pathol.,* March 2008, vol. 214 (4), 472-81
- **MEI SH.** *PLoS Med.,* September 2007, vol. 4 (9), e269
- **SHAO Y.** *Inhal Toxicol.,* June 2018, vol. 30 (7-8), 313-320
- **SUN L.** *Biochem Biophys Res Commun,* 11 April 2007, vol. 357 (3), 779-84
- **CHEN SL.** *J Int Med Res,* January 2009, vol. 37 (1), 68-78
- **TOYAMA K.** *Exp Neurol.,* 27 November 2008, vol. 216 (1), 47-55
- **ONDA T.** *J Cereb Blood Flow Metab.,* 18 July 2007, vol. 28 (2), 329-40
- **LIU FY.** *J Cell Physiol.,* 15 May 2018, vol. 233 (11), 8567-8577
- **HUA J.** *Int J Clin Exp Pathol.,* 15 June 2014, vol. 7 (7), 3580-95
- **PIAO W.** *Angiogenesis,* 11 May 2010, vol. 13 (3), 203-10
- **LI Y.** *Stem Cell Res Ther.,* 16 September 2013, vol. 4 (5), 113
- **CAO L.** *J Biomed Mater Res B Appl Biomater.,* 11 May 2012, vol. 100 (5), 1229-36
- **KOTA S.** *Molecular Therapy:Method & Clinical Development,* September 2018, vol. 10, 281-290
- **J. GOLLEDGE.** *J the American Heart Association Stroke,* 25 February 2014, vol. 45, 1064-1068
- **LIMANGEL A.M.** *Critical Care,* 2010, vol. 14, R91
- **SASCHA D.** *Critical Care,* 2010, vol. 14, 180
- **CHEN J.** *Hum Mol Genet.,* 08 April 2010, vol. 19 (12), 2524-33
- **PITTENGER MF. et al.** *Science,* 1999, vol. 284, 143-7
- **COLTER DC. et al.** *Proc Natl Acad Sci USA.,* 2001, vol. 98, 7841-5
- **KIANI C. et al.** *Cell Res.,* 2002, vol. 12, 19-32
- **AUNG A. et al.** *Arthritis Rheum.,* 2011, vol. 63, 148-58